(19) Europäisches Patentamt / European Patent Office / Office européen des brevets

(11) **EP 3 777 537 A1**

(12) **EUROPEAN PATENT APPLICATION**
published in accordance with Art. 153(4) EPC

(43) Date of publication:
**17.02.2021 Bulletin 2021/07**

(21) Application number: **19781264.7**

(22) Date of filing: **02.04.2019**

(51) Int Cl.:
*A01N 59/08* (2006.01)     *A01N 25/08* (2006.01)
*A01P 1/00* (2006.01)      *A23L 3/3454* (2006.01)
*A61L 2/18* (2006.01)      *C01B 11/06* (2006.01)
*C01B 11/10* (2006.01)     *C02F 1/50* (2006.01)

(86) International application number:
**PCT/JP2019/014649**

(87) International publication number:
**WO 2019/194184 (10.10.2019 Gazette 2019/41)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.04.2018 JP 2018071515**

(71) Applicant: **HONBUSANKEI CO., LTD.**
**Osaka-shi**
**Osaka 540-0001 (JP)**

(72) Inventor: **GODA Hisataka**
**Osaka-shi, Osaka 541-0048 (JP)**

(74) Representative: **Müller-Boré & Partner**
**Patentanwälte PartG mbB**
**Friedenheimer Brücke 21**
**80639 München (DE)**

(54) **MANUFACTURING METHOD FOR OBTAINING NOVEL CHLORINE OXIDE COMPOSITION FROM DEGRADED HYPOCHLORITE**

(57)     The purpose of the present invention is to provide a method for manufacturing a new disinfectant from sodium hypochlorite that has degraded in quality during storage. A method for manufacturing a novel disinfectant from a solution containing hypochlorite ions, chlorate ions, and chloride ions, wherein the method includes: a first reaction step for adding sulfuric acid to the solution and generating chlorine gas; a step in which, in a recovery liquid A, the generated chlorine gas is caused to react with sodium hydroxide or calcium hydroxide and recovered as hypochlorite ions; a second reaction step for adding, to a reaction mother liquid after the first reaction step, sulfuric acid having a higher concentration than that in the first reaction step, and generating chlorine dioxide gas; a step in which, in a recovery liquid B, the generated chlorine dioxide gas is caused to react with sodium hydroxide and hydrogen peroxide and recovered as chlorite ions; and a step for mixing the recovery liquid A and the recovery liquid B and obtaining a novel disinfectant.

**Description**

[Technical Field]

The present invention regenerates sodium hypochlorite, in which chlorine has degraded during storage and chloride ions and chlorate ions have been generated, to newly obtain a useful disinfectant.

[Background Art]

Sodium hypochlorite is a chlorine liquid obtained by passing chlorine through a sodium hydroxide solution. It is known as a useful disinfectant of public water supply systems, pools, and food additives. Meanwhile, sodium hypochlorite is unstable, so that the chlorine component degrades during storage, and chloride ions ($Cl^-$) and chlorate ions ($ClO_3^-$) through a disproportionation reaction are generated, and the efficacy thereof is lost. Chlorate ions ($ClO_3^-$) in a dry and crystallized state continue to lead to accidents such as ignition or explosion due to friction, so that they need to be carefully disposed.

While large scale manufacturers of sodium hypochlorite prevent deterioration in quality by controlling the temperature using a refrigerator or the like, it is challenging for subdividers for users of small quantities, mainly food manufacturers, to manage the temperature, resulting in reduced quality during storage and the aforementioned problems.

When hypochlorite ions decrease and the content of chloride ion and chlorate ion increases in sodium hypochlorite, normal use thereof is no longer possible. The disposal thereof involves dechlorination/neutralization, or commissioning the disposal to a contractor.

[Citation List]

[Patent Literature]

[PTL1] Japanese Patent No. 5931253

[Summary of Invention]

[Solution to Problem]

The present invention solves such a problem. The present invention treats sodium hypochlorite whose quality is deteriorated during storage or recovered sodium hypochlorite as a resource and subjects such sodium hypochlorite to a reaction again to newly produce a useful and valuable disinfectant. The completed disinfectant is a novel chlorine oxide liquid comprising both hypochlorite ions and chlorite ions by re-reacting the components. Furthermore, a solid whose components do not change from long term storage was successfully manufactured by drying the completed disinfectant. It has also been found that the disinfectant not only improves the bactericidal effect, but also has an advantage of having less chlorine odor, so that a value-added product which is capable of absorbing the manufacturing cost associated with recycling can be produced.

Research and development was conducted for a method of regenerating hypochlorous acid by recovering and re-reacting sodium hypochlorite whose quality is deteriorated during storage and reacting chloric acid generated during storage to manufacture a novel disinfectant containing both hypochlorous acid and chlorous acid.

Further, a method of obtaining a new value-added disinfectant that can absorb the manufacturing cost associated with recycling is provided.

The inventor has manufactured a disinfectant with a new product value by re-reacting sodium hypochlorite with deteriorated in quality in order to solve the problem described above.

The disinfectant is a new chlorine oxide liquid or solid containing both hypochlorous acid and chlorous acid by re-recovering sodium hypochlorite with reduced concentration as chlorine gas and reacting sodium chlorate, which is difficult to dispose, and re-recovering the resulting gas as chlorine dioxide gas to stabilize it with alkalinity.

First, the difference in the deterioration in quality due to the difference in the quality of sodium hypochlorite was studied. It is important for the study whether there is a difference in the amounts of generated chlorine ions and chlorate ions associated with the loss of available chlorine. Meanwhile, about 14000 to 26000 ppm of chlorate ions is generated in many general grade 12% sodium hypochlorite solutions upon distribution, and 5000 to 6000 ppm and at most 12000 ppm of chlorate ions in low salt grade 12% sodium hypochlorite solution, with a significant difference in the final quantity of chloride ions produced. Thus, it was found that a reaction/manufacturing method needs to be established while taking the maximum amount of chlorate ions and chloride ions generated into consideration. However, even if available chlorine of sodium hypochlorite is disappeared by degradation, available chlorine at 4% or less does not remain within the specification of sodium hypochlorite, so that sodium hypochlorite cannot be considered as being used as a raw material. For this reason, it was found that target raw material sodium hypochlorite with deteriorated in quality (hereinafter, referred to as deteriorated sodium hypochlorite) are those with available chlorine of 4% or greater and amount of chlorate ion generated up to about 48000 ppm.

Next, the inventor contemplated determining the quantity of hypochlorite ions, chlorate ions, chloride ions, and the like contained in deteriorated sodium hypochlorite and finding the conditions for the gasification by a reaction to obtain a recovery liquid. However, such a method, unlike the reaction/manufacturing methods for various chlorine raw materials that are commonly known, has poorer reactivity compared to a common chemical reaction method using sodium hypochlorite alone or a saturated solution thereof as a raw material because deteriorated sodium hypochlorite concurrently contains many and various chlorine ion components, and chlorate ions and chloride ions are products generated with disappearance of available chlorine. Thus, it was necessary to find a new reaction condition and recovery method.

The concentration of sulfuric acid to be added, acidity in the reaction mother liquor, and the amount of the generated product, chloride ions, in deteriorated sodium hypochlorite are important in the reaction method. However, it was found that chloride ions in particular are difficult to adjust due to being products from sodium hypochlorite, so that it is necessary to increase the yield by combining temperature, acidity, air blowing conditions and the like to adjust the amount of reaction product.

While chlorine gas and chlorine dioxide gas obtained by a reaction are recovered with sodium hydroxide or calcium hydroxide, it was found that if they were not recovered individually, the yield would be reduced and chlorate ions would be generated. It was found that a technique for releasing chlorine gas and then chlorine dioxide gas in a stepwise fashion from a reaction mother liquor to which deteriorated sodium hypochlorite and sulfuric acid were added is required.

Specifically, a first reaction in which chlorine gas is generated from a reaction mother liquor in which sulfuric acid is added to deteriorated sodium hypochlorite as a raw material. Next, a second reaction of generating chlorine dioxide gas shall be performed after adding additional sulfuric acid and the like and changing other conditions. Since it was revealed that chloride ions and chlorate ions increase/decrease depending on the reaction condition such as acidity of the reaction mother liquor during the first reaction, one of the features of the present manufacturing method is to generate chlorine dioxide gas after adjusting, for the second reaction, the reaction mother liquor with a composition that has changed from the first reaction.

Although chlorine gas generated in the first reaction can be recovered with a sodium hydroxide solution or calcium hydroxide solution, a large quantity of chlorate ions would be generated if chlorine dioxide gas generated primarily in the second reaction is blown into only a sodium hydroxide solution or only a calcium hydroxide solution. For this reason, it is necessary to prevent generation of chlorate ions by using a recovery liquid prepared by adding hydrogen peroxide water to a sodium hydroxide solution or calcium hydroxide solution. On the contrary, the presence of hydrogen peroxide would degrade and change chlorine gas to chloride ions.

In view of the above, one of the features of the present manufacturing method is in providing two recovery vessels for a recovery liquid for the first reaction and a recovery liquid for the second reaction and recovering recovery liquid A mainly of chlorine gas and recovery liquid B mainly of chlorine dioxide gas separately, and mixing and stabilizing at a later time.

A mixed solution, which is manufactured by the present manufacturing method and adjusted to meet the specification and standard of sodium hypochlorite for a food additive, has poor storability. Such a solution can only be sold refrigerated.

In this regard, one of the features of the present invention is a solid disinfectant prepared by recovering chlorine gas in the first reaction not only in sodium hydroxide, but also in calcium hydroxide and then mixing it with a recovery liquid obtained from the second reaction and drying the mixture.

When in a solid form, available chlorine needs to be concentrated to a high concentration by drying, so that it is necessary to reduce the alkalinity of the recovery liquid as much as possible, and to recover chloride ions and residual alkaline components while reducing the amount as much as possible. A solid disinfectant manufactured and dried in this manner is characterized by being compliant with the specification for a food additive, high grade chlorinated lime, and the composition of the components should not change when stored at ordinary temperature.

Accordingly, the required reaction conditions and purity of the recovery liquid differ between liquids and solids. Meanwhile, a disinfectant manufactured by this method is characterized by possessing bactericidal features of both hypochlorous acid and chlorous acid, and being compliance to the specification and standard for a food additive, sodium hypochlorite or high grade chlorinated lime, and providing such features in a single agent.

For the materialization thereof, reaction conditions are adjusted with low salt grade sodium hypochlorite and general grade sodium hypochlorite. As the first reaction, the sulfuric acid concentration in the reaction mother liquor is adjusted to 4.0% to 6.37% for low salt grade sodium hypochlorite, and the sulfuric acid concentration in the reaction mother liquor is adjusted to 4.0% to 4.5% for general grade sodium hypochlorite. As the second reaction, the sulfuric acid concentration in the reaction mother liquor is adjusted to 30.0% to 59.4% after adding hydrogen peroxide water to the reaction mother liquor when the final product is a sodium hypochlorite specification compliant product for low salt grade sodium hypochlorite, and the sulfuric acid concentration in the reaction mother liquor is adjusted to 30.0% to 40.0% for high grade chlorinated lime. In addition, 50.0 w/w% to 70.0 w/w% is used as the sulfuric acid concentration. For general grade sodium hypochlorite, the concentration is 25.0% to 30.0% and the sulfuric acid concentration used is 65 w/w%. Furthermore, an intermediate trapping vessel is provided for washing and removing chlorine gas to prevent contamination of recovery liquid from excessive generation of chlorine gas.

It is also necessary to improve the recovery rate by blowing in a large quantity of air into a reaction tank starting from immediately after adding raw materials.

Recovery liquid A and recovery liquid B recovered separately by the present manufacturing method comply with the specification of sodium hypochlorite for food additive when mixed at a ratio of available chlorine concentrations of 1: 0.43 to 1:0.6, given that an available chlorine concentration of recovery liquid A as 1, and comply with the food additive specification for high grade chlorinated lime in the range of available chlorine concentration ratios of up to 1:33.95.

High grade chlorinated lime manufactured at such a formulation was found to be capable of longer storage than commercially sold high grade chlorinated lime and to be compliant with the specification.

The present invention also provides the following.

(Item 1)

A dry solid comprising a hypochlorite and a chlorite.

(Item 2)

The dry solid of item 1, wherein the solid is in a dry granules.

(Item 3)

The dry solid of item 1 or 2, wherein the solid comprises calcium hypochlorite.

(Item 4)

The dry solid of any one of items 1 to 3, wherein the solid has the following properties:

(1) comprises available chlorine at 60.0% or more;
(2) has a chlorine odor;
(3) when 5 ml of water is added to 0.5 g of the solid and shaken and red litmus paper is immersed therein, the litmus paper changes its color to blue and then loses its color; and
(4) when 2 ml of acetic acid (1 → 4) is added to 0.1 g of the solid, the solid dissolves while generating gas, and a solution prepared by adding 5 ml of water thereto and filtering exhibits a reaction of calcium salt.

(Item 5)

The dry solid of any one of items 1 to 4, wherein the solid comprises an $SO_4$ based component in a range from the detection limit or higher and 8100 ppm or lower.

(Item 6)

The dry solid of any one of items 1 to 5, wherein a ratio of the hypochlorite to the chlorite in the solid is 1:5 to 25.

(Item 7)

The dry solid of any one of items 1 to 6, wherein an available chlorine concentration in the solid is within a range of 600,000 ppm to 900,000 ppm, and a free residual chlorine concentration is within a range of 900 ppm to 60,000 ppm.

(Item 8)

A liquid obtained by dissolving the dry solid of any one of items 1 to 7.

(Item 9)

The liquid of item 8, wherein a ratio of hypochlorite ions to chlorite ions is 1: 7 to 35, when diluted with water so that an available chlorine concentration would be 1%.

(Item 10)

The liquid of item 8 or 9, wherein a free residual chlorine concentration is within a range of 150 ppm to 900 ppm, when diluted with water so that an available chlorine concentration would be 1%.

(Item 11)

The liquid of item 8, wherein a ratio of hypochlorite ions to chlorite ions is 1:6 to 30, when diluted with water so that an available chlorine concentration would be 6%.

(Item 12)

The liquid of item 8 or 11, wherein a free residual chlorine concentration is within a range of 1,000 ppm to 6,000 ppm, when diluted with water so that an available chlorine concentration would be 6%.

(Item 13)

The liquid of item 8, wherein a ratio of hypochlorite ions to chlorite ions is 1:6 to 30, when diluted with water so that an available chlorine concentration would be 12%.

(Item 14)

The liquid of item 8 or 13, wherein a free residual chlorine concentration is within a range of 2,500 ppm to 12,000 ppm, when diluted with water so that an available chlorine concentration would be 12%.

(Item 15)

A method of manufacturing a dry solid comprising a hypochlorite and a chlorite, comprising:

a step of preparing a solution comprising a hypochlorite ion, a chlorate ion, and a chloride ion;
a first reaction step for adding sulfuric acid to the solution to generate chlorine gas;
a step of reacting the generated chlorine gas with sodium hydroxide or calcium hydroxide and recovering a reaction product as a hypochlorite ion in recovery liquid A;
a second reaction step for adding sulfuric acid to a reaction mother liquor after the first reaction step at a concentration that is higher than that in the first reaction step to generate chlorine dioxide gas;
a step of reacting the generated chlorine dioxide gas with sodium hydroxide and hydrogen peroxide and recovering a reaction product as a chlorite ion in recovery liquid B;
a step of mixing recovery liquid A with recovery liquid B; and
a step of drying and solidifying the resulting mixture.

(Item 16)

The method of item 15, wherein the recovery liquid A comprises calcium hydroxide.

(Item 17)

The method of item 15 or 16, further comprising a step of adding hydrogen peroxide to the reaction mother liquor after a first reaction.

(Item 18)

The method of any one of items 15 to 17, wherein an available chlorine concentration of the recovery liquid B is within a range of 9.6 to 33.95, given that an available chlorine concentration of the recovery liquid A is 1, in the step of mixing the recovery liquid A with the recovery liquid B.

(Item 19)

The method of any one of items 15 to 18, wherein the recovery liquid A and the recovery liquid B are each slurried and mixed in the step of mixing the recovery liquid A with the recovery liquid B.

(Item 20)

The method of any one of items 15 to 19, wherein the step of mixing the recovery liquid A with the recovery liquid B comprises a step of preliminary drying the recovery liquid A to form a granulation nucleus, slurrying the recovery liquid B, and adding dried recovery liquid A to the slurried recovery liquid B.

(Item 21)

The method of any one of items 15 to 20, wherein the step of drying and solidifying comprises a step of drying with warm air for 20 to 30 minutes.

(Item 22)

The method of any one of items 15 to 21, wherein the step of drying and solidifying comprises reducing moisture content of each of the recovery liquid A and the recovery liquid B to 20% or less.

(Item 23)

A method of manufacturing a new disinfectant from a solution comprising a hypochlorite ion, a chlorate ion, and a chloride ion; comprising:

a step of quantifying a hypochlorite ion concentration, a chlorate ion concentration, and a chloride ion concentration in the solution;
a first reaction step for adding sulfuric acid to the solution to generate chlorine gas;
a step of reacting the generated chlorine gas with sodium hydroxide or calcium hydroxide and recovering a reaction product as a hypochlorite ion in recovery liquid A;
a second reaction step for adding sulfuric acid to a reaction mother liquor after the first reaction step at a concentration that is higher than that in the first reaction step to generate chlorine dioxide gas;
a step of reacting the generated chlorine dioxide gas with sodium hydroxide and hydrogen peroxide and recovering a reaction product as a chlorite ion in recovery liquid B; and
a step of mixing the recovery liquid A with the recovery liquid B to obtain a new disinfectant.

(Item 24)

The method of item 23, wherein the solution comprising a hypochlorite ion, a chlorate ion, and a chloride ion is a solution comprising a hypochlorite with deteriorated quality.

(Item 25)

The method of item 24, wherein the solution comprising a hypochlorite with deteriorated quality is derived from a low salt grade sodium hypochlorite solution.

(Item 26)

The method of item 24, wherein the solution comprising a hypochlorite with deteriorated quality is derived from a general grade sodium hypochlorite solution.

(Item 27)

The method of item 25, wherein the disinfectant is a solid product, and a sulfuric acid concentration in a reaction mother liquor in the first reaction step is 4.00 to 6.37%, a sulfuric acid concentration in a reaction mother liquor in the second reaction step is 30.00 to 40.00%, and a sulfuric acid concentration used in the second reaction step is 50.0 w/w% to 70.0 w/w%.

(Item 28)

The method of item 25, wherein the disinfectant is a liquid product, and a sulfuric acid concentration in a reaction mother liquor in the first reaction step is 4.00 to 6.37%, a sulfuric acid concentration in a reaction mother liquor in the second reaction step is 30.00 to 59.04%, and a sulfuric acid concentration used in the second reaction step is 50.0 w/w% to 70.0 w/w%.

(Item 29)

The method of item 26, wherein a sulfuric acid concentration in a reaction mother liquor in the first reaction step is 4.00 to 4.50%, a sulfuric acid concentration in a reaction mother liquor in the second reaction step is 25.00 to 30.00%, and a sulfuric acid concentration used in the second reaction step is 65 w/w%.

(Item 30)

The method of any one of items 23 to 29, satisfying the following formula:

$$(1) \quad Y = -1.2676X + 9.84393;$$

and

$$(2) \quad X \leq 4;$$

wherein a chloride concentration in a raw material is X%, and a sulfuric acid concentration in a reaction mother liquor is Y% in a first reaction.

(Item 31)

The method of any one of items 23 to 30, wherein the recovery liquid A comprises sodium hydroxide or calcium hydroxide.

(Item 32)

The method of any one of items 23 to 31, wherein the recovery liquid B comprises sodium hydroxide and hydrogen peroxide.

(Item 33)

The method of any one of items 23 to 32, wherein the first reaction step is performed while blowing air.

(Item 34)

The method of any one of items 23 to 33, wherein the second reaction step is performed while blowing air.

(Item 35)

The method of any one of items 23 to 34, wherein an intermediate trapping vessel comprising hydrogen peroxide is provided between a reaction tank and a recovery vessel comprising recovery liquid B.

(Item 36)

The method of any one of items 23 to 35, further comprising a step of adding hydrogen peroxide to a reaction mother liquor after a first reaction.

(Item 37)

The method of any one of items 23 to 36, wherein an available chlorine concentration of the recovery liquid B is 0.43 to 0.6, given that an available chlorine concentration of the recovery liquid A is 1, in the step of mixing the recovery liquid A with the recovery liquid B.

(Item 38)

The method of any one of items 23 to 37, wherein the disinfectant comprises sodium hypochlorite.

(Item 39)

The method of item 38, wherein the disinfectant has the following properties:

(1) comprises available chlorine at 4.0% or more;
(2) has a chlorine odor;
(3) exhibits a reaction of a sodium salt and a reaction of a hypochlorite;
(4) a solution preparing by adding 100 ml of phosphate buffer (pH 8) to 4 ml of an aqueous solution ($1 \rightarrow 25$) of this product has a maximum absorbance at a wavelength of 291 to 294 nm; and
(5) red litmus paper, when immersed in this product, changes its color to blue and then loses its color.

(Item 40)

The method of any one of items 23 to 39, wherein the disinfectant comprises an $SO_4$ based component in a range from the detection limit or higher and 8100 ppm or lower.

(Item 41)

The method of any one of items 23 to 40, wherein a ratio of hypochlorite ions to chlorite ions in the disinfectant is 1:0.24 to 0.3.

(Item 42)

The method of any one of items 23 to 41, wherein an available chlorine concentration in the disinfectant is about 60,000 ppm, and a free residual chlorine concentration is about 60,000 ppm.

(Item 43)

A disinfectant manufactured by the method of any one of items 23 to 42.

(Item 44)

The disinfectant of item 43, wherein a ratio of hypochlorite ions to chlorite ions is 1:0.24 to 0.3.

(Item 45)

The disinfectant of item 43 or 44, wherein an available chlorine concentration in the disinfectant is about 60,000 ppm, and a free residual chlorine concentration is about 60,000 ppm.

(Item 46)

A liquid chlorine oxide manufactured by using the dry solid of any one of items 1 to 7, prepared by a method comprising:

(a) a step of dissolving the dry solid into water to prepare a solution with an elevated pH;
(b) a step of adding a non-calcium inorganic alkaline agent to the solution while maintaining a pH of the solution prepared in step (a) to allow a calcium salt to precipitate and form a solid/liquid mixed phase with a reduced calcium ion concentration in a liquid phase, comprising the liquid phase and a solid phase comprising a calcium salt; and

(c) recovering only the liquid phase from the solid/liquid mixed phase formed in step (b) to obtain a liquid chlorine oxide.

(Item 47)

A liquid chlorine oxide manufactured by using the dry solid of any one of items 1 to 7, prepared by a method comprising:

(a) a step of dissolving the dry solid into water to prepare a solution with a pH of 10.0 or higher;

(b) a step of adding a non-calcium inorganic alkaline agent to the solution while maintaining a pH of the solution prepared in step (a) at 10.0 or higher to allow a calcium salt to precipitate and form a solid/liquid mixed phase with a calcium ion concentration of 24 ppm or less in a liquid phase, comprising the liquid phase and a solid phase comprising a calcium salt; and

(c) recovering only the liquid phase from the solid/liquid mixed phase formed in step (b) to obtain a liquid chlorine oxide.

(Item 48)

The liquid or liquid chlorine oxide of any one of items 8 to 14, 46, and 47, wherein a calcium concentration is substantially at or below a detection limit.

(Item 49)

The liquid or liquid chlorine oxide of any one of items 8 to 14, 46, and 47, wherein a calcium concentration is 24 ppm or less.

(Item 50)

Use of the dry solid of any one of items 1 to 7, the liquid of any one of items 8 to 14, or the liquid or liquid chlorine oxide of any one of items 46 to 49 as a disinfectant.

(Item 51)

Use of the dry solid of any one of items 1 to 7, the liquid of any one of items 8 to 14, the disinfectant of any one of items 43 to 45, or the liquid or liquid chlorine oxide of any one of items 46 to 49 as a food additive.

(Item 52)

Use of the dry solid of any one of items 1 to 7, the liquid of any one of items 8 to 14, the disinfectant of any one of items 43 to 45, or the liquid or liquid chlorine oxide of any one of items 46 to 49, for disinfecting a food product.

(Item 53)

A method of manufacturing a new agent from a solution comprising a hypochlorite ion, a chlorate ion, and a chloride ion, comprising the following steps:

a step of quantifying the concentration of a hypochlorite ion, a chlorate ion, and a chloride ion in the solution;
a first reaction step for adding sulfuric acid to the solution to generate chlorine gas;
a step of reacting the generated chlorine gas with sodium hydroxide or calcium hydroxide and recovering a reaction product as a hypochlorite ion in recovery liquid A;
a second reaction step for adding sulfuric acid to a reaction mother liquor after the first reaction step at a concentration that is higher than that in the first reaction step to generate chlorine dioxide gas;
a step of reacting the generated chlorine dioxide gas with sodium hydroxide and hydrogen peroxide and recovering a reaction product as a chlorite ion in recovery liquid B; and
a step of mixing the recovery liquid A with the recovery liquid B to obtain a new agent.

(Item 54)

The method of item 53, having one or more features from substituting the disinfectant specified in items 24 to 42 with an agent.

(Item 55)

An agent manufactured by the method of item 53 or 54.

(Item 56)

The agent of item 55, having one or more features from substituting the disinfectant specified in item 44 or 45 with an agent.

(Item 57)

Use of the agent of item 55 or 56 as a disinfectant.

(Item 58)

Use of the agent of item 55 or 56 as a food additive.

(Item 59)

Use of the agent of item 55 or 56, for disinfecting a food product.

[Advantageous Effects of Invention]

The present invention obtains a useful and novel disinfectant from sodium hypochlorite, which has deteriorated in quality and a chloride ion and a chlorate ion generated therein. This is an invention of newly obtaining a disinfectant, which meets the specification for sodium hypochlorite or high grade chlorinated lime while having properties of both a hypochlorite ion and a chlorite ion, by using a food additive, i.e., sodium hypochlorite, as a raw material. A liquid wherein a hypochlorite ion and a chlorite ion co-exist has poor storability unless refrigerated, but the present invention has an advantage of allowing long term storage by processing liquid into a dry granular solid.

Sodium hypochlorite and high grade chlorinated lime manufactured by the present method have an advantage in terms of having lower chlorine odor, less burden on workers, and easier to use than a liquid with the same concentration. Even if a complex regeneration method is used, the manufacturing cost can be absorbed and the product can be commercialized.

[Brief Description of Drawings]

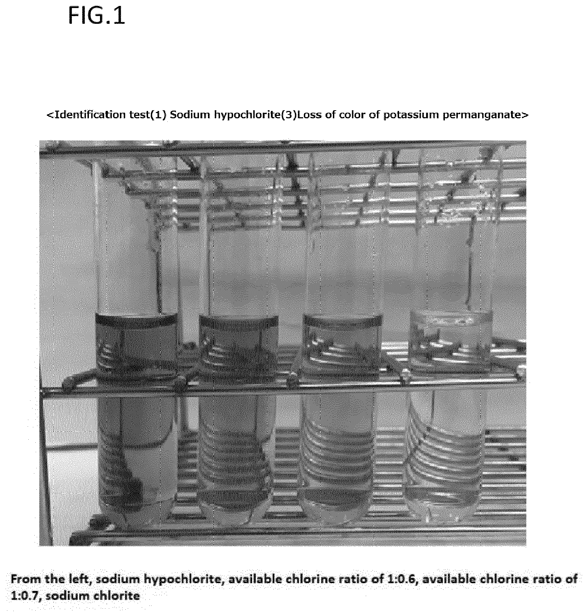

[Figure 1] Figure **1** relates to (Mixed solution of recovery liquid A and recovery liquid B (sodium hypochlorite specification)) and shows discoloration of potassium permanganate. The figure shows, from the left, only sodium hypochlorite, available chlorine ratio of sodium hypochlorite and sodium chlorite of 1:0.6, available chlorine ratio of sodium hypochlorite and sodium chlorite of 1:0.7, and sodium chlorite only.

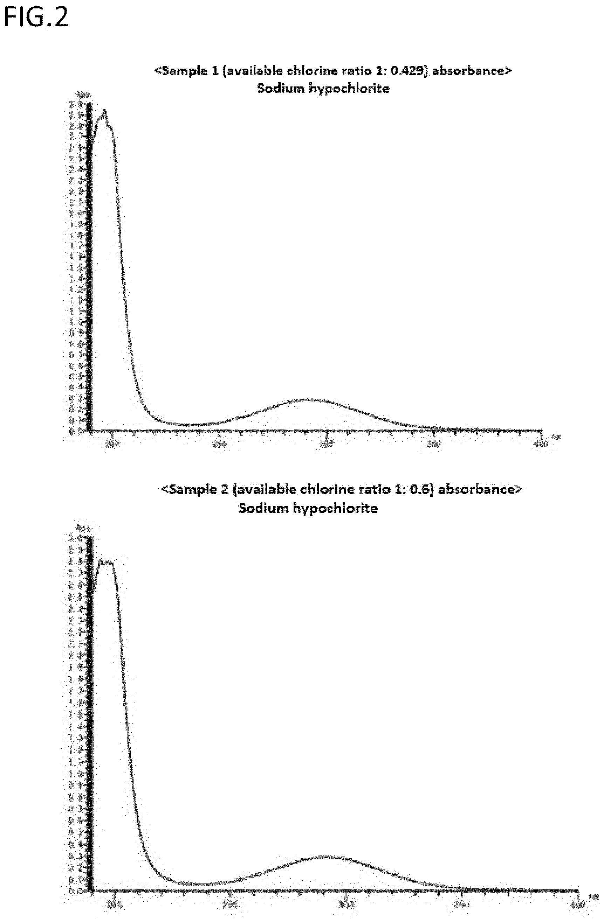

[Figure 2] Figure **2** shows absorbance of sample 1 and sample 2 in (Mixed solution of recovery liquid A and recovery liquid B (sodium hypochlorite specification)).

[Description of Embodiments]

The present invention is described hereinafter while providing the best mode of the present disclosure. Throughout the entire specification, a singular expression should be understood as encompassing the concept thereof in the plural form, unless specifically noted otherwise. Thus, singular articles (e.g., "a", "an", "the" and the like in case of English) should also be understood as encompassing the concept thereof in the plural form, unless specifically noted otherwise. The terms used herein should be understood as being used in the meaning that is commonly used in the art, unless specifically noted otherwise. Therefore, unless defined otherwise, all terminologies and science and technology terms that are used herein have the same meaning as the general understanding of those skilled in the art to which the present invention pertains. In case of a contradiction, the present specification (including the definitions) takes precedence.

(Definitions of the terms)

The terms used herein are described hereinafter.

As used herein, "solution comprising a hypochlorite with deteriorated quality" refers to a solution of sodium hypochlorite, in which chlorine has degraded during storage and chloride ions and chlorate ions have been generated. As used herein, hypochlorite may be abbreviated as "hypo."

As used herein, "low salt grade sodium hypochlorite solution" refers to a sodium hypochlorite solution with a reduced amount of salt. Chlorate ions in 12% low salt grade sodium hypochlorite solution is 5000 to 6000 ppm and at most 12000 ppm or less.

As used herein, "general grade sodium hypochlorite solution" refers to a sodium hypochlorite solution without reduction in the amount of salt. About 14000 to 26000 ppm of chlorate ions is generated in a 12% general grade sodium hypochlorite solution upon distribution.

As used herein, "available chlorine" or "available chlorine concentration" refers to a concentration of chlorine that is effective for bleaching action, contained in a disinfectant such as chlorinated lime. The available chlorine concentration can be quantified, for example, by redox titration ($I_2 + 2Na_2S_2O_3 \rightarrow 2NaI + Na_2S_4O_6$ formula (2)) on released iodine by adding potassium iodide to sodium hypochlorite in a sample according to $Cl_2 + KI \rightarrow I_2 + KCl$ formula (1) with sodium thiosulfate.

As used herein, "free chlorine", "free chlorine concentration", or "free residual chlorine concentration" is a value that is measured according to Appendix 3 (hereinafter, colorimetry (DPD indicator)) in "Testing method for free residual chlorine and bound chlorine specified by the Minister of Health, Labour and Welfare based on the stipulation of Article 17(2) of the Japanese regulation of the Water Supply Act", and is a value obtained by oxidation of a DPD indicator.

As used herein, "$SO_4$ based component" refers to a component derived from sulfuric acid such as sulfuric acid or sulfate.

As used herein, "warm air drying" is performed by sending warm air into the chamber at a volume of 1.9 $m^3$/s under an internal environmental temperature of 50 to 60°C and internal humidity of 10% or less as the drying conditions.

As used herein, "chlorine oxide" refers to any oxide of chlorine. Examples thereof include hypochlorous acid, chlorous acid, chloric acid, perchloric acid, salts thereof, and the like, as well as dichlorine heptoxide, dichlorine hexoxide, dichlorine trioxide, chlorine dioxide, dichlorine monoxide, and the like.

As used herein, "high grade chlorinated lime" satisfies the standards specified for high grade chlorinated lime in Japan's Specifications and Standards for Food Additives, 8th Edition. Specifically, the following is satisfied:

(1) comprises available chlorine at 60.0% or more;
(2) has a chlorine odor;
(3) when 5 ml of water is added to 0.5 g of the solid and shaken and red litmus paper is immersed therein, the litmus paper changes its color to blue and then loses its color; and
(4) when 2 ml of acetic acid (1 → 4) is added to 0.1 g of the solid, the solid dissolves while generating gas, and a solution prepared by adding 5 ml of water thereto and filtrate exhibits a reaction of calcium salt.

As used herein, alkalinity (T. AL) is 1 when, to measure the alkalinity in a sample, the sample is titrated with 0.1 mol/L hydrochloric acid-standard acid solution until the pH reaches 4.0 and 0.1 mol/L hydrochloric acid required for 100 g of the sample to reach a pH of 4.0 is 1 mL. A pH of 4.0 is a second neutralization point of sodium carbonate. Since high grade chlorinated lime has a broad specification so that the proportion of pH adjusting agent and the like generally varies by manufacturer for chlorine oxide, T. AL is often not described in the specification. However, chlorine is contained in the high grade chlorinated lime, so that it shows a high T. AL value and is highly alkaline.

As used herein, "non-calcium inorganic alkaline agent" refers to a (drug) agent having an inorganic alkaline substance with a cation other than calcium. It is understood that any agent having an inorganic alkaline substance with a cation other than calcium can be used. Examples thereof include, but are not limited to, sodium carbonate, disodium hydrogen phosphate, sodium sulfate, and sodium hydroxide.

As used herein, "inorganic alkaline agent with a valency of two or greater" refers to a non-calcium inorganic alkaline agent with a valency of two or greater. A sodium-containing alkaline agent is preferred, but the agent is not limited thereto. Preferred inorganic alkaline agents with a valency of two or greater advantageously have the ability to adjust the pH to 10 or less. This is because the pH of high grade chlorinated lime can be reduced. Examples thereof include, but are not limited to, sodium carbonate, disodium hydrogen phosphate, and sodium sulfate. However, the present invention has demonstrated that an inorganic alkaline agent with a valency of less than two, such as sodium hydroxide, can also be used in some cases. It can be advantageous to add sodium sulfate or the like. Although not wishing to be bound by any theory, addition of sodium sulfate is preferable because this can facilitate solidification of a precipitation layer after a reaction and suppress floating, so that operability is improved. The amount of sodium sulfate added is generally about 0.1 with respect to the amount of other inorganic alkaline agents added of 1. Other amount can be used, as long as the objective can be achieved. Examples thereof include 10% or less, preferably 5% or less, 2% or less, 1% or less, and the like.

As used herein, "recovery liquid A" indicates a recovery liquid that has recovered gas generated in the first reaction step in the method of treating a solution comprising a hypochlorite of the invention. As used herein, "recovery liquid B" indicates a recovery liquid that has recovered gas generated in the second reaction step in the method of treating a solution comprising a hypochlorite of the invention.

(Description of preferred embodiments)

Preferred embodiments of the present invention are described hereinafter. It is understood that the embodiments provided hereinafter are provided to facilitate understanding of the present invention, so that the scope of the present invention should not be limited by the following description. Thus, it is apparent that those skilled in the art can refer to the description herein to make appropriate modifications within the scope of the present invention. It is also understood that the following embodiments of the present invention can be used alone or in combination.

In one aspect, the present invention provides a dry solid comprising a hypochlorite and a chlorite. Examples of the hypochlorite include alkaline metal salts and alkaline earth metal salts of hypochlorous acid such as sodium salt, potassium salt, calcium salt, and magnesium salt. Examples of the chlorite include alkaline metal salts and alkaline earth metal salts of chlorous acid such as sodium salt, potassium salt, calcium salt, magnesium salt, and the like. The composition of components of the solid disinfectant of the invention does not change when stored at ordinary temperature. When a calcium salt is used, the disinfectant can be in compliance with the specification for the food additive, high grade chlorinated lime. Salts such as potassium salt and magnesium salt can be obtained by using an alkaline solution of a corresponding alkaline metal or alkaline earth metal in a recovery liquid or by exchanging a sodium salt or calcium slat with a corresponding metal.

In one embodiment, the solid is in a dry granulate.

In one embodiment, the solid comprises calcium hypochlorite.

In one embodiment, the solid meets the standards specified for high grade chlorinated lime in Japan's Specifications and Standards for Food Additives, 8th Edition. Specifically, the solid has the following properties:

(1) comprises available chlorine at 60.0% or more;
(2) has a chlorine odor;
(3) when 5 ml of water is added to 0.5 g of the solid and shaken and red litmus paper is immersed therein, the litmus paper changes its color to blue and then loses its color; and
(4) when 2 ml of acetic acid (1 → 4) is added to 0.1 g of the solid, the solid dissolves while generating gas, and a solution prepared by adding 5 ml of water thereto and filtrate exhibits a reaction of calcium salt.

In one embodiment, the solid comprises an $SO_4$ based component in the range from the detection limit or higher and 8100 ppm or lower. The $SO_4$ based component can be at 8000 ppm or less, 7000 ppm or less, 6000 ppm or less, 5000 ppm or less, 4000 ppm or less, 3000 ppm or less, 2000 ppm or less, or 1000 ppm or less. The $SO_4$ based component can be at 100 ppm or greater, 200 ppm or greater, 300 ppm or greater, 400 ppm or greater, 500 ppm or greater, 600 ppm or greater, 700 ppm or greater, 800 ppm or greater, 900 ppm or greater, 1000 ppm or greater, or 1100 ppm or greater. Such an $SO_4$ based component is entrainment of sulfuric acid contained in a reaction tank. The amount of $SO_4$ based component can be one of the indicators indicating that a chlorine oxide was prepared by the present invention.

In one embodiment, a ratio of the hypochlorite to the chlorite in the solid is 1:5 to 25. This ratio is a value for a dried and solidified mixed solution obtained by preliminary drying each of recovery liquid A and recovery liquid B used for obtaining the solid to reach a moisture content of about 20% and mixing the solutions. Since free residual chlorine concentration can be considered equal to available chlorine concentration in a solid product, the concentration of hypochlorite ion can be derived from the concentration of free residual chlorine. The concentration of chlorite ion can be measured by ion chromatography. The ratio of hypochlorite to chlorites is derived from the ratio of each ion. The ratio of the hypochlorite to the chlorite in the solid can be 1:9 to 25. The ratio of the hypochlorite to the chlorite in the solid can be 1:5 to 9. The ratio of the hypochlorite to the chlorite in the solid can be such that the chlorite is any numerical value or within any numerical range within the range of 5 to 25, given that the hypochlorite is 1. For example, given that the hyphochlorite is 1, the chlorite can be 5, 6, 7, 8, 9, 10, 11, 12, 13, 14, 15, 16, 17, 18, 19, 20, 21, 22, 23, 24, or 25, or any numerical value or within any numerical range therebetween. In one specific example, the ratio of the hypochlorite to the chlorite in the solid is 1:5.53 to 23.59. The ratio of the hypochlorite to the chlorite in the solid can be 1:8.92 to 23.59. The ratio of the hypochlorite to the chlorite in the solid can be 1:5.53 to 8.92. A dry solid with such a ratio is highly pure, has long term storability, has a high bactericidal effect, and has low chlorine odor. For calcium salt, the specification and standard for high grade chlorinated lime is met. A solid with a value other than those in the range described above can also be prepared as needed.

In one embodiment, an available chlorine concentration in the solid is within a range of 600,000 ppm to 900,000 ppm, and a free residual chlorine concentration is within a range of 900 ppm to 60,000 ppm. The available chlorine concentration in the solid can be any numerical value or within any numerical range within the range of 600,000 ppm to 900,000 ppm. The available chlorine concentration in the solid can be 600,000 ppm, 650,000 ppm, 700,000 ppm, 750,000 ppm, 800,000 ppm, 850,000 ppm, or 900,000 ppm, or within a range of any combination of these numerical values. The free residual chlorine concentration in the solid can be any numerical value or within any numerical range within a range of 900 ppm to 60,000 ppm. The free residual chlorine concentration in the solid can be 900 ppm, 1,000 ppm, 2,000 ppm, 3,000 ppm, 4,000 ppm, 5,000 ppm, 6,000 ppm, 7,000 ppm, 8,000 ppm, 9,000 ppm, 10,000 ppm, 15,000 ppm, 20,000 ppm, 25,000 ppm, 30,000 ppm, 35,000 ppm, 40,000 ppm, 45,000 ppm, 50,000 ppm, 55,000 ppm, or 60,000 ppm, or within a range of any combination of these numerical values. In one specific example, an available chlorine concentration in the solid is within a range of 606,811 ppm to 881,677 ppm, and a free residual chlorine concentration is within a range of 901 ppm to 58,728 ppm. The available chlorine concentration in the solid can be within a range of 606,811 ppm to 881,677 ppm. The available chlorine concentration in the solid can be 606,811 ppm, 616,877 ppm, 632,513 ppm, 647,265 ppm, 781,019 ppm, 782,210 ppm, 824,064 ppm, or 881,667 ppm. In one specific example, the free residual chlorine concentration in the solid can be within a range of 901 ppm to 58728 ppm. The free residual chlorine concentration in the solid can be 901 ppm, 2,145 ppm, 2,625 ppm, 20,785 ppm, 49,314 ppm, 55,916 ppm, or 58,728 ppm. A dry solid with such a concentration is highly pure, has long term storability, has a high bactericidal effect, and has low chlorine odor. For calcium salt, the specification standard for high grade chlorinated lime is met. A solid with a value other than those in the range described above can also be prepared as needed.

In one aspect, the present invention provides a liquid obtained by dissolving the dry solid described above. Examples of solvent used for dissolving the solid include water, alcohol, ether, and the like. Examples of water include any water, such as tap water, well water, sea water, deionized water, and purified water.

In one embodiment, a ratio of hypochlorite ions to chlorite ions is 1: 7 to 35, when diluted with water so that the available chlorine concentration would be 1%. Given that hypochlorite ions are 1, the chlorite ions can be 7, 8, 9, 10, 11, 12, 13, 14, 15, 16, 17, 18, 19, 20, 21, 22, 23, 24, 25, 26, 27, 28, 29, 30, 31, 32, 33, 34, or 35 or any numerical value or within any numerical range therebetween. In one specific example, a ratio of hypochlorite ions to chlorite ions is 1: 7.16 to 34.36, when diluted with water so that the available chlorine concentration would be 1%. A free residual chlorine concentration is within a range of 150 ppm to 900 ppm, when diluted with water so that the available chlorine concentration would be 1%. The free residual chlorine concentration can be 150 ppm, 200 ppm, 250 ppm, 300 ppm, 350 ppm, 400 ppm, 450 ppm, 500 ppm, 550 ppm, 600 ppm, 650 ppm, 700 ppm, 750 ppm, 800 ppm, 850 ppm, or 900 ppm, or any numerical value or within any numerical range between these numerical values. In one specific example, a free residual chlorine concentration is within a range of 187.07 ppm to 836.70 ppm, when diluted with water so that the available chlorine concentration would be 1%. Since free residual chlorine can be degraded during handling, the ratio of hypochlorite ions to chlorite ions can change depending on the degree of dilution. A liquid having such a ratio is highly pure, has a high bactericidal effect, and has low chlorine odor.

A ratio of hypochlorite ions to chlorite ions is 1:6 to 30, when diluted with water so that the available chlorine concentration would be 6%. Given that the hypochlorite ions is 1, the chlorite ions can be 6, 7, 8, 9, 10, 11, 12, 13, 14, 15, 16, 17, 18, 19, 20, 21, 22, 23, 24, 25, 26, 27, 28, 29, or 30, or any numerical value or within any numerical range therebetween. In one specific example, a ratio of hypochlorite ions to chlorite ions is 1:6.31 to 29.54, when diluted with water so that the available chlorine concentration would be 6%. A free residual chlorine concentration is within a range of 1,000 ppm to 6,000 ppm, when diluted with water so that the available chlorine concentration would be 6%. The free residual chlorine concentration can be 1,000 ppm, 1,500 ppm, 2,000 ppm, 2,500 ppm, 3,000 ppm, 3,500 ppm, 4,000 ppm, 4,500 ppm, 5,000 ppm, 5,500 ppm, or 6,000 ppm or any numerical value or within any numerical range between these numerical values. In one specific example, a free residual chlorine concentration is within a range of 1296.01 ppm to 5624.20 ppm, when diluted with water so that the available chlorine concentration would be 6%. Since free residual chlorine can be degraded during handling, the ratio of hypochlorite ions to chlorite ions can change depending on the degree of dilution. A liquid having such a ratio is highly pure, has a high bactericidal effect, and has low chlorine odor.

A ratio of hypochlorite ions to chlorite ions is 1:6 to 30, when diluted with water so that the available chlorine concentration would be 12%. Given that the hypochlorite ions is 1, the chlorite ions can be 6, 7, 8, 9, 10, 11, 12, 13, 14, 15, 16, 17, 18, 19, 20, 21, 22, 23, 24, 25, 26, 27, 28, 29, or 30, or any numerical value or within any numerical range therebetween. In one specific example, a ratio of hypochlorite ions to chlorite ions is 1:6.39 to 28.16, when diluted with water so that the available chlorine concentration would be 12%. A free residual chlorine concentration is within a range of 2,500 ppm to 12,000 ppm, when diluted with water so that the available chlorine concentration would be 12%. The free residual chlorine concentration can be 2,500 ppm, 3,000 ppm, 3,500 ppm, 4,000 ppm, 4,500 ppm, 5,000 ppm, 6,000 ppm, 6,500 ppm, 7,000 ppm, 7,500 ppm, 8,000 ppm, 8,500 ppm, 9,000 ppm, 9,500 ppm, 10,000 ppm, 10,500 ppm, 11,000 ppm, 11,500 ppm, or 12,000 ppm, or any numerical value or within any numerical range between these numerical values. In one specific example, a free residual chlorine concentration is within a range of 2736.70 ppm to 11378.81 ppm, when diluted with water so that the available chlorine concentration would be 12%. Since free residual chlorine can be degraded during handling, the ratio of hypochlorite ions to chlorite ions can change depending on the degree of dilution. A liquid having such a ratio is highly pure, has a high bactericidal effect, and has low chlorine odor.

In one aspect, the present invention provides a method of manufacturing a dry solid comprising a hypochlorite and a chlorite, comprising: a step of preparing a solution comprising a hypochlorite ion, a chlorate ion, and a chloride ion; a first reaction step for adding sulfuric acid to the solution to generate chlorine gas; a step of reacting the generated chlorine gas with sodium hydroxide or calcium hydroxide and recovering a reaction product as a hypochlorite ion in recovery liquid A; a second reaction step for adding sulfuric acid to a reaction mother liquor after the first reaction step at a concentration that is higher than that in the first reaction step to generate chlorine dioxide gas; a step of reacting the generated chlorine dioxide gas with sodium hydroxide and hydrogen peroxide and recovering a reaction product as a chlorite ion in recovery liquid B; a step of mixing recovery liquid A with recovery liquid B; and a step of drying and solidifying the resulting mixture. The method regenerates sodium hypochlorite with deteriorated quality to newly provide a useful disinfectant. The composition of components of the solid disinfectant manufactured and dried by this method does not change when stored at ordinary temperature. When a calcium salt is used, the disinfectant can be in compliance with the Japanese specification for the food additive, high grade chlorinated lime. The dry solid completed by the present invention has an advantage in terms of having lower chlorine odor, less burden on users, and easier to use. Even if a complex regeneration method is used, the manufacturing cost can be absorbed and the product can be commercially sold.

In one embodiment, the recovery liquid A comprises calcium hydroxide.

In one embodiment, the method further comprises a step of adding hydrogen peroxide to a reaction mother liquor after a first reaction. Generation of chlorine gas can be suppressed by the step of adding hydrogen peroxide.

In one embodiment, an available chlorine concentration of the recovery liquid B is within a range of 9.6 to 33.95, given that an available chlorine concentration of the recovery liquid A is 1, in the step of mixing the recovery liquid A with the recovery liquid B. If the available chlorine concentration of recovery liquid A is 1, the available chlorine concentration of recovery liquid B can be 9.6 or more, 9.7 or more, 9.8 or more, 9.9 or more, 10.0 or more, 11 or more, 12 or more, 13 or more, 14 or more, 15 or more, 16 or more, 17 or more, 18 or more, 19 or more, 20 or more, 21 or more, 22 or more, 23 or more, 24 or more, 25 or more, 26 or more, 27 or more, 28 or more, 29 or more, 30 or more, 31 or more, 32 or more, or 33 or more, or within any range of numerical values of 33.95 or less, 33.9 or less, 33.8 or less, 33.7 or less, 33.6 or less, 33.5 or less, 33.4 or less, 33.3 or less, 33.2 or less, 33.1 or less, 33 or less, 32 or less, 31 or less, 30 or less, 29 or less, 28 or less, 27 or less, 26 or less, 25 or less, 24 or less, 23 or less, 22 or less, 21 or less, 20 or less, 19 or less, 18 or less, 17 or less, 16 or less, 15 or less, 14 or less, 13 or less, 12 or less, 11 or less, 10 or less, 9.9 or less, 9.8 or less, or 9.7 or less. If the available chlorine concentration of recovery liquid A is 1, the available chlorine concentration of recovery liquid B can be 9.6, 9.7, 9.8, 9.9, 10, 11, 12, 13, 14, 15, 16, 17, 18, 19, 20, 21, 22, 23, 24, 25, 26, 27, 28, 29, 30, 31, 32, or 33. Preferably, if the available chlorine concentration of recovery liquid A is 1, the available chlorine concentration of recovery liquid B is 20. For available chlorine ratios with the available chlorine concentration of recovery liquid B of less than 9.6, it is understood that the available chlorine would be less than 60% such that the specification for high grade chlorinated would not be met. Thus, this available chlorine ratio is the lower limit. If the available chlorine ratio for recovery liquid B exceeds 33.95, the specification for free residual chlorine and calcium would not be met.

In one embodiment, the recovery liquid A and the recovery liquid B are each slurried and mixed in the step of mixing the recovery liquid A with the recovery liquid B. The overall operation time can be shortened and loss of available chlorine or change in the composition can be prevented more when the solutions are slurried then mixed and dried, relative to cases without slurrying.

In one embodiment, the step of mixing the recovery liquid A with the recovery liquid B comprises a step of preliminary drying the recovery liquid A, forming a granulation nucleus, slurrying the recovery liquid B, and adding dried recovery liquid A to the slurried recovery liquid B. If both recovery liquids are dried in advance to a certain degree, the operability and stability of each slurry are improved.

In one embodiment, the step of drying and solidifying comprises a step of drying with warm air for 20 to 30 minutes. The drying conditions can be an internal environmental temperature of 50 to 60°C and internal humidity of 10% or less. Warm air is sent in at a volume of 1.9 $m^3$/s.

In one embodiment, the step of drying and solidifying comprises reducing moisture content of each of the recovery liquid A and the recovery liquid B to 20% or less. In one embodiment, the moisture content can be 26% or less, 25% or less, 24% or less, 23% or less, 22% or less, 21% or less, 20% or less, 19% or less, 18% or less, 17% or less, 16% or less, 15% or less, 14% or less, 13% or less, 12% or less, 11% or less, or 10% or less. If the moisture content is 26% or greater, the free residual chlorine (hypochlorite ions) and chlorite ions in recovery liquid A react to reduce the available chlorine concentration, increase chlorate ions, and reduce the purity and dry ratio. If the water content exceeds 30%, self-degradation of free residual chlorine (hypochlorite ions) becomes significant such that the free residual chlorine (hypochlorite ions) in particular decreases as well as the purity and dry ratio. It is preferable that the water content is reduced to 20% or less, but a suitable water content can be selected while taking operability or cost into consideration.

In one aspect, the present invention provides a method of manufacturing a new disinfectant from a solution comprising a hypochlorite ion, a chlorate ion, and a chloride ion; comprising: a step of quantifying the concentration of a hypochlorite ion, a chlorate ion, and a chloride ion in the solution; a first reaction step for adding sulfuric acid to the solution to generate chlorine gas; a step of reacting the generated chlorine gas with sodium hydroxide or calcium hydroxide and recovering a reaction product as a hypochlorite ion in recovery liquid A; a second reaction step for adding sulfuric acid to a reaction mother liquor after the first reaction step at a concentration that is higher than that in the first reaction step to generate chlorine dioxide gas; a step of reacting the generated chlorine dioxide gas with sodium hydroxide and hydrogen peroxide and recovering a reaction product as a chlorite ion in recovery liquid B; and a step of mixing the recovery liquid A with the recovery liquid B to obtain a new disinfectant. A mixture of recovery liquid A and recovery liquid B can be used as a disinfectant. This method regenerates sodium hypochlorite with deteriorated quality to newly provide a useful disinfectant. When a sodium salt is used, the disinfectant manufactured by this method can be in compliance with the specification and standard for sodium hypochlorite for a food additive.

In one embodiment, the solution comprising a hypochlorite ion, a chlorate ion, and a chloride ion is a solution comprising a hypochlorite with deteriorated quality.

In one embodiment, the solution comprising a hypochlorite with deteriorated quality is derived from a low salt grade sodium hypochlorite solution.

In one embodiment, the solution comprising a hypochlorite with deteriorated quality is derived from a general grade sodium hypochlorite solution.

It is important to control the contaminated chlorine dioxide gas in recovery liquid A by adjusting the sulfuric acid concentration in the first reaction. It is important to prevent degradation of chlorate ions for the acidity in the reaction mother liquor during the first reaction.

Sulfuric acid during the second reaction is dependent on the sulfuric acid concentration used and the sulfuric acid concentration in the reaction mother liquor during the second reaction. A higher sulfuric acid concentration used during the second reaction is preferable. In the second reaction, it is not just the sulfuric acid concentration in the reaction mother liquor that is important.

In one embodiment, the solution comprising a hypochlorite with deteriorated quality is derived from a low salt grade sodium hypochlorite solution, the disinfectant is a solid product, a sulfuric acid concentration in a reaction mother liquor in the first reaction step is 4.00 to 6.37%, a sulfuric acid concentration in a reaction mother liquor in the second reaction step is 30.00 to 40.00%, and a sulfuric acid concentration used in the second reaction step is 50.0 w/w% to 70.0 w/w%. In further embodiment, if the disinfectant is a solid product, the sulfuric acid concentration in a reaction mother liquor in the first reaction step can be 4.00 %, 4.10 %, 4.20 %, 4.30 %, 4.40 %, 4.50 %, 4.60 %, 4.70 %, 4.80 %, 4.90 %, 5.00 %, 5.10 %, 5.20 %, 5.30 %, 5.40 %, 5.50 %, 5.60 %, 5.70 %, 5.80 %, 5.90 %, 6.00 %, 6.10 %, 6.20 %, 6.30 %, or 6.37 %, and the sulfuric acid concentration in a reaction mother liquor in the second reaction step is 30.0 %, 31.0 %, 32.0 %, 33.0 %, 34.0 %, 35.0 %, 36.0 %, 37.0 %, 38.0 %, 39.0 %, or 40.0 %, and the sulfuric acid concentration used in the second reaction step is 50.0 w/w%, 51.0 w/w%, 52.0 w/w%, 53.0 w/w%, 54.0 w/w%, 55.0 w/w%, 56.0 w/w%, 57.0 w/w%, 58.0 w/w%, 59.0 w/w%, 60.0 w/w%, 61.0 w/w%, 62.0 w/w%, 63.0 w/w%, 64.0 w/w%, 65.0 w/w%, 66.0 w/w%, 67.0 w/w%, 68.0 w/w%, 69.0 w/w%, or 70.0 w/w%.

In one embodiment, the solution comprising a hypochlorite with deteriorated quality is derived from a low salt grade sodium hypochlorite solution, the disinfectant is a liquid product, a sulfuric acid concentration in a reaction mother liquor in the first reaction step is 4.00 to 6.37%, a sulfuric acid concentration in a reaction mother liquor in the second reaction step is 30.00 to 59.04%, and a sulfuric acid concentration used in the second reaction step is 50.0 w/w% to 70.0 w/w%. In further embodiment, the disinfectant is a liquid product, and the sulfuric acid concentration in a reaction mother liquor in the first reaction step is 4.00, 4.10 %, 4.20 %, 4.30 %, 4.40 %, 4.50 %, 4.60 %, 4.70 %, 4.80 %, 4.90 %, 5.00 %, 5.10 %, 5.20 %, 5.30 %, 5.40 %, 5.50 %, 5.60 %, 5.70 %, 5.80 %, 5.90 %, 6.00 %, 6.10 %, 6.20 %, 6.30 %, or 6.37 %, the sulfuric acid concentration in a reaction mother liquor in the second reaction step is 30.0 %, 31.0 %, 32.0 %, 33.0 %, 34.0 %, 35.0 %, 36.0 %, 37.0 %, 38.0 %, 39.0 %, 40.0 %, 41.0 %, 42.0 %, 43.0 %, 44.0 %, 45.0 %, 46.0 %, 47.0 %, 48.0 %, 49.0 %, 50.0 %, 51.0 %, 52.0 %, 53.0 %, 54.0 %, 55.0 %, 56.0 %, 57.0 %, 58.0 %, 59.0 %, or 59.04 %, and the sulfuric acid concentration used in the second reaction step is 50.0 w/w%, 51.0 w/w%, 52.0 w/w%, 53.0 w/w%, 54.0 w/w%, 55.0 w/w%, 56.0 w/w%, 57.0 w/w%, 58.0 w/w%, 59.0 w/w%, 60.0 w/w%, 61.0 w/w%, 62.0 w/w%, 63.0 w/w%, 64.0 w/w%, 65.0 w/w%, 66.0 w/w%, 67.0 w/w%, 68.0 w/w%, 69.0 w/w%, or 70.0 w/w%.

In one embodiment, the solution comprising a hypochlorite with deteriorated quality is derived from a general grade sodium hypochlorite solution, and a sulfuric acid concentration in a reaction mother liquor in the first reaction step is 4.00 to 4.50%, a sulfuric acid concentration in a reaction mother liquor in the second reaction step is 25.00 to 30.00%, and a sulfuric acid concentration used in the second reaction step is 65 w/w%. In further embodiment, the sulfuric acid concentration in a reaction mother liquor in the first reaction step is 4.00 %, 4.10 %, 4.20 %, 4.30 %, 4.40 %, or 4.50 %, the sulfuric acid concentration in a reaction mother liquor in the second reaction step is 25.0 %, 26.0 %, 27.0 %, 28.0 %, 29.0 %, or 30.00 %, and the sulfuric acid concentration used in the second reaction step is 65 w/w%.

In one embodiment, the method satisfies the following formula:

$$(1)\ Y = -1.2676X + 9.84393;$$

and

$$(2)\ X \le 4;$$

wherein a chloride concentration in a raw material is X%, and a sulfuric acid concentration in a reaction mother liquor is Y% in a first reaction.

This formula is derived by determining the upper limit as chlorate ions in the raw material not degrading or increasing and the condition as chlorate ions increasing to 110% or greater to take into consideration the safety factor, and by taking into consideration that the concentration of sulfuric acid which can be added decreases when the chloride concentration is the raw material is high at this time.

In one embodiment, the recovery liquid A comprises sodium hydroxide or calcium hydroxide.

In one embodiment, the recovery liquid B comprises sodium hydroxide and hydrogen peroxide.

A reaction step can be performed while blowing air. Air is useful in preventing chlorine gas or chlorine dioxide gas generated in a reaction tank from returning to a solution or progression of generation of a reverse reaction.

In one embodiment, the first reaction step is performed while blowing air.

In one embodiment, the second reaction step is performed while blowing air.

In one embodiment, an intermediate trapping vessel comprising hydrogen peroxide is provided between a reaction tank and a recovery vessel comprising recovery liquid B. The intermediate trapping vessel prevents chlorine gas from contaminating recovery liquid B.

In one embodiment, the method further comprising a step of adding hydrogen peroxide to a reaction mother liquor after a first reaction. Generation of chlorine gas is suppressed by adding hydrogen peroxide during the second reaction.

In one embodiment, an available chlorine concentration of recovery liquid B is 0.43 to 0.6, given that an available chlorine concentration of recovery liquid A is 1, in the step of mixing the recovery liquid A with recovery liquid B. In one embodiment, given that an available chlorine concentration of recovery liquid A is 1, the available chlorine concentration of recovery liquid B can be 0.43 or more, 0.44 or more, 0.45 or more, 0.46 or more, 0.47 or more, 0.48 or more, 0.49 or more, 0.5 or more, 0.51 or more, 0.52 or more, 0.53 or more, 0.52 or more, 0.53 or more, 0.54 or more, 0.55 or more, 0.56 or more, 0.57 or more, 0.58 or more, or 0.59 or more, and can be in any range of numerical values of 0.6 or less, 0.59 or less, 0.58 or less, 0.57 or less, 0.56 or less, 0.55 or less, 0.54 or less, 0.53 or less, 0.52 or less, 0.51 or less, 0.50 or less, 0.49 or less, 0.48 or less, 0.47 or less, 0.46 or less, 0.45 or less, or 0.44 or less. In such a range, a mixture of recovery liquid A and recovery liquid B can meet the specification of sodium hypochlorite for a food additive.

In one embodiment, the disinfectant comprises sodium hypochlorite.

In one embodiment, the disinfectant is a product meeting the specification and standard of sodium hypochlorite for food additive. Specifically, the disinfectant has the following properties:

(1) comprises available chlorine at 4.0% or more;
(2) has a chlorine odor;
(3) exhibits a reaction of a sodium salt and a reaction of a hypochlorite;
(4) a solution preparing by adding 100 ml of phosphate buffer (pH 8) to 4 ml of an aqueous solution (1 → 25) of this product has a maximum absorbance at a wavelength of 291 to 294 nm; and
(5) red litmus paper, when immersed in this product, changes its color to blue and then loses its color.

In one embodiment, the disinfectant comprises an $SO_4$ based component at a detection limit or higher and 8100 ppm or lower. The $SO_4$ based component can be at 8000 ppm or lower, 7000 ppm or lower, 6000 ppm or lower, 5000 ppm or lower, 4000 ppm or lower, 3000 ppm or lower, 2000 ppm or lower, or 1000 ppm or lower. The $SO_4$ based component can be at 100 ppm or higher, 200 ppm or higher, 300 ppm or higher, 400 ppm or higher, 500 ppm or higher, 600 ppm or higher, 700 ppm or higher, 800 ppm or higher, 900 ppm or higher, 1000 ppm or higher, or 1100 ppm or higher. Such an $SO_4$ based component is entrainment of sulfuric acid contained in a reaction tank. The amount of $SO_4$ based component can be one of the indicators indicating that a chlorine oxide was prepared by the present invention.

In one embodiment, a ratio of hypochlorite ions to chlorite ions in the disinfectant is 1:0.24 to 0.3. For a liquid product, it is difficult to identify a substance and find the concentration from the free residual chlorine concentration. Thus, chlorite ions derived from recovery liquid B were determined by ion chromatography and converted into available chlorine, and subtracted from the available chlorine of the whole. Furthermore, the remaining available chlorine was determined as the available chlorine derived from recovery liquid A and multiplied by a coefficient to obtain hypochlorite ions for the final ion ratio. For the ratio of hypochlorite ions to chlorite ions in the disinfectant, given that a chlorite is 1, a chlorite can be any numerical value or within any numerical range between 0.24 to 0.3. For example, given that a hypochlorite is 1, a chlorite is 0.24, 0.25, 0.26, 0.27, 0.28, 0.29, or 0.30. A disinfectant with such a ratio is highly pure, has a high bactericidal effect, and has low chlorine odor.

In one embodiment, an available chlorine concentration in the disinfectant is about 60,000 ppm, and a free residual chlorine concentration is within a range of about 60,000 ppm. The available chlorine concentration in the disinfectant can be 55,000ppm, 56,000 ppm, 57,000 ppm, 58,000 ppm, 59,000 ppm, 60,000 ppm, 61,000 ppm, 62,000 ppm, 63,000 ppm, 64,000 ppm, or 65,000 ppm, or within a range of any combination of these numerical values. In a specific example, the available chlorine concentration in the disinfectant is 60,114 ppm or 60,814 ppm. The free residual chlorine concentration in the disinfectant can be 55,000 ppm, 56,000 ppm, 57,000 ppm, 58,000 ppm, 59,000 ppm, 60,000 ppm, 61,000 ppm, 62,000 ppm, 63,000 ppm, 64,000 ppm, or 65, 000 ppm, or within a range of any combination of these numerical values. In one specific example, the concentration is 58,157 ppm or 59,380 ppm. A disinfectant with such a concentration is highly pure, has a high bactericidal effect, and has low chlorine odor.

In one aspect, the present invention provides a disinfectant manufactured by any of the methods described above.

In one embodiment, a ratio of hypochlorite ions to the chlorite ions is 1:0.24 to 0.3 in the disinfectant. For the ratio of hypochlorite ions to chlorite ions, given that a hypochlorite is 1, a chlorite can be any numerical value or within any numerical range between 0.24 and 0.3. For example, given that a hypochlorite is 1, a chlorite is 0.24, 0.25, 0.26, 0.27, 0.28, 0.29, or 0.30. A disinfectant with such a ratio is highly pure, has a high bactericidal effect, and has low chlorine odor.

In one embodiment, an available chlorine concentration in the disinfectant is about 60,000 ppm, and a free residual chlorine concentration is about 60,000 ppm. The available chlorine concentration in the disinfectant can be 55,000 ppm, 56,000 ppm, 57,000 ppm, 58,000 ppm, 59,000 ppm, 60,000 ppm, 61,000 ppm, 62,000 ppm, 63,000 ppm, 64,000 ppm, or 65,000 ppm, or within a range of any combination of these numerical values. In a specific example, the concentration is 60,114 ppm or 60,814 ppm. The free residual chlorine concentration in the disinfectant can be 55,000 ppm, 56,000 ppm, 57,000 ppm, 58,000 ppm, 59,000 ppm, 60,000 ppm, 61,000 ppm, 62,000 ppm, 63,000 ppm, 64,000 ppm, or 65,000 ppm, or within a range of any combination of these numerical values. In a specific example, the concentration is 58,157 ppm or 59,380 ppm. A disinfectant with such a concentration is highly pure, has a high bactericidal effect, and has low chlorine odor.

In one embodiment, a liquid chlorine oxide manufactured by using the dry solid is provided, and the liquid chlorine oxide is produced by a method comprising: (a) a step of dissolving the dry solid into water to prepare a solution with an elevated pH; (b) a step of adding a non-calcium inorganic alkaline agent to the solution while maintaining a pH of the solution prepared in step (a) to allow a calcium salt to precipitate and form a solid/liquid mixed phase with a reduced calcium ion concentration in a liquid phase, comprising the liquid phase and a solid phase comprising a calcium salt; and (c) recovering only the liquid phase from the solid/liquid mixed phase formed in step (b) to obtain a liquid chlorine oxide. The pH in step (a) can be 10.0 or greater. The pH in step (b) can be 10.0 or greater. The method can be a method disclosed in Japanese patent No. 5931253. When the dry solid is liquefied and used, a calcium salt can remain as a residue, so that the end users in food processing are concerned with the issue of contamination of food products with a foreign matter. Further, processing of a large quantity of calcium salt is very labor-intensive. Such a liquid chlorine oxide is useful because calcium is removed. The liquid chlorine oxide also has an effect of not precipitating a calcium salt, even during longer-term storage. A product completed by this method is more advantageous than a sodium hypochlorite solution with the same concentration in terms of having lower chlorine odor, less burden on users, and easier to use. Even if high grade chlorinated lime, which is a raw material that can be stored for a long period of time, is used, chemical industrial products can be manufactured. In addition, a calcium salt is not precipitated from a product produced and distributed by this method and chlorine odor is reduced significantly. Therefore, end users can use the product with safety.

In one embodiment, the liquid or liquid chlorine oxide with a calcium concentration substantially at or below a detection limit is provided. Preferably, the calcium concentration in a solution containing chlorine oxide is 24 ppm or less. Alternatively, the calcium concentration in a chlorine oxide containing solution is more preferably 23 ppm or less, 22 ppm or less, 21 ppm or less, 20 ppm or less, 19 ppm or less, 18 ppm or less, 17 ppm or less, 16 ppm or less, 15 ppm or less, 14 ppm or less, 13 ppm or less, 12 ppm or less, 11 ppm or less, 10 ppm or less, 9 ppm or less, 8 ppm or less, 7 ppm or less, 6 ppm or less, 5 ppm or less, 4 ppm or less, 3 ppm or less, 2 ppm or less, 1 ppm or less, 0.9 ppm or less, 0.8 ppm or less, 0.7 ppm or less, 0.6 ppm or less, 0.5 ppm or less, 0.4 ppm or less, 0.3 ppm or less, 0.2 ppm or less, 0.1 ppm or less, or 0.01 ppm or less. If these concentrations are achieved, chlorine odor can be reduced to a level without a problem, preferably achieving a level where chlorine odor substantially cannot be sensed (minute chlorine odor (e.g., 0.1 ppm or less or the like)).

In one embodiment, use of the dry solid described above, the liquid described above, or the liquid or liquid chlorine oxide described above as a disinfectant is provided. In one embodiment, use of the dry solid described above, the liquid described above, the disinfectant described above, or the liquid or liquid chlorine oxide described above as a food additive is provided. In one embodiment, use of the dry solid described above, the liquid described above, the disinfectant described above, or the liquid or liquid chlorine oxide described above for disinfecting a food product is provided.

In one embodiment, a method of manufacturing a new agent from a solution comprising a hypochlorite ion, a chlorate ion, and a chloride ion is provided, and the method comprises: a step of determining a concentration of a hypochlorite ion, a chlorate ion, and a chloride ion in the solution; a first reaction step for adding sulfuric acid to the solution to generate chlorine gas; a step of reacting the generated chlorine gas with sodium hydroxide or calcium hydroxide and recovering a reaction product as a hypochlorite ion in recovery liquid A; a second reaction step for adding sulfuric acid to a reaction mother liquor after the first reaction step at a concentration that is higher than that in the first reaction step to generate chlorine dioxide gas; a step of reacting the generated chlorine dioxide gas with sodium hydroxide and hydrogen peroxide and recovering a reaction product as a chlorite ion in recovery liquid B; and a step of mixing the recovery liquid A with the recovery liquid B to obtain a new agent. This method can have one or more features from substituting the disinfectant specified above with an agent. In one embodiment, an agent manufactured by this method is provided. The agent can have one or more features from substituting the disinfectant specified above with an agent. Use of this agent as a disinfectant, as a food additive, and for disinfecting a food product can be provided.

Since a chlorine oxide produced by the present invention uses sodium hypochlorite with deteriorated quality as a raw material, the chlorine oxide can comprise various components in the raw material. During the stage of processing sodium hypochlorite with deteriorated quality, various reactions can take place to generate various components including various chlorine oxides. Therefore, recovery liquid A and recovery liquid B can contain a hypochlorite and a chlorite, respectively, as a primary component, but not all components can be identified. Thus, the disinfectant of the invention produced by mixing recovery liquid A and recovery liquid B meets the specification for sodium hypochlorite or the specification for high grade chlorinated lime, but can contain components that cannot be identified. Such components that cannot be identified can possibly contribute to the improvement in the bactericidal effect or stability. Thus, it is assumed that an effect is attained, which is different from a simple combination of a hypochlorite and a chlorite.

Reference documents cited herein, such as a scientific article, patent or patent application, are incorporated herein by reference in the same manner as the entire content thereof is specifically described.

The present invention has been described while presenting preferred embodiments to facilitate understanding. Hereinafter, the present invention is described based on the Examples. However, the aforementioned description and the following Examples are provided solely for exemplification, not for limiting the present invention. Thus, the scope of the present invention is not limited to the Embodiments or Examples that are specifically described herein. The scope of the present invention is limited solely by the scope of the claims.

[Examples]

The amount of sulfate ions was determined by the following testing method (ion chromatography).

Preparation of sample solution

A sample is diluted by adding water, and sulfate ions are adjusted to 0 to 40 mg/L.

Preparation of standard solution for calibration curves

Kanto Chemical Co., Ink's Anion mixed standard solution IV is used as the standard solution (1 mL of this solution contains about 40 $\mu$g of sulfate ion).

Measurement condition

Ion chromatography coupled with suppressed conductivity detector is used for measurements under the following conditions.

Filler: ethylenevinylbenzene-divinylbenzene polymer based anion exchange resin
Column tube: inner diameter of 4.0 mm and length of 250 mm Eluent: mixture of 12 mmol/L of sodium carbonate and 5 mmol/L of sodium bicarbonate

Column temperature: room temperature
Flow rate: 1.0 mL/min
Amount of sample solution injected: 250 μL

Calibration curve

250 μL of standard solution is each accurately injected into an ion chromatograph, and calibration curves for sulfate ions are created from the resulting peak area.

Quantification

The concentration (A) of sulfate ions in a sample solution is found from the calibration curves and the peak area obtained from the sample solution. The sulfate ion content (X) [mg/L] in the undiluted sample solution is calculated from the following equation.

$$(X) = A \times K$$

A: concentration of sulfate ion in the sample solution (mg/L)
K: dilution ratio as of preparation of the sample solution.

(Deterioration in quality of sodium hypochlorite)

12% sodium hypochlorite solutions distributed as a food additive are largely categorized into low sodium and general grades. The amount of chlorate ions generated for the low salt grade was about 20000 to 25000 ppm, and the amount of chlorate ions generated for the general grade was about 30000 to 42000 ppm. It is understood, as a theoretical value, that chlorate ions increase about 3500 ppm for each 1% decrease in available chlorine, but it was found that the initial value of chlorate ions is high in some cases, and the ions are generated more than the theoretical value in some cases, depending on the difference in the quality of sodium hypochlorite.

The amount of chlorate ions generated when acid was added to 12% sodium hypochlorite solution to forcibly eliminate available chlorine was about 58000 to 66000 ppm. Thus, it was found that chlorate ions are not generated beyond this value from the 12% sodium hypochlorite solution.

[Table 1]

| Storage temperature: 40°C <br> <Low salt grade> unit: ppm | | | | | | |
|---|---|---|---|---|---|---|
| | | D+0 | D+10 | D+20 | D+30 | |
| Tatsumi Synthetic | Available chlorine | 129000 | 88070 | 79776 | 72527 | |
| | $ClO_3^-$ | 11890 | 26215 | 20449 | 24593 | |
| Nankai Chemical | Available chlorine | 122000 | 75928 | 63896 | 51671 | |
| | $ClO_3^-$ | 5247 | 20882 | 20882 | 25011 | |
| <General grade> unit: ppm | | | | | | |
| | | D+0 | D+10 | D+20 | D+30 | D+40 |
| Osaka Soda | Available chlorine | 122726 | 76033 | 62623 | 49732 | 42345 |
| | $ClO_3^-$ | 14476 | 33111 | 35512 | 43473 | 48870 |
| Nankai Chemical | Available chlorine | 123510 | 75755 | 67344 | 57973 | 51439 |
| | $ClO_3^-$ | 14577 | 29751 | 34235 | 37909 | 39801 |
| <Acid is added to general grade to set the pH to 9.7> unit: ppm | | | | | | |

(continued)

|  |  | D+0 | D+10 |  |
|---|---|---|---|---|
| Osaka Soda | Available chlorine | 122726 | 631 |  |
|  | $ClO_3^-$ | 14476 | 65904 |  |
| Nankai Chemical | Available chlorine | 123510 | 7812 |  |
|  | $ClO_3^-$ | 14577 | 58575 |  |

(Reaction 1 of deteriorated hypo)

Deteriorated sodium hypochlorite contains each of hypochlorite ions, chloride ions, and chlorate ions. First, a preliminary test was conducted for a case of reacting chlorate ions, for which a degradation reaction is expected to entail difficulty, with sulfuric acid. A sodium chlorate segment was provided for comparison of reactivity.

As a result, chlorine dioxide gas was readily generated in the sodium chlorate segment from reacting with sulfuric acid, and chlorite ions were able to be obtained through sodium hydroxide. The yield for chlorite ions increased by adding hydrogen peroxide to sodium hydroxide.

Meanwhile, chlorite ions could not be recovered from deteriorated sodium hypochlorite. Only chloride ions were able to be recovered. Furthermore, addition of hydrogen peroxide to sodium hydroxide resulted in an increase in the amount of chloride ions recovered.

This is understood as follows: when deteriorated sodium hypochlorite was reacted with sulfuric acid, chlorine gas was generated first from hypochlorite ions with high reactivity, and reacted with hydrogen peroxide in the recovery liquid, resulting in an increase in chloride ions.

If hydrogen peroxide in the recovery liquid decreases due to chlorine gas, the recovery rate of chlorite ions in the recovery liquid would decrease and chlorate ions would be generated.

In view of the above, the present manufacturing method uses deteriorated sodium hypochlorite as the reaction mother liquor, but it was found that a two consecutive reaction method for recovering chlorine gas as a first reaction and then recovering chlorine dioxide gas from a single reaction mother liquor is required, and it is necessary to separately recover these gases and subsequently combine the gases.

Accordingly, it was found that it is necessary, as a reaction model, to consider two stages of reaction conditions, i.e., a first reaction for generating chlorine gas from a reaction mother liquor prepared by adding deteriorated sodium hypochlorite and sulfuric acid solution and recovering hypochlorite ions with sodium hydroxide or calcium hydroxide, and then generating chlorine dioxide gas and recovering chlorite ions with sodium hydroxide and hydrogen peroxide.

$$*Cl_2 + 2NaOH \rightarrow NaCl + NaClO + H_2O \ ..... \qquad (1)$$

$$*Cl_2 + 2NaOH + H_2O_2 \rightarrow NaCl + NaClO + H_2O$$

$$NaClO + H_2O_2 \rightarrow NaCl + H_2O + O_2 \ ..... \qquad (2)$$

$$*2ClO_2 + 2NaOH \rightarrow NaClO_2 + NaClO_3 + H_2O \ ..... \qquad (3)$$

$$*2ClO_2 + 2NaOH + H_2O_2 \rightarrow 2NaClO_2 + O_2 + 2H_2O \ ..... \qquad (4)$$

[Table 2]

| <Raw material (added solution) > | | |
|---|---|---|
| A | Deteriorated sodium hypochlorite (*) | 0.1 mL × 50 times = total of 5.0 mL dropped in |
| B | 50 w/w% of sodium chlorate | 0.1 mL × 20 times = total of 2.0 mL dropped in |
| *Measurement value: chlorate ion 15,980 ppm, chloride ion 106,640 ppm | | |

<Receiving solution (reaction tank) >

| 50 w/w% $H_2SO_4$ | 50 mL |
|---|---|
| 35% $H_2O_2$ | 2.33 mL |

The final concentration of hydrogen peroxide: 1.56 % *$H_2SO_4$ at 30 w/w% or less is unsuitable due to low reactivity

<Recovery liquid (absorption vessel)>

|  | (1) | (2) |
|---|---|---|
| 12% NaOH | 50 mL | 50 mL |
| 35% $H_2O_2$ | - | 1.47 mL |

The final concentration of hydrogen peroxide: 1.00 %

<Analysis of recovery liquid> (unit: ppm)

|  |  | $Cl^-$ | $ClO_2^-$ | $ClO_3^-$ |
|---|---|---|---|---|
| Deteriorated hypochlorite segment | A (1) | 556.6 | 0.0 | 0.0 |
|  | A (2) | 990.6 | 0.0 | 0.0 |
| Sodium chlorate segment | B (1) | 63.4 | 157.6 | 260.6 |
|  | B (2) | 191.4 | 303.9 | 5.8 |

(Reaction 2 of deteriorated hypo)

The amount of chlorate ions in deteriorated sodium hypochlorite is low, and reactivity is poor if sulfuric acid is dropped in only by a small amount. Thus, the inventor conceived of generating chlorine gas and chlorine dioxide gas in different steps by performing a reaction in batches, increasing the concentration of sulfuric acid to be added to 65 w/w%, and increasing the temperature during the reaction. The inventor also conceived of preparing two types of solutions in recovery liquid A and recovery liquid B, recovering hypochlorite ions with recovery liquid A in the first reaction, switching the tubing arrangement after the completion of the first reaction, and recovering chlorite ions with recovery liquid B. As a result, chlorine dioxide started to be generated, and chlorite ions started to be recovered. Thus, it was found that the effect of the temperature and acidity in the reaction mother liquor is important. However, the yield was low because the majority of chlorite ions were recovered in recovery liquid A, to which hydrogen peroxide was not added, and chlorate ions were also generated due to the absence of hydrogen peroxide. For this reason, it was found that it is necessary to establish a condition for generating chlorine gas and chlorine dioxide gas in a more stepwise manner and to blow in the gases into each recovery liquid.

[Table 3-1]
(1) Raw material

| Raw material | Available chlorine concentration | $ClO_3^-$ |
|---|---|---|
| Deteriorated sodium hypochlorite (*) | 36360.4 | 27120.5 |

*12% low salt sodium hypochlorite

(2) Reaction condition (first reaction, second reaction)

| | Reaction mother liquor | |
|---|---|---|
| | First reaction | Second reaction |
| Deteriorated hypo | 200 g | |
| 65 % sulfuric acid | 200 g | |
| Total reaction mother liquor | 400 g | |
| Sulfuric acid concentration | 32.5 % | |
| Temperature | 30°C | 70°C |
| Total reaction time | 5h 30m | 3h |
| (Air time thereamong) | 4h | 1h 30m |

(3) Recovery liquid

| | Recovery liquid A | Recovery liquid B |
|---|---|---|
| 2N sodium hydroxide | 200 g | - |
| 1N sodium hydroxide | - | 200 g |
| Hydrogen peroxide | - | 6 g |

[Measurement result] (unit: ppm)

| | Available chlorine concentration | $Cl^-$ | $ClO_2^-$ | $ClO_3^-$ | $SO_4^{2-}$ |
|---|---|---|---|---|---|
| Recovery liquid A | 58781.06 | 30319.8 | 4679.9 | 9979.2 | 198.6 |
| Recovery liquid B | 1055.47 | 49.0 | 259.0 | 0 | 0 |
| Reaction | - | 14491.9 | 0 | 0 | ∞ |

| mother liquor | | | | | |
|---|---|---|---|---|---|

[Table 3-2]

| [Recovery rate of chlorite ions and available chlorine] | | |
|---|---|---|
| | $ClO_2^-$ recovery Theoretical value (21294.74 ppm) | Available chlorine concentration recovery Theoretical value (81097.26 ppm) |
| Recovery liquid A | 21.98 % | 72.48 % |
| Recovery liquid B | 1.22 % | 1.3 % |
| Total | 23.2 % | 73.78 % |

(Reaction 3 of deteriorated hypo)

To find the sulfuric acid concentration required for preferentially generating chlorine gas during the first reaction, the sulfuric acid concentration was set to 50 w/w% to perform a reaction. The sulfuric acid concentration in the reaction mother liquor at this time was 25.0 %.

As a result, recovery of chlorine gas in the first reaction and the recovery of chlorine dioxide gas in the second reaction were able to be controlled in a more stepwise manner. Further, an improvement in the recovery rate of chlorite ions in the recovery liquid B, to which sodium hydroxide and hydrogen peroxide were added, was observed. However, chlorite ions were also recovered in recovery liquid A, perhaps due to chlorine dioxide gas contamination, and chlorate ions were also generated because hydrogen peroxide cannot be added. For this reason, it was found that it is necessary to consider not only the concentration of sulfuric acid, but also the method of adding sulfuric acid.

[Table 4-1]

(1) Raw material

| Raw material | Available chlorine concentration | $ClO_3^-$ |
|---|---|---|
| Deteriorated sodium hypochlorite (*) | 36360.4 | 27120.5 |

*12% low salt sodium hypochlorite

(2) Reaction condition (first reaction, second reaction)

|  | Reaction mother liquor | |
|---|---|---|
|  | First reaction | Second reaction |
| Deteriorated hypo | 200 g | |
| 50% sulfuric acid | 200 g | |
| Total reaction mother liquor | 400 g | |
| Sulfuric acid concentration | 25.00% | |
| Temperature | 30°C | 70°C |
| Reaction time | 3h | 6h |
| (Air time thereamong) | 1h 30m | 4h |

(3) Recovery liquid

|  | Recovery liquid A | Recovery liquid B |
|---|---|---|
| 2N sodium hydroxide | 200 g | - |
| 1N sodium hydroxide | - | 200 g |
| Hydrogen peroxide | - | 6 g |

[Measurement results] (unit: ppm)

|  | Available chlorine concentration | $Cl^-$ | $ClO_2^-$ | $ClO_3^-$ | $SO_4^{2-}$ |
|---|---|---|---|---|---|
| Recovery liquid A | 35533.57 | 19483.8 | 696.9 | 1479.4 | 175.4 |
| Recovery liquid B | 18249.14 | 3083.2 | 8659.4 | 169.4 | 0 |

| Reaction mother liquor | - | 17579.2 | 0 | 0 | ∞ |
|---|---|---|---|---|---|

[Table 4-2]

| [Recovery rate of chlorite ion and available chlorine] | | | | |
|---|---|---|---|---|
| | | Theoretical value | Measurement value | Recovery rate |
| Recovery liquid A | Available chlorine | 36360.4 | 35533.57 | 97.73% |
| Recovery liquid B | Available chlorine | 44736.86 | 18249.14 | 40.79% |
| | $ClO_2$ | 21294.74 | 8659.4 | 40.66% |

(Reaction 4 of deteriorated hypo)

The reaction temperature and acidity due to sulfuric acid are important for reacting deteriorated sodium hypochlorite and recovering gas from a reaction mother liquor in two steps, but if the initial concentration of sulfuric acid is too high, both chlorine gas and chlorine dioxide gas are generated such that the recovery liquid cannot be switched. Therefore, when not only lowering the sulfuric acid concentration but also adding and reacting in two batches, no significant change in the yield was observed. It was found, however, that the amount of chlorine dioxide gas generated during the first reaction was larger when 100 g of 65 w/w% sulfuric acid was added twice, in comparison to when 200 g of 50 w/w% sulfuric acid was added. It was found therefrom that the sulfuric acid concentration in a reaction mother liquor during the first reaction is still excessive, and the reactivity also varies depending on the concentration of sulfuric acid upon addition.

[Table 5-1]

(1) Raw material

| Raw material | Available chlorine concentration | $ClO_3^-$ |
|---|---|---|
| | | |

| Deteriorated sodium hypochlorite(*) | 36360.4 | 27120.5 |
|---|---|---|

*12% low salt sodium hypochlorite

(2) Reaction condition (first reaction, second reaction)

| | Reaction mother liquor | |
|---|---|---|
| | First reaction | Second reaction |
| Deteriorated hypo | 200 g | |
| 65% sulfuric acid | 100 g | 100 g |
| Total added sulfuric acid concentration | 21.67 % | 32.50 % |
| Temperature | 30°C | 70°C |
| Reaction time | 3h | 4h 30 |
| (Air time thereamong) | 1h 30m | 2h 30m |

(3) Recovery liquid

| | Recovery liquid A | Recovery liquid B |
|---|---|---|
| 2N sodium hydroxide | 200g | – |
| 1N sodium hydroxide | – | 200g |
| Hydrogen peroxide | – | 6g |

[Measurement results] (unit: ppm)

| | Available chlorine concentration | $Cl^-$ | $ClO_2^-$ | $ClO_3^-$ | $SO_4^{2-}$ |
|---|---|---|---|---|---|
| Recovery liquid A | 38843.30 | 27151.5 | 1847.7 | 3645.8 | 115.4 |
| Recovery liquid B | 21625.57 | 1117.4 | 8413.8 | 73.7 | 0 |

[Table 5-2]

[Recovery rate of chlorite ion and available chlorine]

| | | Theoretical value | Measurement value | Recovery rate |
|---|---|---|---|---|
| Recovery | Available | 36360.4 | 38843.30 | 106.83 % |

[Table 5-2]

| [Recovery rate of chlorite ion and available chlorine] | | | | |
|---|---|---|---|---|
| | | Theoretical value | Measurement value | Recovery rate |
| Recovery liquid A | Available chlorine | 36360.4 | 38843.30 | 106.83 % |
| Recovery liquid B | Available chlorine | 44736.86 | 21625.57 | 48.34 % |
| | $ClO_2^-$ | 21294.74 | 8413.8 | 39.51 % |

(Reaction 5 of deteriorated hypo)

The temperature and the concentration and amount of sulfuric acid in the first reaction and the second reaction were each determined respectively, and sulfuric acid was added twice to study recovery of chlorine gas and chlorine dioxide gas separately. The sulfuric acid concentration used in the first reaction was 50 w/w%, and 25 g was the amount added, so that the sulfuric acid concentration in the reaction mother liquor was 5.6 %. The sulfuric acid concentration in the second reaction was 65 w/w%, and 250 g was the amount added, so that the sulfuric acid concentration in the reaction mother liquor was 36.8%. It was possible as a result thereof to reduce chlorine dioxide gas contamination in recovery liquid A and to reduce each of chlorite ions and chlorate ions.

The amount of chlorite ions recovered in recovery liquid B increased 20% from the previous amount, which was 60% or more of the expected value. Specifically, it was found that it is important to control chlorine dioxide gas contamination in recovery liquid A by adjusting the sulfuric acid concentration in the first reaction, and to prevent degradation of chlorate ions for the acidity of the reaction mother liquor during the first reaction.

[Table 6-1]

(1) Raw material

| Raw material | Available chlorine concentration | $ClO_3^-$ |
|---|---|---|
| Deteriorated sodium hypochlorite(*) | 36360.4 | 27120.5 |

*12% low salt sodium hypochlorite

(2) Reaction condition (first reaction, second reaction)

|  | Reaction mother liquor | |
|---|---|---|
|  | First reaction | Second reaction |
| Deteriorated hypochlorite | 200 g |  |
| 50% sulfuric acid | 25 g | – |
| 65% sulfuric acid | – | 250 g |
| Total added sulfuric acid concentration | 5.56 % | 36.84 % |
| Temperature | 30°C | 70°C |
| Reaction time | 2h 30m | 4h 30 |
| (Air time thereamong) | 1h 30m | 3h |

(3) Recovery liquid

|  | Recovery liquid A | Recovery liquid B |
|---|---|---|
| 2N sodium hydroxide | 200 g | – |
| 1N sodium hydroxide | – | 200 g |
| Hydrogen peroxide | – | 6 g |

[Measurement results] (unit: ppm)

|  | Available chlorine concentration | $Cl^-$ | $ClO_2^-$ | $ClO_3^-$ | $SO_4^{2-}$ |
|---|---|---|---|---|---|
| Recovery liquid A | 29923.70 | 23857.0 | 73.9 | 154.6 | 86.0 |
| Recovery liquid B | 29948.55 | 5949.0 | 13371.0 | 402.8 | 0 |

[Table 6-2]

| [Recovery rate of chlorite ion and available chlorine] | | | | |
|---|---|---|---|---|
|  |  | Theoretical value | Measurement value | Recovery rate |
| Recovery liquid A | Available chlorine | 36360.4 | 29923.70 | 82.30% |
| Recovery liquid B | Available chlorine | 44736.86 | 29948.55 | 66.94% |
|  | $ClO_2^-$ | 21294.74 | 13371.0 | 62.79% |

(Reaction 6 of deteriorated hypo)

Since the sulfuric acid concentration and amount during the first reaction and second reaction were known, changes in recovery rate were confirmed when the treatment amount was increased 5 times. Further, in order to confirm the presence or absence of gas recovery leakage, the recovery liquids A and B were respectively provided with preliminary recovery tanks recovery liquid. Furthermore, the timing of blowing air was advanced so that air was blown into the reaction tank immediately after the raw materials were charged, and the amount of air was gradually increased.

As a result, after increasing the processing amount by 5-fold, only hypochlorite ions were recovered with recovery liquid A, while the amount of chlorite ions and chlorate ions generated remained low. The amount of chlorite ions in recovery liquid B also increased to 87.42%. The reason for the increase in the recovery rate due to the timing of sending air during the second reaction is understood to be chlorine gas or chlorine dioxide gas generated in a reaction tank, when left to natural recovery, returning to a solution or generating a reverse reaction.

However, during the reaction, gas is generated in the reaction tank, and air is blown into the reaction tank to increase the internal pressure, and a backflow occurs when the blowing of gas or air is weakened. The results of this test show that the recovery rate is good, but a backflow occurs, and sulfate ions are detected in the recovered liquid, so it is necessary to perform a retest. It is necessary to set up a trap tank for stopping a backflow that releases pressure between the reaction tank and the recovery tank. This trap tank serves to prevent backflow, prevent entrainment of droplets from the reaction solution, and further serve as a gas recovery tank recovery liquid.

[Table 7-1]

(1) Raw material

| Raw material | Available chlorine concentration | ClO$_3$$^-$ |
|---|---|---|
| Deteriorated sodium hypochlorite(*) | 36360.4 | 27120.5 |

*12% low salt sodium hypochlorite

(2) Reaction condition (first reaction, second reaction)

| | Reaction mother liquor | |
|---|---|---|
| | First reaction | Second reaction |
| Deteriorated hypo | 1000 g | |
| 50% sulfuric acid | 125 g | – |
| 65% sulfuric acid | – | 1250 g |
| Total added sulfuric acid concentration | 5.56 % | 36.84 % |
| Temperature | 30°C | 70°C |
| Reaction time | 4h 15m | 4h 10m |
| (Air time thereamong) | 2h 45m | 3h 50m |

(3) Recovery liquid

| | Recovery liquid A1 | Recovery liquid A2 | Recovery liquid B1 | Recovery liquid B2 |
|---|---|---|---|---|
| 2N sodium hydroxide | 1000 g | 200 g | – | – |
| 1N sodium hydroxide | – | – | 1000 g | 200 g |
| Hydrogen peroxide | – | – | 30 g | 6 g |

[Measurement results] (unit: ppm)

| | Available | Cl$^-$ | ClO$_2$$^-$ | ClO$_3$$^-$ | SO$_4$$^{2-}$ |
|---|---|---|---|---|---|

30

| | chlorine concentration | | | | |
|---|---|---|---|---|---|
| Recovery liquid A1 | 30573.82 | 27119.3 | 170.4 | 294.9 | 116.5 |
| Recovery liquid A2 | 135.90 | 16.7 | 41.2 | 19.3 | 0 |
| Recovery liquid B1 | 35221.86 | 2605.8 | 18616.1 | 205.0 | 25688.0 |
| Recovery liquid B2 | 106.22 | 16.2 | 33.0 | 0 | 0 |

*Reverse current sulfuric acid contamination occurred in recovery liquid B1.

[Table 7-2]

| [Recovery rate of chlorite ion and available chlorine] | | | | |
|---|---|---|---|---|
| | | Theoretical value | Measurement value | Recovery rate |
| Recovery liquid A1 | Available chlorine | 36360.4 | 30573.82 | 84.09 % |
| Recovery liquid A2 | | | 135.90 | 0.07 % |
| Recovery liquid B1 | Available chlorine | 44736.86 | 35221.86 | 78.73 % |
| Recovery liquid B2 | | | 106.22 | 0.05 % |
| Recovery liquid B1 | $ClO_2^-$ | 21294.74 | 18616.1 | 87.42 % |
| Recovery liquid B2 | | | 33.0 | 0.03 % |

(Reaction 7 of deteriorated hypo)

When it was reexamined whether the recovery rate improves depending on the timing of sending in air by providing a trapping vessel but without any major change, it was found that the recovery rate of available chlorine was better when air is sent in from the early stage of the reaction. Further, reduced chlorite ions and chlorate ions in recovery liquid A and reduced chloride ions in recovery liquid B enabled separate recovery of recovery liquids A and B.

In the first reaction, when deteriorated sodium hypochlorite and 50 w/w% sulfuric acid were added in the stage, chlorine gas was generated rapidly, so it was not necessary to blow air into the reaction tank, and the reaction weakened. After 1 hour and 25 minutes, air was blown in for 2 hours and 50 minutes, and a total of 4 hours and 15 minutes of reaction were performed. Next, in the second reaction, chlorine dioxide gas was generated rapidly when 1000 g of 65 w/w% sulfuric acid was added to the reaction mother liquor, but the reaction weakened 20 minute later, so gradually increase the air for 3 hours and 50 minutes. A total for 4 hours and 15 minutes of reaction are performed. From the above, especially in the second reaction, air is fed to the reaction tank. It was found that the recovery rate is significantly improved by positively feeding and forcing chlorine dioxide gas into the recovery liquid.

[Table 8-1]

(1) Raw material

| Raw material | Available chlorine concentration | $ClO_3^-$ |
|---|---|---|
| Deteriorated sodium hypochlorite(*) | 36360.4 | 27120.5 |

*12% low salt sodium hypochlorite

(2) Reaction condition (first reaction, second reaction)

| | Reaction mother liquor | |
|---|---|---|
| | First reaction | Second reaction |
| Deteriorated hypo | 800 g | |
| 50% sulfuric acid | 100 g | – |
| 65% sulfuric | – | 1000 g |

| acid | | |
|---|---|---|
| Total added sulfuric acid concentration | 5.56 % | 36.84 % |
| Temperature | 30°C | 70°C |
| Reaction time | 4h 15m | 4h 5m |
| (Air time) | 2h 50m | 3h 50m |

(3) Recovery liquid

| | Recovery liquid A1 | Recovery liquid A2 | Recovery liquid B1 | Recovery liquid B2 |
|---|---|---|---|---|
| 2N sodium hydroxide | 800 g | 200 g | – | – |
| 1N sodium hydroxide | – | – | 800 g | 200 g |
| Hydrogen peroxide | – | – | 24 g | 6 g |

[Measurement results] (unit: ppm)

| | Available chlorine concentration | $Cl^-$ | $ClO_2^-$ | $ClO_3^-$ | $SO_4^{2-}$ |
|---|---|---|---|---|---|
| Recovery liquid A1 | 30643.64 | 25265.9 | 77.9 | 225.8 | 31.9 |
| Recovery liquid A2 | 93.33 | 17.9 | 23.7 | 12,9 | 0 |
| Recovery liquid B1 | 35902.82 | 729.3 | 16857.0 | 38.5 | 86.8 |
| Recovery liquid B2 | 112.52 | 41.1 | 33.8 | 0 | 0 |

[Table 8-2]

| [Recovery rate of chlorite ion and available chlorine] | | | | |
|---|---|---|---|---|
| | | Theoretical value | Measurement value | Recovery rate |
| Recovery liquid A1 | Available chlorine | 36360.4 | 30643.64 | 84.28% |
| Recovery liquid A2 | | | 93.33 | 0.06% |
| Recovery liquid B1 | Available chlorine | 44736.86 | 35902.82 | 80.25% |
| Recovery liquid B2 | | | 112.52 | 0.06% |
| Recovery liquid B1 | $ClO_2^-$ | 21294.74 | 16857.0 | 79.16% |

(continued)

| [Recovery rate of chlorite ion and available chlorine] | | | | |
|---|---|---|---|---|
| | | Theoretical value | Measurement value | Recovery rate |
| Recovery liquid B2 | | | 33.8 | 0.03% |

(Reaction 8 of deteriorated hypo)

To re-examine the difference in recovery rates depending on the timing of starting air blowing, the recovery rate was studied when the air blowing start time was intentionally delayed.

In this regard, a natural reaction was performed for 2 hours and 30 minutes during the second reaction, and air was blown in at a stage where bubbling or gas generation became low. This method is a method conducted at the early stage of research.

As a result, the recovery rate was dramatically reduced, so that it was reconfirmed that the recovery rate can be elevated if air is blown more aggressively from the early stage of reaction. It was found that rapid bubbling due to generation of chlorine dioxide gas during the second reaction affects the productivity, but is about 15 minutes or less, so that it is necessary to send in air into a reaction tank by this point and gradually increase the amount of air in order to improve the recovery rate.

[Table 9-1]

(1) Raw material

| Raw material | Available chlorine concentration | $ClO_3^-$ | $Cl^-$ |
|---|---|---|---|
| Deteriorated sodium hypochlorite(*) | 36360.4 | 27120.5 | 54989.0 |

*12% low salt sodium hypochlorite

(2) Reaction condition (first reaction, second reaction)

|  | Reaction mother liquor | |
|---|---|---|
|  | First reaction | Second reaction |
| Deteriorated hypo | 800 g |  |
| 50% sulfuric acid | 100 g | - |
| 65% sulfuric acid | - | 1000 g |
| Total added sulfuric acid concentration | 5.60 % | 36.84 % |
| Temperature | 30°C | 70°C |
| Reaction time | 4h 25m | 5h |
| (Air time) | 2h 30m | 2h 30m |

Recovery liquid

|  | Recovery liquid A1 | Recovery liquid A2 | Recovery liquid B1 | Recovery liquid B2 |
|---|---|---|---|---|
| 2N sodium hydroxide | 800 g | 200 g | - | - |
| 1N sodium hydroxide | - | - | 800 g | 200 g |
| Hydrogen peroxide | - | - | 24 g | 6 g |

[Measurement results] (unit: ppm)

|  | Available chlorine concentration | $Cl^-$ | $ClO_2^-$ | $ClO_3^-$ | $SO_4^{2-}$ |
|---|---|---|---|---|---|
| Recovery liquid A1 | 30279.15 | 22465.2 | 48.4 | 143.1 | 36.8 |
| Recovery liquid A2 | 46.46 | 6.2 | 12.7 | 5.3 | 0 |
| Recovery liquid B1 | 30533.27 | 3519.8 | 14290.6 | 175.1 | 88.6 |
| Recovery liquid B2 | 87.35 | 26.9 | 20.0 | 0 | 0 |

[Table 9-2]

| [Recovery rate of chlorite ion and available chlorine] | | | | |
|---|---|---|---|---|
| | | Theoretical value | Measurement value | Recovery rate |
| Recovery liquid A1 | Available chlorine | 36360.4 | 30279.15 | 83.28 % |
| Recovery liquid A2 | | | 46.46 | 0.03 % |
| Recovery liquid B1 | Available chlorine | 44736.86 | 30533.27 | 68.25 % |
| Recovery liquid B2 | | | 87.35 | 0.05 % |
| Recovery liquid B1 | $ClO_2^-$ | 21294.74 | 14290.6 | 67.11 % |
| Recovery liquid B2 | | | 20.0 | 0.02 % |

(Reaction 9 of deteriorated hypochlorite)

The chemical concentration is important when studying a useful disinfectant. While the amount of recovery liquid was set to about equal to the amount of raw material deteriorated sodium hypochlorite up to this point, a concentration recovery test was conducted to find whether the recovery rate decreases when the amount of the recovery liquid was halved.

As a result, it was possible to obtain a recovery liquid with a concentration of available chlorine derived from hypochlorous acid of 8.38%, which was about 3%, and a concentration of available chlorine derived from chlorous acid of 4.96%, which was about 3.5%. However, concentrated recovery with recovery liquid A was relatively easy, whereas the recovery rate for recovery liquid B was poor, only recovering 63.04% of the expected value of recovery.

When the reaction mother liquor after obtaining recovery liquid A and the reaction mother liquor after obtaining recovery liquid B were measured, it was found that chlorate ions increased from 24378.5 ppm to 33990.4 ppm in the reaction mother liquor after the first reaction. In contrast, it was found that chloride ions decreased from 50079.5 ppm to 23532.4 ppm. The increase in chlorate ions is understood to be due to a re-disproportionation reaction when becoming strongly acidic from addition of sulfuric acid. The decrease in chloride ions upon addition of sulfuric acid is understood from generation of hydrochloric acid that contributed to the reaction.

These can be explained by the following chemical formulas. Chloride ions generated in deteriorated sodium hypochlorite generate hydrochloric acid in the presence of sulfuric acid, and subsequently in the second reaction, chlorine dioxide gas is generated from hydrochloric acid and chloric acid.

$$2NaClO + H_2SO_4 \rightarrow Na_2SO_4 + Cl_2 + H_2O \ ..... \qquad \text{(5) first reaction}$$

$$2NaCl + H_2SO_4 \rightarrow 2HCl + Na_2SO_4 \ ..... \qquad \text{(6) first reaction, second reaction}$$

$$NaClO + 2HCl \rightarrow NaCl + Cl_2 + H_2O \ ..... \qquad \text{(7) first reaction}$$

$$3NaClO \rightarrow 2NaClO + NaClO_3 \ ..... \qquad \text{(8) first reaction}$$

$$2NaClO_3 + H_2SO_4 \rightarrow 2HClO_3 + Na_2SO_4 \ ..... \qquad \text{(9) second reaction}$$

$$HClO_3 + HCl \rightarrow HClO_2 + HClO \ ..... \qquad \text{(10) second reaction}$$

$$HClO_3 + HClO_2 \rightarrow 2ClO_2 + H_2O \ ..... \qquad \text{(11) second reaction}$$

[Table 10-1]

(1) Raw material

| Raw material | Available chlorine concentration | $ClO_3^-$ | $Cl^-$ |
|---|---|---|---|
| Deteriorated sodium hypochlorite(*) | 49293.84 | 24378.5 | 50079.5 |

*12% low salt sodium hypochlorite

(2) Reaction condition (first reaction, second reaction)

| | Reaction mother liquor | |
|---|---|---|
| | First reaction | Second reaction |
| Deteriorated | 2000 g | |

| hypo | | |
|---|---|---|
| 50% sulfuric acid | 500 g | – |
| 65% sulfuric acid | – | 2000 g |
| Total added sulfuric acid concentration | 10.00 % | 34.44 % |
| Temperature | 30°C | 70°C |
| Reaction time | 4h 5m | 2h 55m |
| (Air time) | 2h | 2h 40m |

(3) Recovery liquid

| | Recovery liquid A | Recovery liquid B |
|---|---|---|
| 4N sodium hydroxide | 1000 g | – |
| 2N sodium hydroxide | – | 1000 g |
| Hydrogen peroxide | – | 55 g |

[Measurement results] (unit: ppm)

| | Available chlorine concentration | $Cl^-$ | $ClO_2^-$ | $ClO_3^-$ | $SO_4^{2-}$ |
|---|---|---|---|---|---|
| After first reaction | – | 23532.4 | 0 | 33990.4 | 159909.8 |
| Recovery liquid A | 83778.79 | 102926.7 | 405.4 | 2901.4 | 90.6 |
| After second reaction | – | 20751.4 | 0 | 0 | ∞ |
| Recovery liquid B | 49571.71 | 583.7 | 26587.5 | 62.2 | 55.1 |

[Table 10-2]

| [Recovery rate of chlorite ion and available chlorine] | | | | |
|---|---|---|---|---|
| | | Theoretical value | Measurement value | Recovery rate |
| Recovery liquid A | Available chlorine | 98587.68 | 83778.79 | 84.98 % |
| Recovery liquid B | Available chlorine | 78636.26 | 49571.71 | 63.04 % |
| | $ClO_2^-$ | 37430.86 | 26587.5 | 71.03 % |

(Reaction 10 of deteriorated hypo)

To find the upper limit of the sulfuric acid concentration in the reaction mother liquor in the first and second reactions, a test was conducted which adjusted the acidity and set the sulfuric acid concentration in the reaction mother liquor to 6.37 % and 37.03 s%, respectively. Concentrated recovery was also conducted.

As a result, the recovery rate for recovery liquid A was 69.87%, but some chlorite ions and chlorate ions were also being generated. Thus, it was found that further increase in the acidity in the first reaction is not suitable.

As for the recovery rate for recovery liquid B, the recovery rate of available chlorine increased from 63.04% to 74.48% and the recovery rate of chlorite ions increased from 71.03% to 78.91%. Thus, it was considered that acidity was lacking at the upper limit. The current test also confirmed that chlorate ions increased and chloride ions decreased in the reaction mother liquor after the completion of the first reaction.

[Table 11-1]

(1) Raw material

| Raw material | Available chlorine concentration | $ClO_3^-$ | $Cl^-$ |
|---|---|---|---|
| Deteriorated sodium hypochlorite(*) | 54843.73 | 21571.0 | 45351.5 |

*12% low salt sodium hypochlorite

(2) Reaction condition (first reaction, second reaction)

| | Reaction mother liquor | |
|---|---|---|
| | First reaction | Second reaction |
| Deteriorated | 1600 g | |

| hypo | | |
|---|---|---|
| 50% sulfuric acid | 200 g | – |
| 65% sulfuric acid | 25 g | 2000 g |
| Total added sulfuric acid concentration | 6.37 % | 37.03 % |
| Temperature | 30°C | 70°C |
| Reaction time | 6h 50m | 3h 15m |
| (Air time) | 4h 5m | 3h |

(3) Recovery liquid

| | Recovery liquid A1 | Recovery liquid B1 |
|---|---|---|
| 4N sodium hydroxide | 800 g | – |
| 2N sodium hydroxide | – | 800 g |
| Hydrogen peroxide | – | 38.4 g |

[Table 11-2]

[Measurement results] (unit: ppm)

| | Weight before reaction | Weight after reaction | Rate of increase |
|---|---|---|---|
| Recovery liquid A | 800.00 g | 879.90 g | 109.99 % |
| Recovery liquid B | 838.40 g | 892.72 g | 106.48 % |

| | Available chlorine concentration | $Cl^-$ | $ClO_2^-$ | $ClO_3^-$ | $SO_4^{2-}$ |
|---|---|---|---|---|---|
| After first reaction | – | 30689.4 | 0 | 38833.0 | 90011.4 |
| Recovery liquid A | 76635.55 | 86579.0 | 246.3 | 1124.9 | 136.4 |
| After second reaction | – | 23072.2 | 0 | 0 | ∞ |
| Recovery liquid B | 52123.42 | 771.9 | 26555.2 | 569.8 | 28.0 |

[Recovery rate of chlorite ion and available chlorine]

|  |  | Theoretical value | Measurement value | Recovery rate |
|---|---|---|---|---|
| Recovery liquid A | Available chlorine | 109687.47 | 76635.55 | 69.87% |
| Recovery liquid B | Available chlorine | 69984.78 | 52123.42 | 74.48% |
|  | $ClO_2^-$ | 33312.76 | 26555.2 | 79.71% |

(Reaction 11 of deteriorated hypo)

The reaction method up to this point conducted a second reaction using sulfuric acid solution with almost the same weight as deteriorated sodium hypochlorite. However, chlorate ions generated in deteriorated sodium hypochlorite are increased via the first reaction, but are insufficient as a concentration that increases the reactivity.

In this regard, the difference in yield when the second reaction was performed with a sulfuric acid concentration in the reaction mother liquor during the second reaction of 50% or greater was investigated. The second reaction was performed to study the recovery rate after using 70 w/w% sulfuric acid as the sulfuric acid and setting the sulfuric acid concentration in the reaction mother liquor to 58.47% or greater.

Since it was revealed that chloric acid was regenerated after the completion of the first reaction, hydrogen peroxide was further added to the reaction mother liquor as a reducing agent after adding sulfuric acid to confirm whether the recovery rate also improves.

As a result, it was confirmed that when 70 w/w% sulfuric acid was used to set the sulfuric acid concentration in the reaction mother liquor to 58.47% or greater, the chlorate ions in the reaction mother liquor were completely degraded, and the recovery rate of available chlorine was dramatically elevated. Since the recovery rate of available chlorine was 83.46% and the recovery rate of chlorite ions was 99.63%, it was found that a preferred result is attained by using a concentration of sulfuric acid added during the second reaction of 65 w/w% or greater or 70 w/w% if possible, and the sulfuric acid concentration in the reaction mother liquor of up to 59.39%.

However, a large amount of chloride ions are generated in recovery liquid B, which is larger than the amount of chlorite ions. This result is not a problem in the case of liquid product such as those of sodium hypochlorite, but when processed into powder products such as high grade chlorinated lime or the like, the concentration ratio decreases as the amount of inorganic salts increases, and high concentration of available chlorine cannot be obtained.

Meanwhile, it was confirmed that when sulfuric acid was added to raw material deteriorated sodium hypochlorite and then hydrogen peroxide was added, the recovery rate decreased significantly. It is understood that this is in accordance with the following chemical reactions. It can be understood that hydrochloric acid generated by a reaction between sulfuric acid and chloride ion was degraded and failed to contribute to the reaction.

$$H_2O_2 + 2HCl \rightarrow 2H_2O + Cl_2 \ ..... \qquad (12)$$

It was found that, if the generated hydrochloric acid is decomposed before the reaction, not only is the recovery rate lowered, but also the generated chlorine gas is mixed in the recovery liquid B and decomposed by the hydrogen peroxide in the recovery liquid B, , leading to generate a large amount of chloride ions.

[Table 12-1]

(1) Raw material

<Raw material (Added solution)>

|  | Available chlorine concentration | $ClO_3^-$ | $Cl^-$ |
|---|---|---|---|
| Deteriorated sodium hypochlorite(*) | 46299.26 | 31019.5 | 56959.5 |

*12% low salt sodium hypochlorite

(2) Reaction condition (first reaction, second reaction)

|  | First reaction | Dispense reaction mother liquor | Second reaction (1) | Second reaction (2) | Second reaction (3) |
|---|---|---|---|---|---|
| Deteriorated hypo | 400g | | 100g | 100g | 100g |
| 50% sulfuric acid | 50g | | — | — | — |
| 65% sulfuric acid | 6.25g | | — | — | — |
| 70％sulfuric acid | — | | 500g | 500g | 500g |
| 35% hydrogen peroxide | — | | — | 2.38g | 9.5g |
| Reaction mother liquor (total) | 456.25g | | 600g | 602.38g | 609.5g |
| Total added sulfuric acid concentration | 6.37% | | 59.39% | 59.16% | 58.47% |
| Reaction temperature | Ordinary temperature | | 65 to 70°C | 65 to 70°C | 65 to 70°C |
| Reaction time | 3h55m | | 5h30m | 5h30m | 5h30m |
| (Air time) | 3h | | 5h30m | 5h30m | 5h30m |

(3) Recovery liquid

|  | Recovery liquid A | Recovery liquid B1 | Recovery liquid B2 | Recovery liquid B3 |
|---|---|---|---|---|

| 2N sodium hydroxide | 400 g | – | – | – |
|---|---|---|---|---|
| 1N sodium hydroxide | – | 100 g | 100 g | 100 g |
| Hydrogen peroxide | – | 2.4 g + 2.0 g | 2.4 g + 2.0 g | 2.4 g + 2.0 g |

[Table 12-2]

[Measurement results] (unit: ppm)

| | Available chlorine concentration | $Cl^-$ | $ClO_2^-$ | $ClO_3^-$ |
|---|---|---|---|---|
| After first reaction | 26.31 | 25333.4 | 0 | 37519.8 |
| After second reaction (1) | 19.31 | 1378.8 | 0 | 0 |
| After second reaction (2) | 30.92 | 1499.0 | 0 | 0 |
| After second reaction (3) | 27.40 | 1256.6 | 0 | 0 |

| | Available chlorine concentration | $Cl^-$ | $ClO_2^-$ | $ClO_3^-$ |
|---|---|---|---|---|
| Recovery liquid A | 43228.25 | 31250.0 | 0 | 124.8 |
| Recovery liquid B1 | 40792.42 | 25884.9 | 23180.5 | 2704.2 |
| Recovery liquid B2 | 40861.64 | 22431.7 | 22605.8 | 819.1 |
| Recovery liquid B3 | 34860.44 | 26768.6 | 17853.3 | 1688.5 |

[Recovery rate of chlorite ion and available chlorine] (unit: ppm)

| | | Theoretical | Measurement | Recovery |
|---|---|---|---|---|

|  |  | value | value | rate |
|---|---|---|---|---|
| Recovery liquid A | Available chlorine | 46299.26 | 43228.25 | 93.37 % |
| Recovery liquid B1 | Available chlorine | 48878.59 | 40792.42 | 84.46 % |
|  | $ClO_2^-$ | 23266.21 | 23180.5 | 99.63 % |
| Recovery liquid B2 | Available chlorine | 48878.59 | 40861.64 | 83.60 % |
|  | $ClO_2^-$ | 23266.21 | 22605.8 | 97.16 % |
| Recovery liquid B3 | Available chlorine | 48878.59 | 34860.44 | 71.32 % |
|  | $ClO_2^-$ | 23266.21 | 17853.3 | 76.76 % |

(Reaction 12 of deteriorated hypo)

To find the lower limit value of sulfuric acid concentration in the reaction mother liquor during the second reaction, the recovery rate was studied again with the sulfuric acid in the reaction mother liquor set to 35.7 %. The reaction mother liquors after the completion of the reaction were each measured to study the residual components after the completion of the first reaction and the second reaction.

As a result, a high recovery rate was attained, although limited to the specification for sodium hypochlorite, due to a large quantity of chloride ions in recovery liquid B.

[Table 13-1]

(1) Raw material

| Raw material | Available chlorine concentration | $ClO_3^-$ | $Cl^-$ |
|---|---|---|---|
| Deteriorated sodium hypochlorite(*) | 46299.26 | 31019.5 | 56959.5 |

*12% low salt sodium hypochlorite

(2) Reaction condition (first reaction, second reaction)

|  | First reaction | Dispense reaction | Second reaction |
|---|---|---|---|

| | | | |
|---|---|---|---|
| Deteriorated hypo | 200g | mother liquor | 100g |
| 50% sulfuric acid | 25.0g | | — |
| 65% sulfuric acid | 3.13g | | 100g |
| 35% hydrogen peroxide | — | | — |
| Reaction mother liquor (total) | 228.13g | | 200g |
| Total added sulfuric acid concentration | 6.37% | | 35.69% |
| Reaction temperature | Ordinary temperature | | 65 to 70°C |
| Reaction time | 3h25m | | 6h |
| (Air time) | 3h | | 6h |

(3) Recovery liquid

| | Recovery liquid A | Recovery liquid B |
|---|---|---|
| 2N sodium hydroxide | 200 g | – |
| 1N sodium hydroxide | – | 100 g |
| Hydrogen peroxide | – | 2.4 g + 2 g |

[Table 13-2]

[Measurement results] (unit: ppm)

| | Available chlorine concentration | $Cl^-$ | $ClO_2^-$ | $ClO_3^-$ |
|---|---|---|---|---|
| Reaction mother liquor after first reaction | 19.61 | 30615.0 | 0 | 44510.4 |
| Reaction mother liquor after second reaction | 24.56 | 942.8 | 0 | 0 |

| | Available chlorine concentration | Cl⁻ | ClO₂⁻ | ClO₃⁻ |
|---|---|---|---|---|
| Recovery liquid A | 41759.13 | 39759.2 | 0 | 202.9 |
| Recovery liquid B | 35751.32 | 28129.0 | 21741.3 | 2428.8 |

[Recovery rate of chlorite ion and available chlorine]
(unit: ppm)

| | | Theoretical value | Measurement value | Recovery rate |
|---|---|---|---|---|
| Recovery liquid A | Available chlorine | 46299.26 | 41759.13 | 90.19 % |
| Recovery liquid B | Available chlorine | 48878.59 | 35751.32 | 73.14 % |
| | ClO₂⁻ | 23266.21 | 21741.3 | 93.45 % |

(Reaction 14 of deteriorated hypo)

The production was carried out as follows:
Low salt grade sodium hypochlorite was used as a raw material, and the concentrations of sulfuric acid added to the reaction mother liquor during the first reaction and the second reaction were all adjusted to 70 w/w%, and then the sulfuric acid concentration in the reaction mother liquor during the first reaction was set to 6.37% and the sulfuric acid concentration during the second reaction to 35.7%.

It is understood as a result thereof that sulfuric acid during the first reaction is dependent on the sulfuric acid concentration in the reaction mother liquor during the first reaction, regardless of the concentration of sulfuric acid to be added.

It was found that sulfuric acid during the second reaction is dependent on the concentration of the sulfuric acid used and the concentration of sulfuric acid in the reaction mother liquor during the second reaction. It was also found that a higher concentration of sulfuric acid used during the second reaction is preferable, e.g., 70% w/w or at least 65% w/w. It was found that it is not just the sulfuric acid concentration in the reaction mother liquor that is important in the second reaction.

The recovery yield of recovery liquid B is excellent, exceeding 80%. Meanwhile, the concentration of chloride ions in recovery liquid B is higher than that of chlorite ions. Such composition can be used only for the sodium hypochlorite specification.

[Table 14-1]

(1) Raw material

| Raw material | Available chlorine concentration | ClO₃⁻ | Cl⁻ |
|---|---|---|---|
| Deteriorated sodium hypochlorite(*) | 46299.26 | 31019.5 | 56959.5 |

*12% low salt sodium hypochlorite

(2) Reaction condition (first reaction, second reaction)

|  | First reaction | Dispense reaction mother liquor | Second reaction |
|---|---|---|---|
| Deteriorated hypo | 200g |  | 100g |
| 70% sulfuric acid | 20.02g |  | 100g |
| Reaction mother liquor (total) | 220.02g |  | 200g |
| Total added sulfuric acid concentration | 6.37% |  | 38.19% |
| Reaction temperature | Ordinary temperature |  | 65 to 70°C |
| Reaction time | 3h25m |  | 5h30m |
| (Air time) | 3h |  | 5h30m |

(3) Recovery liquid

|  | Recovery liquid A | Recovery liquid B |
|---|---|---|
| 2N sodium hydroxide | 200 g |  |
| 1N sodium hydroxide |  | 100 g |
| Hydrogen peroxide | – | 4.4 g + 0.1 g |

[Table 14-2]

[Measurement results] (unit: ppm)

|  | Available chlorine concentration | $Cl^-$ | $ClO_2^-$ | $ClO_3^-$ |
|---|---|---|---|---|
| After first reaction | 19.26 | 30037.0 | 0 | 42540.6 |
| After second reaction | 12.61 | 1989.0 | 0 | 0 |

|  | Available chlorine concentration | $Cl^-$ | $ClO_2^-$ | $ClO_3^-$ |
|---|---|---|---|---|
| Recovery liquid A | 43112.57 | 30211.7 | 99.0 | 339.6 |
| Recovery | 41365.76 | 26040.7 | 23688.2 | 1201.5 |

| liquid B | | | | |
|----------|--|--|--|--|

[Recovery rate of chlorite ion and available chlorine]

| | | Theoretical value | Measurement value | Recovery rate |
|---|---|---|---|---|
| Recovery liquid A | Available chlorine | 46299.26 | 43112.57 | 93.12 % |
| Recovery liquid B | Available chlorine | 48833.28 | 41365.76 | 84.71 % |
| | $ClO_2^-$ | 23244.64 | 23688.2 | 101.91 % |

(Reaction 15 of deteriorated hypo)

The reactivity and recovery rate in the present manufacturing method were studied using deteriorated sodium hypochlorite, not only with reduced quality of low salt sodium hypochlorite, but also 12% general grade sodium hypochlorite solution.

General grade sodium hypochlorite characteristically tends to have a lower drug unit price, larger quantity of chloride ions in the composition, and more chlorate ions from the beginning compared to low salt sodium hypochlorite.

In this test, since there were an excess amount of chloride ions in the raw material, the sulfuric acid concentration of the reaction mother liquor during the first reaction was set low to 4.47%, and sodium chloride was further added in order to achieve complete degradation of chlorate ions in the reaction mother liquor to proactively promote the generation of hydrochloric acid during the second reaction.

In this regard, 10% calcium hydroxide was used for recovery liquid A. In addition, since it is confirmed that chloride ions tend to increase in recovery liquid B upon pursuing the recovery rate of recovery liquid B too far therewith, recovery was performed after passing through an intermediate trapping vessel (containing hydrogen peroxide). There were two possibilities for chloride ions at this time, either hydrochloric acid entrainment from a reaction tank is accompanied, or there is chlorine gas contamination.

As a result, the recovery rate for recovery liquid A was excellent, but chloride ions and chlorate ions both decreased despite reducing the sulfuric acid concentration in the reaction mother liquor during the first reaction. This was a result that is different from a result upon using low salt sodium hypochlorite as the raw material.

The recovery rate for recovery liquid B was 20-something percent. In addition, a large quantity of chloride ions was recovered in a chloride ion trapping vessel (containing hydrogen peroxide). Chloride ions in recovery liquid B also exhibited a high numerical value.

It is understood that these results are due to excessive generation of hydrochloric acid from strong acidification. Chloric acid is inherently more readily degraded to produce chlorine dioxide gas under strong acidic conditions. Meanwhile, a large quantity of chloride ions are already generated in the current general grade deteriorated sodium hypochlorite, leading to excessive generation of hydrochloric acid in the presence of strong sulfuric acid. It was found that, as a result thereof, a secondary reaction generating chlorine gas upon degradation of sodium chlorate progresses such that the primary reaction hardly progresses, resulting in dramatic decrease in the recovery rate. For this reason, further addition of sodium chloride is not needed when using general grade deteriorated sodium hypochlorite. At the same time, chloride ions generated in a large quantity in recovery liquid B were assumed to be chlorine gas contamination as a result of progress of a secondary reaction.

Therefore, a chlorate ion degradation reaction has a primary reaction and a secondary reaction, and the reaction ratio varies depending on the reaction conditions. In particular, it is understood that a secondary reaction progresses more readily when reaching near the end of the reaction and chlorate ions decrease.

$$2NaClO_3 + 4HCl \rightarrow 2ClO_2 + Cl_2 + 2NaCl + 2H_2O \ ..... \qquad (13) \ \text{primary reaction}$$

$$NaClO_3 + 6HCl \rightarrow 3Cl_2 + NaCl + 3H_2O \ ..... \qquad (14) \ \text{secondary reaction}$$

*For general grade deteriorated sodium hypochlorite in particular, the progression of primary and secondary reactions readily changes depending on the acidity or the amount of hydrochloric acid generated.

[Table 15-1]

(1) Raw material

| Raw material | Available chlorine concentration | $Cl^-$ | $ClO_2^-$ | $ClO_3^-$ |
|---|---|---|---|---|
| Deteriorated sodium hypochlorite(*) | 41784 | 196966 | 0 | 45204 |

*General grade sodium hypochlorite

(2) Reaction condition (first reaction, second reaction)

| | First reaction | Use reaction mother liquor | Second reaction |
|---|---|---|---|
| Deteriorated hypo | 200g | | 235g |
| 30% sulfuric acid | 35g | | — |
| 65% sulfuric acid | — | | 1200g |
| sodium chloride | — | | 10g |
| Reaction mother liquor (total) | 235g | | 1445g |
| Total added sulfuric acid concentration | 4.47% | | 54.71% |
| Reaction time | Ordinary temperature | | 65 to 70°C |
| Reaction time | 2h | | 5h |
| (Air time) | 1h50m | | 4h30m |

(3) Recovery liquid etc.

| | Recovery liquid A | Intermediate trap | Recovery liquid B |
|---|---|---|---|
| 10% calcium hydroxide | 200 g | – | – |
| 2N sodium hydroxide | – | – | 200 g |
| Deionized water | – | 200 g | – |
| Hydrogen peroxide | – | 10 g | 12 g |

[Table 15-2]

[Measurement results] (unit: ppm)

|  | Available chlorine concentration | $Cl^-$ | $ClO_2^-$ | $ClO_3^-$ |
|---|---|---|---|---|
| After first reaction | 4005 | 132829 | 0 | 36950 |
| After second reaction | 438 | 10132 | 132 | 0 |

|  | Available chlorine concentration | $Cl^-$ | $ClO_2^-$ | $ClO_3^-$ |
|---|---|---|---|---|
| Recovery liquid A | 31541 | 34819 | 0 | 0 |
| Trap | 29 | 77991 | 6 | 229 |
| Recovery liquid B | 14921 | 55822 | 7202 | 980 |

[Recovery rate of chlorite ion and available chlorine]

|  |  | Theoretical value | Measurement value | Recovery rate |
|---|---|---|---|---|
| Recovery liquid A | Available chlorine | 41784 | 31541 | 75.49 % |
| Recovery liquid B | Available chlorine | 72414 | 14921 | 20.61 % |
|  | $ClO_2^-$ | 34469 | 7202 | 20.89 % |

(Reaction 16 of deteriorated hypo

It is confirmed that if general grade deteriorated sodium hypochlorite is used, the progression of the primary reaction and secondary reaction is more likely to reverse compared to low salt deteriorated sodium hypochlorite. For example, chlorate ions tend to readily degrade already as of the first reaction. This is due to excessive generation of hydrochloric acid from a reaction between sulfuric acid and a large quantity of chloride ions generated in deteriorated sodium hypochlorite. Meanwhile, chloride ions produced in the raw material are difficult to remove in advance.

In this regard, for a reaction of general grade deteriorated sodium hypochlorite, a preliminary test was conducted, which added hydrogen peroxide in advance during the second reaction and then added sulfuric acid. This is for adding a chlorine dioxide gas generation pathway through a reaction between chlorate ions and hydrogen peroxide in the presence of sulfuric acid, and degrading and chlorinating excessively generated hydrochloric acid to prevent chlorate ions from generating chlorine gas due to progression of a secondary reaction.

In the preliminary test, addition of sulfuric acid during the second reaction was divided into two batches and an interim analysis was performed in order to study the change in composition of reaction mother liquor in a reaction. The final sulfuric acid concentration of the second reaction was set to 37.37 %.

It was found as a result thereof that 80% or more of chlorate ions in the reaction mother liquor is degraded in about 1 hour, even at the sulfuric acid concentration (21.57%) of the second reaction (1). However, gas could not be aptly recovered with the equipment settings in the preliminary test, such that the recovery rate could not be investigated.

Meanwhile, since 80% or more of chlorate ions degraded in about an hour in the second reaction and the composition was chlorite ions > chlorate ions, a tendency of higher ratio of chlorite ion generation due to progression in the primary reaction was confirmed.

However, when the reaction was continued by adding 65 w/w% sulfuric acid to set the final sulfuric acid concentration in the reaction mother liquor to 37.37%, nearly only chloride ions were recovered, and purity as chlorite ions of recovery liquid B decreased dramatically. It was found from the above that, since chlorate ions are degraded in a short period of time due to excessive generation of hydrochloric acid when using general grade deteriorated sodium hypochlorite, how the primary reaction is preferentially progressed during the reaction time becomes important.

[Table 16-1]

(1) Raw material

| Raw material | Available chlorine concentration | Cl⁻ | ClO₂⁻ | ClO₃⁻ |
|---|---|---|---|---|
| Deteriorated sodium hypochlorite (*) | 41784 | 193312 | 15 | 43651 |

*12% general grade sodium hypochlorite

(2) Reaction condition (first reaction, second reaction)

| | First reaction | Use reaction mother liquor | Second reaction (1) | Second reaction (2) |
|---|---|---|---|---|
| Deteriorated hypo | 200g | | 219.78g | |
| 50% sulfuric acid | 19.78g | | — | — |
| 65% sulfuric acid | — | | 93.83g | 187.66g |
| 35% hydrogen peroxide | — | | 15g | — |
| Reaction mother liquor (total) | 219.78g | | 328.61g | 516.27g |
| Total added sulfuric acid concentration | 4.5% | | 21.57% | 37.37% |
| Reaction temperature | Ordinary temperature | | Ordinary temperature | 60 to 70°C |
| Reaction time | 2h | | 1h20m | 5h |
| (Air time) | 1h50m | | 1h | 5h |

(3) Recovery liquid

| | Recovery liquid A | Recovery liquid B (1) | Recovery liquid B (2) |
|---|---|---|---|
| 2N sodium hydroxide | 200 g | 200 g | 200 g |
| Hydrogen peroxide | – | – | 10 g |

[Table 16-2]

[Measurement results] (unit: ppm)

| | Available chlorine | Cl⁻ | ClO₂⁻ | ClO₃⁻ | ClO₃⁻ degradation |
|---|---|---|---|---|---|

| | concentration | | | | rate |
|---|---|---|---|---|---|
| After first reaction | 636 | 147891 | 65 | 40818 | 6.49 % |
| After second reaction (1) | 949 | 113658 | 188 | 4354 | 83.61 % |
| After second reaction (2) | 107 | 65209 | 188 | 0 | 100.00 % |

| | Available chlorine concentration | $Cl^-$ | $ClO_2^-$ | $ClO_3^-$ |
|---|---|---|---|---|
| Recovery liquid A | 30664 | 31692 | 0 | 0 |
| Recovery liquid B (1) | 8200 | 1392 | 2319 | 3122 |
| Recovery liquid B (2) | 3106 | 12728 | 1158 | 204 |

[Recovery rate of chlorite ion and available chlorine]

| | | Theoretical value | Measurement value | Recovery rate |
|---|---|---|---|---|
| Recovery liquid A | Available chlorine | 40872 | 30664 | 75.02 % |
| Recovery liquid B (1) | Available chlorine | 74121 | 8200 | 11.06 % |
| | $ClO_2^-$ | 35282 | 2319 | 6.57 % |
| Recovery liquid B (2) | Available chlorine | 70592 | 3106 | 4.40 % |
| | $ClO_2^-$ | 33602 | 1158 | 3.45 % |

(Reaction 17 of deteriorated sodium hypochlorite)

The sulfuric acid concentration in the reaction mother liquor was set to 4.0% to confirm the sulfuric acid conditions for the first reaction of general grade deteriorated sodium hypochlorite. This is because degradation of chlorate ions was understood to result from generation of hydrochloric acid slightly expressively during the first reaction when the sulfuric acid concentration was set to 4.5%.

The sulfuric acid concentration in the reaction mother liquor during the second reaction was set to 30%. This was because about 20% of chlorate ions remained and did not reach complete degradation at a sulfuric acid concentration of about 20%. As in the previous experiment, hydrogen peroxide was added before adding sulfuric acid. Normally, hydrogen peroxide is added to suppress the generation of chlorine dioxide or generates chlorine dioxide gas at a low sulfuric acid concentration, thus often exhibiting different reactions depending on the conditions. In this reaction, hydrogen peroxide was added to suppress a secondary reaction.

Furthermore, air was sent in in large quantities immediately after addition of sulfuric acid, and filling an empty trapping vessel (reflux prevention/gas recovery vessel) was prioritized. This is because of the concern for gas returning to a solution or generation of a reverse reaction if a reaction tank is left filled with gas.

Available chlorine of recovery liquid B was measured every hour, and a recovery graph of the available chlorine was prepared. At the same time, the change in composition in recovery liquid B was studied.

It was confirmed as a result thereof that chlorate ions were completely degraded after 1 hour of the second reaction. It was also confirmed that the recovery rate of available chlorine at the time was 57.7% and the recovery rate 2 hours later did not change, so that the second reaction was completed in about 2 hours. Accordingly, it was found that the second reaction would be completed with reaction and recovery of at most about 2 hours and 30 minutes even if residual gas in the equipment or piping is taken into consideration. It can be understood that the composition in recovery liquid B was chlorite ions > chloride ions, so that the primary reaction preferentially progressed under the current test conditions.

Therefore, what is important in the second reaction is preferentially progressing the primary reaction and preventing the generation of chloride ions to increase the purity. For this reason, the recovery rate improved as a result of progressing the primary reaction in the current test, but the sulfuric acid concentration at which chlorate ions completely degrade and the sulfuric acid concentration at which the primary/secondary reactions change are not necessarily the same. The same applies to the addition and amount of hydrogen peroxide.

[Table 17-1]

(1) Raw material

| Raw material | Available chlorine concentration | $Cl^-$ | $ClO_2^-$ | $ClO_3^-$ |
|---|---|---|---|---|
| Deteriorated sodium hypochlorite (*) | 41784 | 193312 | 15 | 43651 |

*12% general grade sodium hypochlorite

(2) Reaction condition (first reaction, second reaction)

| | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 174.35g |
| 35% hydrogen peroxide | — | | 15g |
| Reaction mother liquor (total) | 217.39g | | 406.74g |
| Total added sulfuric acid concentration | 4.0% | | 30.0% |
| Reaction temperature | Ordinary temperature | | 60 to 70°C |
| Reaction time | 2h | | 3h |
| (Air time) | 1h50m | | 3h |

(3) Recovery liquid

| | Recovery liquid A | Recovery liquid B |
|---|---|---|
| 2N sodium hydroxide | 200 g | 200 g |
| Hydrogen peroxide | - | 10 g |

[Table 17-2]

[Measurement results] (unit: ppm)

| Reaction | | Available | $Cl^-$ | $ClO_2^-$ | $ClO_3^-$ | $ClO_3^-$ | |
|---|---|---|---|---|---|---|---|

| mother liquor | | chlorine concentration | | | | degradation rate |
|---|---|---|---|---|---|---|
| After first reaction | | 4719 | 155274 | 172 | 42874 | 1.8 % |
| After second reaction | After 1 hr | - | 73664 | 150 | 0 | 100.0 % |
| | After 2 hr | - | 94317 | 139 | 0 | 100.0 % |
| | After 3 hr | 347 | 87311 | 0 | 0 | 100.0 % |

| | | Available chlorine concentration | Cl⁻ | ClO₂⁻ | ClO₃⁻ |
|---|---|---|---|---|---|
| Recovery liquid A | | 25414 | 26386 | 0 | 0 |
| Recovery liquid B | After 1 hr | 40716 | 8747 | 20717 | 213 |
| | After 2 hr | 40447 | 9323 | 22976 | 274 |
| | After 3 hr | 39600 | 8488 | 20766 | 289 |
| Recovery liquid B (reserve) | After 2 hr | 317 | – | – | – |

[Recovery rate of chlorite ion and available chlorine]

| | | | Theoretical value | Measurement value | Recovery rate |
|---|---|---|---|---|---|
| Recovery liquid A | | Available chlorine | 40872 | 25414 | 62.2 % |
| Recovery liquid B | After 1 hr | Available chlorine | 70592 | 40716 | 57.7 % |
| | | ClO₂⁻ | 33602 | 20717 | 61.7 % |
| | After 2 hr | Available chlorine | 70592 | 40447 | 57.3 % |
| | | ClO₂⁻ | 33602 | 22976 | 68.4 % |
| | After 3 hr | Available chlorine | 70592 | 39600 | 56.1 % |
| | | ClO₂⁻ | 33602 | 20766 | 61.8 % |

[Table 18]

Recovery amoutn and composition in second reaction 1

(Reaction 18 of deteriorated hypo)

Since general grade sodium hypochlorite contains a large quantity of chloride ions, primary and secondary reactions are readily exchanged if reaction conditions become too excessive.

In this regard, a test was conducted to find which of the primary reaction and the secondary reaction preferentially progresses when reacted while setting the sulfuric acid concentration in the reaction mother liquor during the second reaction to 40.0%.

As a result, chloride ions were generated from recovery liquid B and chloride ions > chlorite ions when the sulfuric acid concentration in the reaction mother liquor during the second reaction was increased to 40.0 %. Thus, it is understood that the secondary reaction preferentially progressed. Since the recovery rate also decreased dramatically, it was found that generation of chlorine gas and a secondary reaction progress when the sulfuric acid concentration in the reaction mother liquor is increased to 40.0 %, even if hydrogen peroxide is added.

[Table 19-1]

(1) Raw material

| Raw material | Available chlorine concentration | Cl⁻ | ClO₂⁻ | ClO₃⁻ |
|---|---|---|---|---|
| Deteriorated sodium hypochlorite (*) | 40764 | 202482 | 32 | 47442 |

*12% general grade sodium hypochlorite

(2) Reaction condition (first reaction, second reaction)

| | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 337.04 g |
| 35% hydrogen peroxide | — | | 15g |
| Reaction mother liquor (total) | 217.39g | | 569.43 g |
| Total added sulfuric acid concentration | 4.0% | | 40.0% |
| Reaction temperature | Ordinary temperature | | 60 to 70°C |
| Reaction time | 1h | | 2h |
| (Air time) | 1h | | 2h |

(3) Recovery liquid

| | Recovery liquid A | Recovery liquid B |
|---|---|---|
| 2N sodium hydroxide | 200 g | 200 g |
| Hydrogen peroxide | – | 10 g |

[Table 19-2]

[Measurement results] (unit: ppm)

| Reaction | | Available | Cl⁻ | ClO₂⁻ | ClO₃⁻ | ClO₃⁻ |
|---|---|---|---|---|---|---|

| mother liquor | | chlorine concentration | | | | degradation rate |
|---|---|---|---|---|---|---|
| After first reaction | | 4625 | 150555 | 0 | 40811 | 14.0 % |
| After second reaction | After 1 hr | - | 62671 | 0 | 0 | 100.0 % |
| | After 2 hr | 166 | 49244 | 0 | 0 | 100.0 % |

| | | Available chlorine concentration | $Cl^-$ | $ClO_2^-$ | $ClO_3^-$ |
|---|---|---|---|---|---|
| Recovery liquid A | | 25805 | 28427 | 19 | 0 |
| Recovery liquid B | After 1 hr | 32076 | 36712 | 16083 | 1323 |
| | After 2 hr | 31467 | 39656 | 17514 | 1704 |

[Recovery rate of chlorite ion and available chlorine]

| | | | Theoretical value | Measurement value | Recovery rate |
|---|---|---|---|---|---|
| Recovery liquid A | | Available chlorine | 40764 | 25805 | 63.3 % |
| Recovery liquid B | After 1 hr | Available chlorine | 76722 | 32076 | 41.8 % |
| | | $ClO_2^-$ | 36520 | 16083 | 44.0 % |
| | After 2 hr | Available chlorine | 76722 | 31467 | 41.0 % |
| | | $ClO_2^-$ | 36520 | 17514 | 48.0 % |

[Table 20]

Recovery amount and composition in second reaction 2

(Reaction 19 of deteriorated hypo)

When the sulfuric acid concentration in the reaction mother liquor during the second reaction was 40.0%, a secondary reaction progressed, and a large quantity of chloride ions derived from chlorine gas were generated, which deteriorated the recovery rate.

Next, a test was conducted with the sulfuric acid concentration in the reaction mother liquor set to 30.0% and without addition of hydrogen peroxide in advance. By not suppressing chlorine gas in the second reaction, it was expected that the reactivity would improve as a whole while a large quantity of chloride ions would be generated in this test.

As a result, hydrogen peroxide in recovery liquid B was insufficient so that hydrogen peroxide was further added after 24 minutes during the second reaction. This is understood as follows: chlorine gas or chlorine dioxide gas was recovered beyond expectation in recovery liquid B, and as a result of insufficient hydrogen peroxide, chlorine gas became chloride ions, and chlorine dioxide gas was recovered as chlorate ions. Since the composition of recovery was chloride ions > chlorite ions, it is understood that chlorine gas contamination due to progression of a secondary reaction was the majority thereof.

While there was loss of recovery due to insufficient hydrogen peroxide in recovery liquid B, an increasing trend was found in the recovery rate of chlorite ions as a result of increased reactivity. Although the recovery rate of chlorite ions increased, a large quantity of chloride ions are concurrently generated, so that the purity would decrease. Without a certain degree of purity, high concentration recovery would be difficult when concentrated and dried, so that high grade chlorinated lime would no longer be able to be processed. In any case, it is necessary to suppress the generation of chlorine gas by adding hydrogen peroxide during the second reaction and to add an intermediate trapping vessel for preventing chlorine gas from contaminating recovery liquid B.

[Table 21-1]

(1) Raw material

| Raw material | Available chlorine concentration | $Cl^-$ | $ClO_2^-$ | $ClO_3^-$ |
|---|---|---|---|---|
| Deteriorated sodium hypochlorite (*) | 40764 | 202482 | 32 | 47442 |

*12% general grade sodium hypochlorite

(2) Reaction condition (first reaction, second reaction)

| | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 161.49g |
| 35% hydrogen peroxide | — | | — |
| Reaction mother liquor (total) | 217.39g | | 378.88g |
| Total added sulfuric acid concentration | 4.0% | | 30.0% |
| Reaction temperature | Ordinary temperature | | 60 to 70°C |
| Reaction time | 1h | | 2h |
| (Air time) | 1h | | 2h |

(3) Recovery liquid

| | Recovery liquid A | Recovery liquid B |
|---|---|---|
| 2N sodium hydroxide | 200 g | 200 g |
| Hydrogen peroxide | – | 12.0 g + 2.5 g* |

*Further addition of hydrogen peroxide (due to insufficient hydrogen peroxide upon recovery)

[Table 21-2]

[Measurement results] (unit: ppm)

| Reaction mother liquor | | Available chlorine concentration | $Cl^-$ | $ClO_2^-$ | $ClO_3^-$ | $ClO_3^-$ degradation rate |
|---|---|---|---|---|---|---|
| After first reaction | | 4233 | 151082 | 0 | 41338 | 12.9 % |
| After second reaction | After 1 hr | – | 63548 | 26 | 0 | 100.0 % |
| | After 2 hr | 89 | 57550 | 17 | 0 | 100.0 % |

| | | Available chlorine concentration | Cl⁻ | ClO₂⁻ | ClO₃⁻ |
|---|---|---|---|---|---|
| Recovery liquid A | | 27195 | 32280 | 0 | 0 |
| Recovery liquid B | After 1 hr | 42316 | 56848 | 23654 | 3664 |
| | After 2 hr | 42932 | 55804 | 23403 | 3679 |

[Recovery rate of chlorite ion and available chlorine]

| | | | Theoretical value | Measurement value | Recovery rate |
|---|---|---|---|---|---|
| Recovery liquid A | | Available chlorine | 40764 | 27195 | 66.7 % |
| Recovery liquid B | After 1 hr | Available chlorine | 75025 | 42316 | 56.4 % |
| | | ClO₂⁻ | 35754 | 23654 | 66.2 % |
| | After 2 hr | Available chlorine | 75025 | 42932 | 57.2 % |
| | | ClO₂⁻ | 35754 | 23403 | 65.5 % |

[Table 22]

Recovery amount and composition in second reaction 3

(Reaction 20 of deteriorated hypo)

To study the reaction conditions in cases using general grade sodium hypochlorite, the first reaction is conducted by using general grade deteriorated sodium hypochlorite as a raw material and setting the sulfuric acid concentration in the reaction mother liquor to 4.0% using 50 w/w % sulfuric acid while adding air. The reaction time therein was one hour. Recovery used sodium hydroxide (or calcium hydroxide) for recovery liquid A.

The second reaction was conducted by adding hydrogen peroxide in advance to suppress the generation of chloride ions in the reaction mother liquor that has completed the first reaction and setting the sulfuric acid concentration in the reaction mother liquor to 30.0% using 65 w/w% sulfuric acid and raising the temperature to 60 to 70°C while sending air. The reaction time therein can be 2 hours to 2 hours and 30 minutes. Recovery used sodium hydroxide and hydrogen peroxide for recovery liquid B via an intermediate trapping vessel containing hydrogen peroxide. Chlorine gas washing by having a hydrogen peroxide solution pass through in the intermediate trapping vessel serves the role of removing chloride ions. As a result, highly pure recovery liquid B is recovered, and a subsequent drying step achieves a level where it is possible to concentrate the product to a high concentration.

Both chlorine dioxide gas and chlorine gas are necessarily generated in the present invention, as shown in reaction formulas (13) and (14). Even if chlorine gas is washed and removed by using hydrogen peroxide in an intermediate trapping vessel and chlorine dioxide gas is allowed to pass therethrough, only about half the amount of chlorine gas generated can be removed according to reaction formulas (15) and (16). If chlorine gas is removed using another method at a high level, chlorine dioxide gas, which is also an oxidant, would react. Thus, the recovery liquid in the present invention using sodium hypochlorite as a raw material necessarily contains chloride ions.

$$2NaClO_3 + 4HCl \rightarrow 2ClO_2 + Cl_2 + 2NaCl + 2H_2O \ldots \qquad (13) \text{ primary reaction}$$

$$NaClO_3 + 6HCl \rightarrow 3Cl_2 + NaCl + 3H_2O \ldots \qquad (14) \text{ secondary reaction}$$

$$Cl_2 + 3H_2O_2 \rightarrow 2HCl + 2H_2O + 2O_2 \ldots \qquad (15)$$

$$2HCl + H_2O_2 \rightarrow 2H_2O + Cl_2 \ldots \qquad (16)$$

[Table 23-1]

(1) Raw material

| Raw material | Available chlorine concentration | $Cl^-$ | $ClO_2^-$ | $ClO_3^-$ |
|---|---|---|---|---|
| Deteriorated sodium hypochlorite (*) | 40209 | 191805 | 57 | 47063 |

*12% general grade sodium hypochlorite

## (2) Reaction condition (first reaction, second reaction)

|  | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 174.35g |
| 35% hydrogen peroxide | — | | 15g |
| Reaction mother liquor (total) | 217.39g | | 406.74g |
| Total added sulfuric acid concentration | 4.0% | | 30.0% |
| Reaction temperature | Ordinary temperature | | 60 to 70°C |
| Reaction time | 1h | | 2h |
| (Air time) | 1h | | 2h |

## (3) Recovery liquid

|  | Recovery liquid A | Intermediate trap | Recovery liquid B |
|---|---|---|---|
| 2N sodium hydroxide | 200 g | – | 200 g |
| Hydrogen peroxide | – | 20 g | 20 g |
| Deionized water | – | 200 g | – |

[Table 23-2]

[Measurement results] (unit: ppm)

| Reaction mother liquor |  | Available chlorine concentration | $Cl^-$ | $ClO_2^-$ | $ClO_3^-$ | $ClO_3^-$ degradation rate |
|---|---|---|---|---|---|---|
| After first reaction |  | 4478 | 155929 | 153 | 42275 | 10.2 % |
| After second reaction | After 1 hr | – | 81120 | 40 | 0 | 100.0 % |
|  | After 2 hr | 93 | 95345 | 0 | 0 | 100.0 % |

| | | Available chlorine concentration | Cl⁻ | ClO₂⁻ | ClO₃⁻ |
|---|---|---|---|---|---|
| Recovery liquid A | | 27000 | 30005 | 0 | 0 |
| Intermediate trap | After 1 hr | 3176 | 3170 | 243 | 55 |
| | After 2 hr | 929 | 3191 | 146 | 69 |
| Recovery liquid B | After 1 hr | 38709 | 4405 | 19471 | 31 |
| | After 2 hr | 39464 | 4474 | 19651 | 34 |

[Recovery rate of chlorite ion and available chlorine]

| | | | Theoretical value | Measurement value | Recovery rate |
|---|---|---|---|---|---|
| Recovery liquid A | | Available chlorine | 40209 | 27000 | 67.2 % |
| Recovery liquid B | After 1 hr | Available chlorine | 72650 | 38709 | 53.3 % |
| | | ClO₂⁻ | 34581 | 19471 | 56.3 % |
| | After 2 hr | Available chlorine | 72650 | 39464 | 54.3 % |
| | | ClO₂⁻ | 34581 | 19651 | 56.8 % |

[Table 24]

(Reaction 21 of deteriorated hypo)

The second reactions up to this point were performed at a temperature of 60 to 70°C. However, the reactivity is high when using general grade sodium hypochlorite, and chloride ions are generated in large quantity and in excess under the same reaction conditions for low salt grade sodium hypochlorite. Thus, an adjustment such as reducing the sulfuric acid concentration in the reaction mother liquor was required.

In this regard, the reactivity at ordinary temperature (no temperature control) during the second reaction was studied.

It was found as a result thereof that the recovery rate does not change significantly even without particularly adjusting the temperature during the second reaction when using general grade sodium hypochlorite as the raw material.

[Table 25-1]

(1) Raw material

| Raw material | Available chlorine concentration | $Cl^-$ | $ClO_2^-$ | $ClO_3^-$ |
|---|---|---|---|---|
| Deteriorated sodium hypochlorite (*) | 36673 | 184639 | 30 | 47093 |

*12% general grade sodium hypochlorite

(2) Reaction condition (first reaction, second reaction)

| | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 174.35g |
| 35% hydrogen peroxide | — | | 15g |
| Reaction mother liquor (total) | 217.39g | | 406.74g |
| Total added sulfuric acid concentration | 4.0% | | 30.0% |
| Reaction temperature | Ordinary temperature | | Ordinary temperature |
| Reaction time | 1h | | 2h |
| (Air time) | 1h | | 2h |

(3) Recovery liquid

| | Recovery liquid A | Intermediate trap | Recovery liquid B |
|---|---|---|---|
| 2N sodium hydroxide | 200 g | - | 200 g |
| Hydrogen peroxide | - | 20 g | 20 g |
| Deionized water | - | 200 g | - |

[Table 25-2]

[Measurement results] (unit: ppm)

| Reaction mother liquor | | Available chlorine concentration | $Cl^-$ | $ClO_2^-$ | $ClO_3^-$ | $ClO_3^-$ degradation rate |
|---|---|---|---|---|---|---|
| After first reaction | | 4244 | 142437 | 0 | 39320 | 16.51% |
| After second reaction | After 1 hr | - | 86240 | 0 | 0 | 100.0 % |
| | After 2 hr | 0 | 83357 | 0 | 0 | 100.0 % |

| | | Available chlorine concentration | Cl$^-$ | ClO$_2$$^-$ | ClO$_3$$^-$ |
|---|---|---|---|---|---|
| Recovery liquid A | | 25824 | 23752 | 16 | 0 |
| Intermediate trap | After 1 hr | 1421 | 2861 | 413 | 65 |
| | After 2 hr | 259 | 2751 | 184 | 76 |
| Recovery liquid B | After 1 hr | 38665 | 3541 | 20240 | 28 |
| | After 2 hr | 39915 | 3943 | 20667 | 36 |

[Recovery rate of chlorite ion and available chlorine]

| | | | Theoretical value | Measurement value | Recovery rate |
|---|---|---|---|---|---|
| Recovery liquid A | | Available chlorine | 36673 | 25824 | 70.4 % |
| Recovery liquid B | After 1 hr | Available chlorine | 72696 | 38665 | 53.2% |
| | | ClO$_2$$^-$ | 34603 | 20240 | 58.5% |
| | After 2 hr | Available chlorine | 72696 | 39915 | 54.9% |
| | | ClO$_2$$^-$ | 34603 | 20667 | 59.7% |

(Reaction 22 of deteriorated hypo)

The amount of sodium hydroxide in recovery liquid B in the second reaction is studied. It is preferable that the amount of alkali in the second reaction is excessive for preventing recovery leakage. However, a high amount of inorganic alkali salt reduces the concentration ratio when dried, leading to a decrease in the final concentration.

In this regard, the change in recovery rates was studied with 0.5 N sodium hydroxide in recovery liquid B.

It was found as a result thereof that T. AL of recovery liquid B before the second reaction was 515.46 and 149.25 after completion of the reaction, so that 366.21 of T. AL was consumed in the reaction. The recovery rate of recovery liquid tends to decrease somewhat, but the drying efficiency increases. Thus, the solution can be dried into powder comprising available chlorine at a very high concentration.

However, chlorate ions contained in deteriorated sodium hypochlorite are present at a low concentration. Further, chloride ions are also produced as a byproduct. Thus, it is impossible to attain a recovery rate that is equivalent to a theoretical value. In addition, about 35-fold concentration is required for drying and concentrating the recovery liquid obtained therefrom to a high concentration. As a result, the residual alkali and chloride ions are also concentrated 35-fold.

To materialize the above, it is necessary to control chloride ions and the amount of alkali in the recovery liquid. Meanwhile, when using sodium hypochlorite as a raw material, a certain amount of chloride ions with respect to chlorite ions is necessarily generated. Thus, it is important to control inorganic salt and residual alkali in recovery liquid B.

[Table 26-1]

(1) Raw material

| Raw material | Available chlorine concentration | $Cl^-$ | $ClO_2^-$ | $ClO_3^-$ |
|---|---|---|---|---|
| Deteriorated sodium hypochlorite (*) | 34887 | 177329 | 34 | 45837 |

*12% general grade sodium hypochlorite

(2) Reaction condition (first reaction, second reaction)

| | First reaction | Use reaction mother liquor | Second reaction |
|---|---|---|---|
| Deteriorate hypo | 200g | | 217.39g |
| 50% sulfuric acid | 17.39g | | — |
| 65% sulfuric acid | — | | 174.35g |
| 35% hydrogen peroxide | — | | 15g |
| Reaction mother liquor (total) | 217.39g | | 406.74g |
| Total added sulfuric acid concentration | 4.0% | | 30.0% |
| Reaction temperature | Ordinary temperature | | Ordinary temperature |
| Reaction time | 1h | | 2h |
| (Air time) | 1h | | 2h |

(3) Recovery liquid

| | Recovery liquid A | Intermediate trap | Recovery liquid B |
|---|---|---|---|
| 0.5N hydrogen peroxide | 200 g | – | 200 g |
| Hydrogen peroxide | – | 20 g | 20 g |
| Deionized water | – | 200 g | – |

[Table 26-2]

[Measurement results] (unit: ppm)

| Reaction mother liquor | | Available chlorine concentration | Cl⁻ | ClO₂⁻ | ClO₃⁻ | ClO₃⁻ degradation rate |
|---|---|---|---|---|---|---|
| After first reaction | | 3500 | 152845 | 17 | 42230 | 7.9 % |
| After second reaction | After 1 hr | – | 88897 | 37 | 0 | 100.0 % |
| | After 2 hr | 0 | 81825 | 43 | 0 | 100.0 % |

| | | Available chlorine concentration | Cl⁻ | ClO₂⁻ | ClO₃⁻ |
|---|---|---|---|---|---|
| Recovery liquid A | | 25477 | 28239 | 0 | 0 |
| Intermediate trap | After 1 hr | 1939 | 2444 | 318 | 64 |
| | After 2 hr | 733 | 2437 | 193 | 78 |
| Recovery liquid B | After 1 hr | 36030 | 2931 | 16796 | 9 |
| | After 2 hr | 36668 | 3077 | 16451 | 8 |

[Recovery rate of chlorite ion and available chlorine]

| | | | Theoretical value | Measurement value | Recovery rate |
|---|---|---|---|---|---|
| Recovery liquid A | | Available chlorine | 34887 | 25477 | 73.0 % |
| Recovery liquid B | After 1 hr | Available chlorine | 70757 | 36030 | 50.9 % |
| | | ClO₂⁻ | 33681 | 16796 | 49.9 % |
| | After 2 hr | Available chlorine | 70757 | 36668 | 51.8 % |
| | | ClO₂⁻ | 33681 | 16451 | 48.8 % |

[Table 26-3]

[T.AL before and after recovery of recovery liquid B]

| Before recovery | 515.46 |
|---|---|
| After recovery | 149.25 |
| Loss due to recovery | 366.21 |

[Drying efficiency of only recovery liquid B]

| | Concentration factor | $Cl^-$ | $ClO_2^-$ | $ClO_3^-$ | Available chlorine |
|---|---|---|---|---|---|
| Before drying | 1 | 3077 | 16451 | 8 | 36668 |
| After drying (120 min) | 35 | 98535 | 436480 | 700 | 936850 |
| Dry yield (*) | | 91.5 % | 75.8 % | 250 % | 73.0 % |

Actual measurement value after drying/(Before drying × 35)

(Reaction 23 of deteriorated hypo)

To conduct a test for investigating whether the sulfuric acid concentrations in the first reaction and the second reaction are determined by the composition of the raw material, reaction conditions were investigated using low salt grade sodium hypochlorite of another manufacturer with a large amount of chloride was used as the raw material. Mohr's method was used for the measurement of the chloride concentration in the raw material for quantification that is separate from chlorine ions in sodium hypochlorite to study the reaction conditions for chloride concentration and sulfuric acid concentration.

It was found as a result thereof that when reacted at a sulfuric acid concentration in the reaction mother liquor of 6.37% with respect to available chlorine at 50,511 ppm and chloride concentration at 42186 ppm in the raw material, available chlorine decreased to 598 ppm and chlorine was able to be recovered in recovery liquid A, but at the same time decreased due to degradation of chlorate ions, while there was excess of sulfuric acid. When reacted with the sulfuric acid concentration in the reaction mother liquor in the second reaction at 58.00%, chloride ions derived from chlorine in recovery liquid B were generated. It is understood that a secondary reaction mainly progressed. For this reason, it was found that it is excessive for a second reaction with the sulfuric acid concentration set to 58.00% with respect to a chloride concentration in the reaction mother liquor after the completion of the first reaction of 15,523 ppm.

[Table 27-1]

(1) Raw material

| Raw material | Available | Cl | $Cl^-$ | $ClO_2^-$ | $ClO_3^-$ |
|---|---|---|---|---|---|

| | chlorine concentration | | | | |
|---|---|---|---|---|---|
| Deteriorated sodium hypochlorite (*) | 50511 | 42186 | 65305 | 193 | 33644 |

*12% low salt grade sodium hypochlorite

(2) Reaction condition (first reaction, second reaction)

| | First reaction | Use reaction mother liquor | Second reaction |
|---|---|---|---|
| Deteriorated hypo | 180g | | 206.28g |
| 50% sulfuric acid | 26.28g | | — |
| 65% sulfuric acid | — | | 1633.32g |
| 35% hydrogen peroxide | — | | 13.5g |
| Reaction mother liquor (total) | 206.28g | | 1853.10g |
| Total added sulfuric acid concentration | 6.37% | | 58.0% |
| Reaction temperature | Ordinary temperature | | 40 to 50°C |
| Reaction time | 1h | | 2h |
| (Air time) | 1h | | 2h |

(3) Recovery liquid

| | Recovery liquid A | Intermediate trap | Recovery liquid B |
|---|---|---|---|
| 10% calcium hydroxide | 180 g | - | - |
| 0.6N sodium hydroxide | - | - | 180 g |
| Hydrogen peroxide | - | - | 9 g |

*For low salt grade sodium hypochlorite, no intermediate trap

[Table 27-2]

[Measurement results] (unit: ppm)

| Reaction mother liquor | | Available chlorine concentration | Cl | Cl⁻ | ClO₂⁻ | ClO₃⁻ | ClO₃⁻ degradation rate |
|---|---|---|---|---|---|---|---|
| After first reaction | After 1 hr | 598 | 15523 | 17683 | 0 | 29507 | 12.3 % |
| After second reaction | After 2 hr | 0 | 1144 | 2483 | 0 | 0 | 100.0 % |

| | | Available chlorine concentration | Cl | Cl⁻ | ClO₂⁻ | ClO₃⁻ |
|---|---|---|---|---|---|---|
| Recovery liquid A | After 1 hr | 42570 | – | 50386 | 0 | 0 |
| Recovery liquid B | After 2 hr | 30209 | – | 20646 | 14722 | 80 |

[Recovery rate of chlorite ion and available chlorine]

| | | | Theoretical value | Measurement value | Recovery rate |
|---|---|---|---|---|---|
| Recovery liquid A | After 1 hr | Available chlorine | 50511 | 42570 | 84.28 % |
| Recovery liquid B | After 2 hr | Available chlorine | 51160 | 30209 | 59.05 % |
| | | ClO₂⁻ | 24352 | 14722 | 60.46 % |

(Reaction 24 of deteriorated hypo)

Reproducibility was examined by using first reaction conditions that are the same as those in (Reaction 23 of deteriorated hypochlorite), and the sulfuric acid concentration in the reaction mother liquor during the second reaction was set to 30.0%.

It was confirmed as a result thereof that 5.15% of chlorate ions were degraded after the first reaction and sulfuric acid was also excessive for chloride concentration in the raw material of 43429 ppm, and there is somewhat of a variation in the degradation rate of chlorate ions. While the second reaction was conducted at a 30% sulfuric acid concentration for a chloride concentration in the reaction mother liquor after the completion of the first reaction of 18225 ppm, chloride ions and chlorate ions were hardly generated in recovery liquid B, which enabled reactions that were mainly the primary reaction and recovery at a high purity.

[Table 28-1]

(1) Raw material

| Raw material | Available chlorine concentration | Cl | Cl$^-$ | ClO$_2^-$ | ClO$_3^-$ |
|---|---|---|---|---|---|
| Deteriorated sodium hypochlorite (*) | 49897 | 43429 | 62483 | 39 | 32922 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 229.20g |
| 50% sulfuric acid | 29.20g | | — |
| 65% sulfuric acid | — | | 167.60g |
| 35% hydrogen peroxide | — | | 15g |
| Reaction mother liquor (total) | 229.20g | | 411.80g |
| Total sulfuric acid concentration | 6.37% | | 30.0% |
| Reaction temperature | Ordinary temperature | | 40 to 50°C |
| Reaction time | 1h | | 2h |
| (Air time) | 1h | | 2h |

(3) Recovery liquid

| | Recovery liquid A | Intermediate trap | Recovery liquid B |
|---|---|---|---|
| 10% calcium hydroxide | 200 g | – | – |
| 0.6N sodium | – | – | 200 g |

| hydroxide | | | |
|---|---|---|---|
| Hydrogen peroxide | – | – | 10 g |

*For low salt grade sodium hypochlorite, no intermediate trap

[Table 28-2]

[Measurement results] (unit: ppm)

| Reaction mother liquor | | Available chlorine concentration | Cl | Cl⁻ | ClO₂⁻ | ClO₃⁻ | ClO₃⁻ degradation rate |
|---|---|---|---|---|---|---|---|
| After first reaction | After 1 hr | 267 | | 18225 | 21460 | 0 | 31227 | 5.15 % |
| After second reaction | After 2 hr | 0 | | 13965 | 13931 | 0 | 0 | 100.0 % |

| | | Available chlorine concentration | Cl | Cl⁻ | ClO₂⁻ | ClO₃⁻ |
|---|---|---|---|---|---|---|
| Recovery liquid A | After 1 hr | 39421 | – | 46547 | 63 | 0 |
| Recovery liquid B | After 2 hr | 36676 | – | 107 | 18079 | 29 |

[Recovery rate of chlorite ion and available chlorine]

| | | | Theoretical value | Measurement value | Recovery rate |
|---|---|---|---|---|---|
| Recovery liquid A | After 1 hr | Available chlorine | 49897 | 39421 | 79.00 % |
| Recovery liquid B | After 2 hr | Available chlorine | 53241 | 36676 | 68.89 % |
| | | ClO₂⁻ | 25343 | 18079 | 71.34 % |

(Reaction 25 of deteriorated hypo)

Manufacturing was performed with the sulfuric acid concentration in the reaction mother liquor during the first reaction set to 4.00% for a chloride concentration in the raw material of 41983 ppm.

As a result, 14397 ppm of available chlorine remained in the reaction mother liquor after the completion of the first reaction, and chlorate ions increased 13.82%, but reaction was insufficient for available chlorine.

If there is a large quantity of available chlorine in the reaction mother liquor, there is a concern for generation of chloride ions in recovery liquid B, but in actuality, there were few chloride ions with recovery at a high purity, and the recovery rate also increased due to the effect of increased chlorate ions in the reaction mother liquor after the completion of the first reaction.

[Table 29-1]

(1) Raw material

| Raw material | Available chlorine concentration | Cl | Cl$^-$ | ClO$_2^-$ | ClO$_3^-$ |
|---|---|---|---|---|---|
| Deteriorated sodium hypochlorite (*) | 50989 | 41983 | 71421 | 136 | 34262 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 217.39g |
| 50% sulfuric acid | 17.39g | | — |
| 65% sulfuric acid | — | | 174.35g |
| 35% hydrogen peroxide | — | | 15g |
| Reaction mother liquor (total) | 217.39g | | 406.74g |
| Total added sulfuric acid concentration | 4.00% | | 30.0% |
| Reaction temperature | Ordinary temperature | | 40 to 50°C |
| Reaction time | 1h | | 2h |
| (Air time) | 1h | | 2h |

(3) Recovery liquid

| | Recovery liquid A | Intermediate trap | Recovery liquid B |
|---|---|---|---|
| 10% calcium hydroxide | 200 g | - | - |
| 0.6N sodium hydroxide | - | - | 200 g |
| Hydrogen peroxide | - | - | 10 g |

*For low salt grade sodium hypochlorite, no intermediate trap

[Table 29-2]

[Measurement results] (unit: ppm)

| Reaction mother liquor | | Available chlorine concentration | Cl | Cl⁻ | ClO₂⁻ | ClO₃⁻ | ClO₃⁻ degradation rate |
|---|---|---|---|---|---|---|---|
| After first reaction | After 1 hr | 14397 | 33344 | 49041 | 0 | 38998 | −13.82 % |
| After second reaction | After 2 hr | 0 | 24229 | 36841 | 0 | 0 | 100.0 % |

(Reaction 26 of deteriorated hypo)

Manufacturing was performed with the sulfuric acid concentration in the reaction mother liquor during the first reaction set to 5.00% for a chloride concentration in the raw material of 41416 ppm. It was confirmed as a result thereof that 13281 ppm of available chlorine remained in the reaction mother liquor after the completion of the first reaction, and chlorate ions decreased 1.36%, which was somewhat excessive.

[Table 30-1]

(1) Raw material

| Raw material | Available chlorine concentration | Cl | Cl⁻ | ClO₂⁻ | ClO₃⁻ |
|---|---|---|---|---|---|
| Deteriorated sodium hypochlorite (*) | 50017 | 41416 | 68782 | 173 | 33191 |

*12% low salt grade sodium hypochlorite

(2) Reaction condition (first reaction, second reaction)

| | First reaction | Use reaction | Second reaction |
|---|---|---|---|

| Deteriorated hypo | 200g | mother liquor | 222.22g |
| 50% sulfuric acid | 22.22g | | — |
| 65% sulfuric acid | — | | 171.59g |
| 35% hydrogen peroxide | — | | 15g |
| Reaction mother liquor (total) | 222.22g | | 408.81g |
| Total added sulfuric acid concentration | 5.00% | | 30.0% |
| Reaction temperature | Ordinary temperature | | 40 to 50°C |
| Reaction time | 1h | | 2h |
| (Air time) | 1h | | 2h |

(3) Recovery liquid

| | Recovery liquid A | Intermediate trap | Recovery liquid B |
| 10% calcium hydroxide | 200g | – | – |
| 0.6N sodium hydroxide | – | – | 200 g |
| Hydrogen peroxide | – | – | 10 g |

*For low salt grade sodium hypochlorite, no intermediate trap

[Table 30-2]

[Measurement results] (unit: ppm)

| Reaction mother liquor | | Available chlorine concentration | Cl | Cl$^-$ | ClO$_2^-$ | ClO$_3^-$ | ClO$_3^-$ degradation rate |
|---|---|---|---|---|---|---|---|
| After first reaction | After 1 hr | 13281 | 25145 | 35178 | 0 | 32740 | 1.36 % |
| After second reaction | After 2 hr | 0 | 24229 | 36841 | 0 | 0 | 100.0 % |

| | | Available chlorine concentration | Cl | Cl$^-$ | ClO$_2^-$ | ClO$_3^-$ |
|---|---|---|---|---|---|---|

# EP 3 777 537 A1

| Recovery liquid A | After 1 hr | 27397 | – | 31279 | 0 | 0 |
|---|---|---|---|---|---|---|
| Recovery liquid B | After 2 hr | 37758 | – | 1659 | 19517 | 16 |

[Recovery rate of chlorite ion and available chlorine]

| | | | Theoretical value | Measurement value | Recovery rate |
|---|---|---|---|---|---|
| Recovery liquid A | After 1 hr | Available chlorine | 50017 | 27397 | 54.78 % |
| Recovery liquid B | After 2 hr | Available chlorine | 53676 | 37758 | 70.35 % |
| | | $ClO_2^-$ | 25550 | 19517 | 76.39 % |

(Reaction 27 of deteriorated hypo)

Manufacturing was performed with the sulfuric acid concentration in the reaction mother liquor during the first reaction set to 6.00% for a chloride concentration in the raw material of 41416 ppm. It was confirmed as a result thereof that 13281 ppm of available chlorine remained in the reaction mother liquor after the completion of the first reaction, and chlorate ions decreased 3.6%, which was excessive. Further, the recovery rate of recovery liquid B decreased for the decrease in chlorate ions.

[Table 31-1]

(1) Raw material

| Raw material | Available chlorine concentration | Cl | Cl$^-$ | ClO$_2^-$ | ClO$_3^-$ |
|---|---|---|---|---|---|
| Deteriorated sodium hypochlorite (*) | 50017 | 41416 | 68782 | 173 | 33191 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 227.27g |
| 50% sulfuric acid | 27.27g | | — |
| 65% sulfuric acid | — | | 168.70g |
| 35% hydrogen peroxide | — | | 15g |
| Reaction mother liquor (total) | 227.27g | | 410.97g |
| Total added sulfuric acid concentration | 6.00% | | 30.0% |
| Reaction temperature | Ordinary temperature | | 40 to 50°C |
| Reaction time | 1h | | 2h |
| (Air time) | 1h | | 2h |

(3) Recovery liquid

| | Recovery liquid A | Intermediate trap | Recovery liquid B |
|---|---|---|---|
| 10% calcium hydroxide | 200g | - | - |
| 0.6N sodium hydroxide | - | - | 200 g |
| Hydrogen peroxide | - | - | 10 g |

*For low salt grade sodium hypochlorite, no intermediate trap

[Table 31-2]

[Measurement results] (unit: ppm)

| Reaction mother liquor | | Available chlorine concentration | Cl | Cl$^-$ | ClO$_2^-$ | ClO$_3^-$ | ClO$_3^-$ degradation rate |
|---|---|---|---|---|---|---|---|
| After first reaction | After 1 hr | 156 | 18411 | 23358 | 0 | 31996 | 3.60 % |
| After second reaction | After 2 hr | 0 | 12798 | 17474 | 0 | 0 | 100.0 % |

| | | Available chlorine concentration | Cl | Cl$^-$ | ClO$_2^-$ | ClO$_3^-$ |
|---|---|---|---|---|---|---|
| Recovery liquid A | After 1 hr | 39835 | – | 47737 | 0 | 0 |
| Recovery liquid B | After 2 hr | 35482 | – | 149 | 19713 | 55 |

[Recovery rate of chlorite ion and available chlorine]

| | | | Theoretical value | Measurement value | Recovery rate |
|---|---|---|---|---|---|
| Recovery liquid A | After 1 hr | Available chlorine | 50017 | 39835 | 79.64 % |
| Recovery liquid B | After 2 hr | Available chlorine | 53676 | 35482 | 66.10 % |
| | | ClO$_2^-$ | 25550 | 19713 | 77.16 % |

(Reaction 28 of deteriorated hypo)

Manufacturing was performed with the sulfuric acid concentration in the reaction mother liquor during the first reaction set to 4.50% for a chloride concentration in the raw material of 42614 ppm. Since it was expected that available chlorine would remain in the reaction mother liquor after the completion of the first reaction, the reaction time including blowing air was set to 2 hours.

It was confirmed as a result thereof that available chlorine remained at 13333 ppm in the reaction mother liquor after the completion of the first reaction, and chlorate ions increased 12.56%. Until now, increase/decrease in chlorate ions and degradation of available chlorine in the raw material were studied using a sulfuric acid concentration in the reaction mother liquor of 4.0%, 4.5%, 5.0%, 6.0%, and 6.37% with respect to the raw material in the same lot. Meanwhile, chlorate ions increased up to 4.0% or 4.5% with respect to a chloride concentration of about 42000 ppm in the raw material, but chlorate ions started to rapidly decrease from 5.0%.

In view of the above, the concentration of sulfuric acid that can be added is determined depending on the chloride concentration in the raw material, and if sulfuric acid is added for complete degradation of available chlorine in the raw material, chlorate ions decrease in some cases depending on the chloride concentration and the subsequent yield decreases. Thus, the sulfuric acid concentration during the first reaction is determined with respect to the chloride concentration in the raw material. If the chloride concentration is excessive, a large quantity of sulfuric acid cannot be added even if the available chlorine concentration is high.

[Table 32-1]

(1) Raw material

| Raw material | Available chlorine concentration | Cl | Cl⁻ | ClO₂⁻ | ClO₃⁻ |
|---|---|---|---|---|---|
| Deteriorated sodium hypochlorite (*) | 50626 | 42614 | 69081 | 93 | 32999 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 219.78g |
| 50% sulfuric acid | 19.78g | | — |
| 65% sulfuric acid | — | | 172.98g |
| 35% hydrogen peroxide | — | | 15g |
| Reaction mother liquor (total) | 219.78g | | 407.76g |
| Total added sulfuric acid concentration | 4.50% | | 30.0% |
| Reaction temperature | Ordinary temperature | | 40 to 50°C |
| Reaction time | 2h | | 2h |
| (Air time | 2h | | 2h |

(3) Recovery liquid

| | Recovery liquid A | Intermediate trap | Recovery liquid B |
|---|---|---|---|
| 10% calcium hydroxide | 200 g | - | - |
| 0.6N sodium hydroxide | - | - | 200 g |
| Hydrogen peroxide | - | - | 10 g |

*For low salt grade sodium hypochlorite, no intermediate trap

[Table 32-2]

[Measurement results] (unit: ppm)

| Reaction mother liquor | | Available chlorine concentration | Cl | Cl$^-$ | ClO$_2^-$ | ClO$_3^-$ | ClO$_3^-$ degradation rate |
|---|---|---|---|---|---|---|---|
| After first reaction | After 2 hr | 8113 | 29013 | 39179 | 0 | 36426 | -12.56 % |
| After second reaction | After 2 hr | 46 | 21392 | 30668 | 0 | 0 | 100.0 % |

| | | Available chlorine concentration | Cl | Cl$^-$ | ClO$_2^-$ | ClO$_3^-$ |
|---|---|---|---|---|---|---|
| Recovery liquid A | After 2 hr | 29392 | – | 32731 | 0 | 0 |
| Recovery liquid B | After 2 hr | 38133 | – | 777 | 20878 | 69 |

[Recovery rate of chlorite ion and available chlorine]

| | | | Theoretical value | Measurement value | Recovery rate |
|---|---|---|---|---|---|
| Recovery liquid A | After 2 hr | Available chlorine | 50626 | 29392 | 58.06 % |
| Recovery liquid B | After 2 hr | Available chlorine | 53365 | 38133 | 71.46 % |
| | | ClO$_2^-$ | 25402 | 20878 | 82.19 % |

(Reaction 29 of deteriorated sodium hypochlorite)

Up to this point, the sulfuric acid concentration of 4.5% with respect to the chloride concentration in the raw material of about 42000 ppm was considered as being within the appropriate range. Meanwhile, a reaction test was conducted using a raw material with a chloride concentration in the raw material of about 35000 ppm in another lot. Since the sulfuric acid concentration was 42000/35000 = 1.2, 4.5% × 1.2 = 5.4% was estimated as the appropriate value, but this condition is a safe numerical value taking into consideration variation in reactions where the chlorate ions increase to about 110% or greater. For this reason, the first reaction was performed with the concentration set to 6.0%.

It was confirmed as a result thereof that chlorate ions after the completion of the first reaction was about 104%, which is within the appropriate range where chlorate ions do not decrease, but is 110% or less. It was also confirmed that recovery liquid B that had undergone the second reaction was able to be recovered with high purity with low chloride ions.

[Table 33-1]

(1) Raw material

| Raw material | Available chlorine concentration | Cl | Cl$^-$ | ClO$_2$$^-$ | ClO$_3$$^-$ |
|---|---|---|---|---|---|
| Deteriorated sodium hypochlorite (*) | 67786 | 34728 | 63679 | 164 | 29228 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 227.27g |
| 50% sulfuric acid | 27.27g | | — |
| 65% sulfuric acid | — | | 168.70g |
| 35% hydrogen peroxide | — | | 15g |
| Reaction mother liquor (total) | 227.27g | | 410.97g |
| Total added sulfuric acid concentration | 6.00% | | 30.0% |
| Reaction temperature | Ordinary temperature | | 40 to 50℃ |
| Reaction time | 2h | | 2h |
| (Air time) | 2h | | 2h |

(3) Recovery liquid

| | Recovery liquid A | Intermediate trap | Recovery liquid B |
|---|---|---|---|
| 10% calcium hydroxide | 200 g | - | - |
| 0.6N sodium hydroxide | - | - | 200 g |
| Hydrogen peroxide | - | - | 10 g |

*For low salt grade sodium hypochlorite, no intermediate

trap

[Table 33-2]

[Measurement results] (unit: ppm)

| Reaction mother liquor | | Available chlorine concentration | Cl | Cl$^-$ | ClO$_2^-$ | ClO$_3^-$ | ClO$_3^-$ degradation rate |
|---|---|---|---|---|---|---|---|
| After first reaction | After 2 hr | 5024 | 18516 | 23083 | 0 | 30406 | -4.03 % |
| After second reaction | After 2 hr | 27 | 15401 | 19628 | 0 | 0 | 100.0 % |

| | | Available chlorine concentration | Cl | Cl$^-$ | ClO$_2^-$ | ClO$_3^-$ |
|---|---|---|---|---|---|---|
| Recovery liquid A | After 2 hr | 38517 | – | 44500 | 0 | 0 |
| Recovery liquid B | After 2 hr | 32308 | – | 374 | 16871 | 86 |

[Recovery rate of chlorite ion and available chlorine]

| | | | Theoretical value | Measurement value | Recovery rate |
|---|---|---|---|---|---|
| Recovery liquid A | After 2 hr | Available chlorine | 67686 | 38517 | 56.82 % |
| Recovery liquid B | After 2 hr | Available chlorine | 47267 | 3230 | 68.35 % |
| | | ClO$_2^-$ | 22499 | 16871 | 74.99 % |

(Reaction 30 of deteriorated hypo)

A formula for adding concentration of sulfuric acid in the reaction mother liquor during the first reaction uses no degradation or increase of chlorate ions in the raw material as the upper limit, and an increase in chlorate ions of 110% or greater as the conditions in view of the safety rate.

If the chloride concentration in the raw material is high at this time, the concentration of sulfuric acid that can be added decreases. Thus, the calculation formula is the following. Further, (2) X ≤ 4 so that available chlorine in the raw material does not remain excessively.

$$(1) \quad Y = -1.2676X + 9.84393$$

$$(2) \quad X \le 4$$

(Y: sulfuric acid concentration in the reaction mother liquor, X: chloride concentration in the raw material)
In this regard, a test was conducted by adding 5.4% of sulfuric acid during the first reaction as computed by the calculation formulation so that the sulfuric acid concentration would be 40.0% during the second reaction.

As a result thereof, the chlorate ion concentration after the completion of the first reaction increased to 118.24%, and the recovery rate of recovery liquid B after the second reaction was 93.7%. The chloride ions in recovery liquid B increased to 3827 ppm. It was confirmed that increasing the sulfuric acid concentration to enhance the recovery rate results in increase of chloride ions as byproduct. If the final product is a sodium hypochlorite solution, recovery liquid B with high concentration of the chloride may be acceptable recovery liquid. However, for high grade chlorinated lime, if the concentration of chloride is over 4,000ppm, the concentration rate decreases at the time of drying. As a result, high grade chlorinated lime with a high concentration cannot be manufactured.

[Table 34-1]

(1) Raw material

| Raw material | Available chlorine concentration | Cl | $Cl^-$ | $ClO_2^-$ | $ClO_3^-$ |
|---|---|---|---|---|---|
| Deteriorated sodium hypochlorite (*) | 69262 | 35273 | 64849 | 186 | 28951 |

*12% low salt grade sodium hypochlorite

(2) Reaction condition (first reaction, second reaction)

| | First | Use | Second |
|---|---|---|---|

|  | reaction | reaction mother liquor | reaction |
|---|---|---|---|
| Deteriorate hypo | 200g |  | 224.32g |
| 50% sulfuric acid | 24.22g |  | — |
| 65% sulfuric acid | — |  | 334.31g |
| 35% hydrogen peroxide | — |  | 15g |
| Reaction mother liquor (total) | 224.22g |  | 573.53g |
| Total added sulfuric acid concentration | 5.40% |  | 40.0% |
| Reaction temperature | Ordinary temperature |  | 40 to 50°C |
| Reaction time | 2h |  | 2h |
| (Air time) | 2h |  | 2h |

(3) Recovery liquid

|  | Recovery liquid A | Intermediate trap | Recovery liquid B |
|---|---|---|---|
| 10% calcium hydroxide | 200 g | - | - |
| 0.6N sodium hydroxide | - | - | 200 g |
| Hydrogen peroxide | - | - | 10 g |

*For low salt grade sodium hypochlorite, no intermediate trap

[Table 34-2]

[Measurement results] (unit: ppm)

| Reaction mother liquor |  | Available chlorine concentration | Cl | Cl⁻ | $ClO_2^-$ | $ClO_3^-$ | $ClO_3^-$ degradation rate |
|---|---|---|---|---|---|---|---|
| After first reaction | After 2 hr | 8190 | 22326 | 31092 | 0 | 34233 | -18.24 % |
| After second reaction | After 2 hr | 0 | 10737 | 15218 | 0 | 0 | 100.0 % |

| | | Available chlorine concentration | Cl | Cl$^-$ | ClO$_2^-$ | ClO$_3^-$ |
|---|---|---|---|---|---|---|
| Recovery liquid A | After 2 hr | 34581 | – | 40663 | 0 | 0 |
| Recovery liquid B | After 2 hr | 43868 | – | 3827 | 24169 | 50 |

[Recovery rate of chlorite ion and available chlorine]

| | | | Theoretical value | Measurement value | Recovery rate |
|---|---|---|---|---|---|
| Recovery liquid A | After 2 hr | Available chlorine | 69262 | 34581 | 49.93 % |
| Recovery liquid B (from raw material) | After 2 hr | Available chlorine | 46819 | 43868 | 93.70% |
| | | ClO$_2^-$ | 22286 | 24169 | 108.45 % |
| Recovery liquid B (from primary reaction) | After 2 hr | Available chlorine | 62065 | 43868 | 70.68 % |
| | | ClO$_2^-$ | 29543 | 24169 | 81.81 % |

(Reaction 31 of deteriorated hypo)

A reaction was performed using low salt grade sodium hypochlorite as a raw material, with the sulfuric acid concentration in the reaction mother liquor during the second reaction set to 45.0%. As a result, chloride ions in recovery liquid B started to increase, and the recovery rate decreased. This indicates that a secondary reaction producing chlorine gas upon degradation of chlorate ions in the reaction mother liquor started to progress. It was found in view of the above that chlorine gas is rapidly generated when the sulfuric acid concentration in the second reaction exceeds 45%, so that the concentration ratio decreases when manufacturing a solid product.

[Table 35-1]

(1) Raw material

| Raw material | Available chlorine concentration | Cl | Cl$^-$ | ClO$_2^-$ | ClO$_3^-$ |
|---|---|---|---|---|---|
| | | | | | |

| Deteriorated sodium hypochlorite (*) | 50310 | | 41858 | 72590 | 176 | 32780 |
|---|---|---|---|---|---|---|

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 219.78g |
| 50% sulfuric acid | 19.78g | | — |
| 65% sulfuric acid | — | | 478.81g |
| 35% hydrogen peroxide | — | | 15g |
| Reaction mother liquor (total) | 219.78g | | 713.59g |
| Total added sulfuric acid concentration | 4.50% | | 45.0% |
| Reaction temperature | Ordinary temperature | | 40 to 50°C |
| Reaction time | 2h | | 2h |
| (Air time) | 2h | | 2h |

(3) Recovery liquid

| | Recovery liquid A | Intermediate trap | Recovery liquid B |
|---|---|---|---|
| 10% calcium hydroxide | 200 g | – | – |
| 0.6N sodium hydroxide | – | – | 200 g |
| Hydrogen peroxide | – | – | 10 g |

*For low salt grade sodium hypochlorite, no intermediate trap

[Table 35-2]

[Measurement results] (unit: ppm)

| Reaction mother liquor | | Available chlorine concentration | Cl | Cl$^-$ | ClO$_2^-$ | ClO$_3^-$ | ClO$_3^-$ degradation rate |
|---|---|---|---|---|---|---|---|

| | | | | | | |
|---|---|---|---|---|---|---|
| After first reaction | After 2 hr | 6447 | 28370 | 39851 | 0 | 35871 | −9.43 % |
| After second reaction | After 2 hr | 0 | 9193 | 12354 | 0 | 0 | 100.0 % |

| | | Available chlorine concentration | $Cl$ | $Cl^-$ | $ClO_2^-$ | $ClO_3^-$ |
|---|---|---|---|---|---|---|
| Recovery liquid A | After 2 hr | 28606 | – | 32014 | 0 | 0 |
| Recovery liquid B | After 2 hr | 44382 | – | 11143 | 23653 | 95 |

[Recovery rate of chlorite ion and available chlorine]

| | | | Theoretical value | Measurement value | Recovery rate |
|---|---|---|---|---|---|
| Recovery liquid A | After 2 hr | Available chlorine | 50310 | 28606 | 56.86 % |
| Recovery liquid B (from raw material) | After 2 hr | Available chlorine | 53011 | 44382 | 83.72 % |
| | | $ClO_2^-$ | 25233 | 23653 | 93.74 % |
| Recovery liquid B (from primary reaction) | After 2 hr | Available chlorine | 63747 | 44382 | 69.62% |
| | | $ClO_2^-$ | 30344 | 23653 | 77.95 % |

(Reaction 32 of deteriorated hypo)

A reaction was performed using low salt grade sodium hypochlorite as a raw material, with the sulfuric acid concentration in the reaction mother liquor during the second reaction set to 40.0%.

When the present manufacturing method was practiced using low salt grade sodium hypochlorite, the chloride concentration in the raw material was already excessive for the reaction. Thus, even if the generation of chlorine gas is prevented with hydrogen peroxide water, chloride ions derived from chlorine gas would be generated in recovery liquid B. This phenomenon occurs when the sulfuric acid concentration is high, or when the chloride concentration in the raw material is high. When a solid product is manufactured by the present manufacturing method, the product cannot be concentrated to a high concentration unless the chloride ions in recovery liquid B is about 4000 ppm. Thus, the upper limit value would be 40.0% (however, this is not limited thereto, and can be up to 59.4% when a liquid product is manufactured because it is not necessary to take concentration ratio into consideration.)

[Table 36-1]

(1) Raw material

| Raw material | Available chlorine concentration | Cl | Cl$^-$ | ClO$_2^-$ | ClO$_3^-$ |
|---|---|---|---|---|---|
| Deteriorated sodium hypochlorite (*) | 49923 | 41977 | 63409 | 151 | 31096 |

*12% low salt grade sodium hypochlorite

(2) Reaction condition (first reaction, second reaction)

| | First reaction | Use reaction mother liquor | Second reaction |
|---|---|---|---|
| Deteriorated hypo | 200g | | 219.78g |
| 50% sulfuric acid | 19.78g | | — |
| 65% sulfuric acid | — | | 336.09g |
| 35% hydrogen peroxide | — | | 15g |
| Reaction mother liquor (total) | 219.78g | | 570.87g |
| Total added sulfuric acid concentration | 4.50% | | 40.0% |
| Reaction temperature | Ordinary temperature | | 40 to 50°C |
| Reaction time | 2h | | 2h |
| (Air time) | 2h | | 2h |

(3) Recovery liquid

| | Recovery liquid A | Intermediate trap | Recovery liquid B |
|---|---|---|---|
| 10% calcium hydroxide | 200 g | – | – |
| 0.6N sodium hydroxide | – | – | 200 g |
| Hydrogen peroxide | – | – | 10 g |

*For low salt grade sodium hypochlorite, no intermediate trap

[Table 36-2]

[Measurement results] (unit: ppm)

| Reaction mother liquor | | Available chlorine concentration | Cl | $Cl^-$ | $ClO_2^-$ | $ClO_3^-$ | $ClO_3^-$ degradation rate |
|---|---|---|---|---|---|---|---|
| After first reaction | After 2 hr | 6055 | 29049 | 40830 | 0 | 36331 | -16.83 % |
| After second reaction | After 2 hr | 0 | 9193 | 12354 | 0 | 0 | 100.0 % |

| | | Available chlorine concentration | Cl | $Cl^-$ | $ClO_2^-$ | $ClO_3^-$ |
|---|---|---|---|---|---|---|
| Recovery liquid A | After 2 hr | 29468 | – | 28953 | 0 | 0 |
| Recovery liquid B | After 2 hr | 45900 | – | 4095 | 25238 | 79 |

[Recovery rate of chlorite ion and available chlorine]

| | | | Theoretical value | Measurement value | Recovery rate |
|---|---|---|---|---|---|
| Recovery liquid A | After 2 hr | Available chlorine | 49923 | 29468 | 59.03% |
| Recovery liquid B (from raw material) | After 2 hr | Available chlorine | 50288 | 45900 | 91.27 % |
| | | $ClO_2^-$ | 23937 | 25238 | 105.43 % |
| Recovery liquid B (from primary reaction) | After 2 hr | Available chlorine | 64565 | 45900 | 71.09 % |
| | | $ClO_2^-$ | 30733 | 25238 | 82.12 % |

(Sulfuric acid concentration during reaction)

The raw material deteriorated sodium hypochlorite is categorized into low salt and general grades, which have different chloride ion and chloride concentrations. The suitable sulfuric acid concentration also varies depending on the concentration thereof.

The condition for a suitable sulfuric acid concentration during the first reaction is conversion of available chlorine into chlorine gas without degrading chlorate ions in the raw material. The sulfuric acid concentration in the reaction mother liquor is determined by measuring the chloride concentration in the raw material.

If a high concentration of chloride is contained in the raw material at this time, chlorate ions would be degraded, so that the amount of sulfuric acid added cannot be increased, resulting in residual available chlorine. Meanwhile, if sulfuric acid is excessively added to reduce available chlorine, chlorate ions would be degraded so that the subsequent yield of recovery liquid B would decrease. However, if too much available chlorine remains, chloride ions would be generated in recovery liquid B in the next stage, so that the purity would decrease. In such a case, it is necessary to extend the air blow period to 2 hours and remove available chlorine from the reaction mother liquor.

In view of the test data up to this point, the computational formula at this time is the following. The formula takes into consideration the safety rate, with the rate of increase in the chlorate ion concentration after the completion of the first reaction at 110% or greater. Further, (2) X≤4 so that available chlorine in the raw material does not remain excessively.

$$(1) \quad Y = -1.2676X + 9.84393$$

$$(2) \quad X \leq 4$$

(Y: sulfuric acid concentration in the reaction mother liquor, X: chloride concentration in the raw material)

Next, the second reaction uses the reaction mother liquor after the completion of the first reaction as the raw material, but the raw material contains sufficient and often somewhat excessive chlorides for the reaction. If the sulfuric acid concentration in the reaction mother liquor is raised too much, only chlorine gas would be generated by the progression of a secondary reaction so that a large quantity of chloride ions would be generated in recovery liquid B. The same applies when the sulfuric acid concentration is too low.

Accordingly, the condition for a suitable sulfuric acid concentration during the second reaction is complete degradation of chlorate ions in the reaction mother liquor as well as control of secondary reactions so that chloride ions are not generated in recovery liquid B in large quantities.

However, a close relationship between the concentration of chlorate ions and chloride and the concentration of sulfuric acid in the reaction mother liquor after the completion of the first reaction is not so much seen. For example, chloride ions are contained in an excess of 3-fold to 7.5-fold after the completion of the primary reaction of low salt sodium hypochlorite and general grade sodium hypochlorite, but the recovery rate at a sulfuric acid concentration of 30% was excellent for both cases.

In this regard, the sulfuric acid concentration in the reaction mother liquor of the second reaction is the following in view of the data up to this point.

[Table 37-1]

|  | First reaction | Second reaction | |
|---|---|---|---|
|  |  | Solid product | Liquid product |
| Low salt grade sodium hypochlorite | 4.00 to 6.37% | 30.00 to 40.00% | 30.00 to 59.04% |
| General grade sodium hypochlorite | 4.00 to 4.50% | 25.00 to 30.00% |  |
| *For general grade sodium hypochlorite, a chlorine gas removing step using an intermediate trap is essential. | | | |

(List of reaction condition data)

[Table 37-2]

| Test No. |  | 1 | 2 | 3 | 4 | 5 | 6 | 7 | 8 | 9 | 10 | 11 |
|---|---|---|---|---|---|---|---|---|---|---|---|---|
| Grade |  | Low salt | Low salt | Low salt | Low salt | Low salt | Low salt | Low salt | Low salt | Low salt | Low salt | Low salt |
| Raw material — Available chlorine |  | 50989 | 50626 | 50017 | 50017 | 67786 | 50511 | 49897 | 69262 | 69262 | 50310 | 49923 |
| Raw material — Cl⁻ |  | 71421 | 69081 | 68782 | 68782 | 63679 | 65305 | 62483 | 64849 | 64849 | 72590 | 41977 |
| Raw material — Cl (Mohr's method) |  | 41983 | 42614 | 41416 | 41416 | 34728 | 42186 | 43429 | 35273 | 35273 | 41858 | 63409 |

| | | | | | | | | | | | | | |
|---|---|---|---|---|---|---|---|---|---|---|---|---|---|
| | ClO$_2$⁻ | | | 136 | 93 | 173 | 173 | 164 | 193 | 39 | 186 | 186 | 176 | 151 |
| | ClO$_3$⁻ | | | 34262 | 32999 | 33191 | 33191 | 29228 | 33644 | 32922 | 28951 | 28951 | 32780 | 31096 |
| Reaction | H$_2$SO$_4$ | First | | 4.00% | 4.50% | 5.00% | 6.00% | 6.00% | 6.37% | 6.37% | 5.40% | 6.00% | 4.50% | 4.50% |
| | | Second | | 30.00% | 30.00% | 30.00% | 30.00% | 30.00% | 58.00% | 30.00% | 40.00% | 40.00% | 45.00% | 40.00% |
| Reaction mother liquid after first reaction | Available chlorine | | | 14397 | 8113 | 13281 | 156 | 5024 | 598 | 267 | 8190 | 9695 | 6447 | 6055 |
| | Cl⁻ | | | 49041 | 39179 | 35178 | 23358 | 23083 | 17683 | 21460 | 31092 | 23358 | 39851 | 40830 |
| | Cl(Mohr's method) | | | 33344 | 29013 | 25145 | 18411 | 18516 | 15523 | 18225 | 22326 | 18523 | 28370 | 29049 |
| | ClO$_2$⁻ | | | 0 | 0 | 0 | 0 | 0 | 0 | 0 | 0 | 0 | 0 | 0 |
| | ClO$_3$⁻ | | | 38998 | 36426 | 32740 | 31996 | 30406 | 29507 | 31227 | 34233 | 29723 | 35871 | 36331 |
| | Increase/decrease in chloric acid | | | 113.82% | 110.39% | 98.64% | 96.40% | 104.03% | 87.70% | 94.85% | 118.24% | 102.67% | 109.43% | 116.83% |
| | Loss of available chlorine | | | 28.24% | 16.03% | 26.55% | 0.31% | 7.41% | 1.18% | 0.54% | 11.82% | 14.00% | 12.81% | 12.13% |
| Recovery liquid A | Available chlorine | | | 21401 | 29392 | 27397 | 39835 | 38517 | 42570 | 39421 | 34581 | 37973 | 28606 | 29468 |
| | Cl⁻ | | | 22295 | 32731 | 31279 | 47737 | 44500 | 50386 | 46547 | 40663 | 41444 | 32014 | 28953 |
| | ClO$_2$⁻ | | | 0 | 0 | 0 | 0 | 0 | 0 | 63 | 0 | 0 | 0 | 0 |
| | ClO$_3$⁻ | | | 0 | 0 | 0 | 0 | 0 | 0 | 0 | 0 | 0 | 0 | 0 |
| | Recovery rate | | | 41.97% | 58.06% | 54.78% | 79.64% | 56.82% | 84.28% | 79.99% | 49.93% | 54.83% | 56.86% | 59.03% |
| Recovery liquid B | Available chlorine | | | 42758 | 38133 | 37758 | 35482 | 32308 | 30209 | 36676 | 43868 | 39619 | 44382 | 45900 |
| | Cl⁻ | | | 2509 | 777 | 1659 | 149 | 374 | 20646 | 107 | 3827 | 2310 | 11143 | 4095 |
| | ClO$_2$⁻ | | | 23131 | 20878 | 19517 | 19713 | 16871 | 14722 | 18079 | 24169 | 21717 | 23653 | 25238 |
| | ClO$_3$⁻ | | | 0 | 69 | 16 | 55 | 86 | 80 | 29 | 50 | 36 | 95 | 79 |
| | Recovery rate(raw material) | | | 77.17% | 71.46% | 70.35% | 66.10% | 68.35% | 59.05% | 68.89% | 93.70% | 84.62% | 83.72% | 91.27% |
| | Recovery rate(first reaction) | | | 62.37% | 58.91% | 64.18% | 60.35% | 57.82% | 58.75% | 63.37% | 70.68% | 72.53% | 69.62% | 71.09% |

Unit: ppm

(Mixed solution of recovery liquid A and recovery liquid B (sodium hypochlorite specification))

Recovery liquid A and recovery liquid B were obtained by re-reacting deteriorated sodium hypochlorite as the raw material while preventing regeneration of chlorate ions. To comply with the specification and standards of sodium hypochlorite for a food additive by mixing the solutions, it is necessary to have an available chlorine concentration of

**EP 3 777 537 A1**

4% or greater and to meet other identification tests.

In this regard, the available chlorine ratio upon mixing recovery liquid A and recovery liquid B was studied.

It was found as a result thereof that the mixture is compliant with the specification, with the available chlorine concentration of recovery liquid B of 0.6 as the upper limit, given that the available chlorine concentration of recovery liquid A was 1. At 0.7, the mixture did not meet the testing item of entry (3) hypochlorous acid in the identification test (1). In light of the above, it is desirable to combine the solutions at an available chlorine concentration ratio of 0.43 to 0.6 when constructing the mixture from the viewpoint of the objective of the invention. Further, the disinfectant manufactured at this available chlorine concentration ratio was also compliant with the specification and standards for sodium hypochlorite by an analysis conducted by an external organization.

[Table 38]

|  | Sample 1 | Sample 2 | Sample 3 |
|---|---|---|---|
| Recovery liquid A; Available chlorine | 4200 | 41875 | 39412 |
| Recovery liquid B; Available chlorine | 18000 | 25125 | 27588 |
| Total available chlorine | 60000 | 67000 | 67000 |
| Available chlorine concentration ratio | 0.429 | 0.6 | 0.7 |

<Test result>

Japan's Specifications and Standards for Food Additives, 8th Edition, D Component specification/storage standard, each article, "sodium hypochlorite"

|  |  | Sample 1 | Sample 2 | Sample 3 |  |
|---|---|---|---|---|---|
| Content |  | Compliant (6.11%) | Compliant (6.74%) | Compliant (6.74%) | 4% or greater |
| Attribute |  | Compliant | Compliant | Compliant | Light yellow liquid with chlorine odor |
| Identification test (1) | Sodium salt (1) | Compliant | Compliant | Compliant | Inflammatory reaction while exhibiting yellow color |
|  | Hypochlorous acid (1) | Compliant | Compliant | Compliant | Bubbling from generation of gas |
|  | Hypochlorous acid (2) | Compliant | Compliant | Compliant | Alkaline, and iodine is released |
|  | Hypochlorous acid (3) | Compliant | Compliant | Noncompliant | Potassium permanganate does not lose color |
| Identification test (2) |  | Compliant | Compliant | Compliant | A maximum absorbance section at wavelength of 291 to 294 |
| Identification test (3) |  | Compliant | Compliant | Compliant | Red litmus paper changes color to blue and then loses color |

The available chlorine concentration and free residual chlorine concentration of the final liquid product when combined at the following ratios were measured in the same manner.

[Table 39]

| Available chlorine ratio (A:B) | 1:0.43 | 1:0.6 |
|---|---|---|
| Available chlorine concentration derived from recovery liquid A Recovery liquid | 41,958 | 37,500 |
| Available chlorine concentration derived from recovery liquid B Recovery liquid | 18.042 | 22,500 |

(continued)

| | | |
|---|---|---|
| Total available chlorine | 60,000 | 60,000 |
| Available chlorine concentration | 60,114 | 60,814 |
| Free residual chlorine concentration | 59,380 | 58,157 |

The free residual chlorine concentration is measured with a DPD reagent to be 58,157 to 59,380 ppm. Since a value that is approximately the value of the available chlorine concentration is obtained, it is understood that a reaction is also exhibited for those other than hypochlorous acid.

Due to the difficulty to find from the free residual chlorine concentration when it is a liquid product, the concentration of chlorite ion derived from the recovery liquid B recovery liquid was found by ion chromatography and converted into an available chlorine concentration, which was then subtracted from the available chlorine concentration of the whole. Furthermore, the remaining available chlorine concentration was determined as the recovery liquid A derived available chlorine concentration, which was multiplied by a coefficient to find the hypochlorite ion concentration for the final ion ratio.

[Table 40]

| | | |
|---|---|---|
| Available chlorine concentration ratio A:B | 1:0.43 | 1:0.6 |
| Recovery liquid A derived available chlorine concentration | 41958 | 37500 |
| Recovery liquid B derived available chlorine concentration | 18042 | 22500 |
| Total available chlorine | 60000 | 60000 |
| | | |
| Available chlorine concentration (A + B) | 60523 | 60788 |
| $ClO_2^-$ (IC) | 7609 | 9084 |
| Recovery liquid B derived available chlorine concentration B* | 15985 | 19084 |
| Recovery liquid A derived available chlorine concentration* | 44538 | 41704 |
| $ClO^-$ | 32334 | 30277 |
| | | |
| Available chlorine concentration ratio of final liquid | 1:0.36 | 1:0.46 |
| Ion ratio $ClO^-:ClO_2^-$ | 1:0.24 | 1:0.30 |
| * was calculated by the following conversion formula (Conversion formula) <br> Available chlorine concentration $\times$ 0.476 = chlorite ion concentration ($ClO_2^-$) <br> Available chlorine concentration $\times$ 0.726 = hypochlorite ion concentration ($ClO^-$) | | |

The conversion formulas were obtained by using a known concentration of chlorite ion or hypochlorite ion concentration and deriving the relationship with the available chlorine concentration.

(Storability of mixed solution)

The storability of a disinfectant (specifications and standards compliant product for sodium hypochlorite as food additive) manufactured based on the present manufacturing method was studied.

It was found as a result thereof that a disinfectant with an initial available chlorine concentration of about 6.94% was manufactured, whose available chlorine concentration at D + 30 decreases to about 5% (about 72%) at a refrigeration temperature (6°C) and the available chlorine concentration at D + 3 decreases to about 3.8% (about 54%) at 40°C. It was found that a disinfectant (liquid) containing both hypochlorite ions and chlorite ions has very poor storability, so that it is difficult to sell the disinfectant at ordinary temperatures and is preferably sold refrigerated.

[Table 41]

| |
|---|
| <6°C> (unit: ppm) |

(continued)

| | PET tin container | | polyester container | |
|---|---|---|---|---|
| | Available chlorine | Outer appearance | Available chlorine | Outer appearance |
| At start | 69412 | No change | 69412 | No change |
| D+1 | 68946 | No change | 68908 | No change |
| D+3 | 66213 | No change | 66204 | No change |
| D+7 | 62973 | No change | 62748 | No change |
| D+10 | 60988 | No change | 60319 | No change |
| D+20 | 54454 | No change | 53875 | No change |
| D+30 | 49986 | No change | 50210 | No change |
| D+40 | 47603 | No change | 47205 | No change |
| D+50 | 45791 | No change | 44875 | No change |
| <40°C> (unit: ppm) | | | | |
| | PET tin container | | polyester container | |
| | Available chlorine | Outer appearance | Available chlorine | Outer appearance |
| At start | 69412 | No change | 69412 | No change |
| D+1 | 44215 | No change | 44508 | No change |
| D+3 | 37883 | Gas generation | 37873 | Gas generation |
| D+7 | 34468 | Gas generation | 34181 | Gas generation |
| D+10 | 32588 | Gas generation | 32543 | Gas generation |
| D+20 | 26587 | Gas generation | 26505 | Gas generation |
| D+30 | 22131 | Gas generation | 22724 | Gas generation |
| D+40 | 20301 | Gas generation | 19845 | Gas generation |
| D+50 | 17859 | Gas generation | 17540 | Gas generation |

(Examination of recovery liquid composition during first reaction)

Improvement in the storability and available chlorine concentration was studied by using calcium hydroxide for the recovery liquid in the first reaction and drying and solidifying the solution. It was found as a result thereof that a significant change in the recovery rate is not observed between recovery at 1 part of 20% calcium hydroxide to 1 part of deteriorated sodium hypochlorite and recovery at 1 part of 20% calcium hydroxide to 2 parts of deteriorated sodium hypochlorite, so that concentrated recovery is possible.

The recovery rate was the highest when recovered at 1 part of 10% calcium hydroxide to 1 part of deteriorated sodium hypochlorite. It is understood that this is due to high fluidity of calcium hydroxide slurry. It was found in view of the above that it is preferable to concentrate available chlorine in a subsequent drying step than concentration at the available chlorine stage.

[Table 42]

|  |  | (1) | (2) | (3) | (4) | (5) |
|---|---|---|---|---|---|---|
| Reaction mother liquor | Deteriorated hypo* | 200 g | 200 g | 400 g | 400 g | 100 g |
|  | 30% sulfuric acid | 35 g | 35 g | 70 g | 70 g | 17.5 g |
| Temperature |  | Ordinary temperature | Ordinary temperature | Ordinary temperature | Ordinary temperature | Ordinary temperature |
| Reaction time |  | 2h | 2h | 2h | 3h | 2h |
| Air |  | 2h | 2h | 2h | 3h | 2h |

*Low salt sodium hypochlorite

|  | Recovery liquid A1 | Recovery liquid A2 | Recovery liquid A3 | Recovery liquid A4 | Recovery liquid A5 |
|---|---|---|---|---|---|
| 20% calcium hydroxide | 200 g | 200 g | 200 g | 200 g | – |
| 10% calcium hydroxide | – | – | – | – | 200 g |

[Measurement results] (unit: ppm)

|  | Available chlorine concentration | $ClO_3^-$ | Available chlorine recovery rate |
|---|---|---|---|
| Recovery liquid A1 | 33118 | 21 | 57.68 % |
| Recovery liquid A2 | 32254 | 97 | 57.34 % |
| Recovery liquid A3 | 61077 | 335 | 61.18 % |
| Recovery liquid A4 | 58788 | 2529 | 60.60 % |
| Recovery liquid A5 | 17070 | 570 | 66.43 % |

(Mixture solution of recovery liquid A and recovery liquid B (high grade chlorinated lime))

Deteriorated sodium hypochlorite was re-reacted as the raw material, and recovery liquid A and recovery liquid B were obtained to prevent generation of chlorate ions. Meanwhile, it is necessary that the mixture thereof complies with the specification and standard of high grade chlorinated lime for a food additive. The mixture needs an available chlorine concentration of 60% or greater, and to meet the identification tests.

For the achievement thereof, the available chlorine concentration ratio is adjusted so that the available chlorine concentration of recovery liquid B is within the range of 9.6 to 33.95 given that the available chlorine concentration of recovery liquid A is 1, and the drying step is performed. In view of the characteristic of this product, the primary blending ratio is recovery liquid B of 9.6 to recovery liquid A of 1 in order to avoid the ratio of recovery liquid B from becoming too high.

It was also found that the yield deteriorates depending on the drying step or the order of addition. Since free residual chlorine in particular is readily degraded, a granulation nucleus is formed in advance with only recovery liquid A, and then recovery liquid B is concentrated to a certain degree and slurried. When each is mixed and dried in this state, free residual chlorine tend not to degrade, so that a dry solid with very few changes in composition before and after drying can be obtained.

A dry solid manufactured by the above method was compliant with the specification standard for high grade chlorinated lime according to the analysis of an external organization.

Since high grade chlorinated lime manufactured by this manufacturing method has a special composition compared to high grade chlorinated lime that is commercially available and distributed, it is understood to be desirable to sell such high grade chlorinated lime as a bactericidal agent (food additive formulation) or a disinfectant rather than selling high grade chlorinated lime as an independent product.

(Standard composition ratio of recovery liquid A and recovery liquid B)

If recovery liquid A and recovery liquid B are mixed and dried, the available chlorine ratio cannot be measured in the final product due to different concentration ratio upon drying of each solution. For this reason, samples were prepared after measuring the available chlorine concentration of recovery liquid A and recovery liquid B, which were independently dried and used, as a preliminary test to find the lower limit of the available chlorine ratio of recovery liquid A and recovery liquid B.

[Table 43]

(Maximum drying of recovery liquid A or recovery liquid B alone)

| | Recovery liquid A | Recovery liquid B |
|---|---|---|
| Available chlorine | 228,600 | 994,851 |
| Cl- | 157,380 | 96090 |
| ClO2- | 0 | 464,890 |

| | Sample 1 | Sample 2 | Sample 2 |
|---|---|---|---|
| Recovery liquid A derived available chlorine | 66000 | 69400 | 66380 |
| Recovery liquid B derived available chlorine | 70400 | 700600 | 703620 |
| Total available chlorine | 770000 | 770000 | 770000 |
| Available chlorine concentration ratio | 9.6 | 10.1 | 10.6 |

(For only the data presented above, recovery liquid A and recovery liquid B were each dried to the maximum, and the powders were mixed with each other and a small amount of water was added, and the mixture was accurately weighted to obtain the data to investigate the constitution model.)

[Table 44]

| <Test result> Japan's Specifications and Standards for Food Additives, 8th Edition, D Component specification/storage standard, each article, "high grade chlorinated lime" | | | | |
|---|---|---|---|---|
| | Sample 1 | Sample 2 | Sample 3 | |
| Content | Compliant (77.0%) | Compliant (77.0%) | Compliant (77.0%) | 60% or greater |
| Attribute | Compliant | Compliant | Compliant | Light yellow liquid with chlorine order |
| High grade chlorinated lime (1) | Compliant | Compliant | Compliant | Litmus test paper changes color to blue and then loses color |
| High grade chlorinated lime (2) | Compliant | Compliant | Compliant | Exhibits a reaction of calcium salt |

(Drying step)

The drying conditions are an internal environmental temperature of 50 to 60°C and internal humidity of 10% or less. Warm air is sent in at a volume of 1.9 m$^3$/s. It is not necessary to apply the warm air directly on the liquid in the drying chamber at this time. The warm air intended to maintain internal temperature and humidity.

The yield and the composition after drying are more stabilized if a granulation nucleus of recovery liquid A and a slurry of recovery liquid B are each formed and then mixed. In particular, available chlorine is degraded and chloride ions and chlorate ions are concurrently generated if hypochlorite ions and chlorite ions coexist. In such a case, not only the available chlorine concentration of the final product decreases, but the purity also decreases due to a secondary component.

[Table 45]

```
┌─────────────────────────┐           ┌─────────────────────────┐
│   Recovery solution A   │           │    Recovery solution    │
└─────────────────────────┘           └─────────────────────────┘
            │                                     │
            ▼                                     ▼
┌─────────────────────────┐           ┌─────────────────────────┐
│   Preliminary drying    │           │  Preliminary concentration │
└─────────────────────────┘           └─────────────────────────┘
            │                                     │
            │  ┌─────────────────────────┐        │
            │  │   Granulation nucleus   │───────▶│
            │  └─────────────────────────┘        │
            └─────────────────────────────────────┤
                                                  ▼
                                        ┌─────────────────┐
                                        │     Drying      │
                                        └─────────────────┘
```

(Change in yield due to difference in drying step)

Compared to the No. 1 method, a decrease in free residual chlorine is much lower, and generation of secondary components, chloride ions and chlorate ions, is also lower for the No. 2 method.

[Table 46]

| Composition ratio | No. 1 | No. 2 |
|---|---|---|
| Recovery liquid A | 1 | 1 |
| Recovery liquid B | 20.01 | 20.00 |
| Available chlorine concentration (ppm) | 327166 | 327166 |
| Drying step | After preliminary drying recovery liquid B, recovery liquid A is added. After 30 minutes of drying with warm air, the mixture is dried by air (inside temperature is about 20°C to 30°C) . | Recovery liquid A is pre-dried, and a granulation nucleus is formed. Recovery liquid B is then slurried. Dried recovery liquid A is added to the recovery liquid B slurry, and dried with warm air for 20 to 30 minutes. |
| Drying time | Recovery liquid B: 30 minutes Recovery liquid A + recovery liquid B: 60 minutes Total of 90 | Recovery liquid A: 75 minutes Recovery liquid B: 75 minutes Recovery liquid A + recovery liquid |

(continued)

| Composition ratio | No. 1 | | No. 2 | |
|---|---|---|---|---|
| | | | minutes | B: 20 minutes Total of 170 minutes |
| Each concentration after drying (ppm) | Available chlorine | | 669,700 | 782,210 |
| | Free residual chlorine | | 807 | 55,916 |
| | Cl | | 129,626 | 107,503 |
| | $Cl^-$ | | 141,490 | 118,690 |
| | $ClO_2^-$ | | 290,940 | 305,230 |
| | $ClO_3^-$ | | 42,320 | 10,410 |
| | $Ca^{2+}$ | | 8.82 % | 9.62 % |
| Dry yield | Available chlorine | | 70.87 % | 78.17 % |
| | Free residual chlorine | | 1.79 % | 117.37 % |
| | Cl | | 128.45 % | 102.12 % |
| Concentration ratio | | | 22.6 | 22.9 |

(Change in yield due to difference in moisture during the drying step)

[Table 47]

| | | A1 | A2 | A3 | | B1 | B2 | B3 | | C1 |
|---|---|---|---|---|---|---|---|---|---|---|
| A solution : B solution | | 1 : 33.95 | 1 : 33.95 | 1 : 33.95 | | 1 : 21.57 | 1 : 20.01 | 1 : 20.00 | | 1 : 9.61 |
| Moisture after preliminary drying (whole) | | 30.80 % | 26.00 % | 20.80 % | | 26.40% | 20.90% | 18.00% | | 20.09 % |
| Total drying time | | 300min | 195min | 195min | | 315min | 180min | 170min | | 195min |
| Each concentration | Available chlorine | 881667 | 647265 | 824064 | | 632513 | 781019 | 782210 | | 616877 |
| | Free residual chlorine | 901 | 2145 | 20785 | | 2625 | 49314 | 55916 | | 58728 |
| | Cl | 122417 | 144862 | 101873 | | 132291 | 124480 | 107503 | | 136948 |
| | $Cl^-$ | 128372 | 157560 | 107320 | | 140420 | 122170 | 118690 | | 141812 |
| | $ClO_2^-$ | 400315 | 272150 | 349840 | | 280580 | 319280 | 305230 | | 235600 |
| | $ClO_3^-$ | 32855 | 29760 | 16840 | | 35670 | 9400 | 10410 | | 36800 |
| | $Ca^{2+}$ | 6.53% | 5.28% | 5.64% | | 8.94% | 9.57% | 9.62% | | 15.40 % |

The stability upon mixing and drying solution A and solution B was affected by the moisture content retained before drying and the available chlorine ratio upon mixing recovery liquid A and recovery liquid B.

The present manufacturing method attained improved operability or stability when dried in advance to a certain degree by forming a granulation nucleus with recovery liquid A and forming a slurry with recovery liquid B. As a rule of thumb, it is preferable to dry each recovery liquid in advance until the overall moisture content before drying is 20% or less.

If the moisture content is 26% or greater at this time, the available chlorine concentration decreases, chlorate ions increase, and purity and drying rate decrease due to a reaction between chlorite ions and free residual chlorine (hypochlorite ions) in recovery liquid A. If the moisture content exceeds 30%, free residual chlorine (hypochlorite ions) undergoes significant self-degradation so that free residual chlorine (hypochlorite ions) particularly decreases, and the purity and drying rate also decrease.

Furthermore, an available chlorine ratio upon mixing recovery liquid A with recovery liquid B of 1:20 produces the least secondary components and results in a high purity. Thus, the best result was attained with an available chlorine ratio set to 1:20 and moisture content of 20% or less when drying recovery liquid A and recovery liquid B are dried while keeping them stable.

(Calculated value for a solid product in the segment of 20% moisture content after preliminary drying)

[Table 48]

| | A1 | A2 | A3 | | B1 | B2 | B3 | | C1 |
|---|---|---|---|---|---|---|---|---|---|
| A solution : B solution | 1: 33.95 | 1: 33.95 | 1: 33.95 | | 1: 21.57 | 1: 20.01 | 1: 20.00 | | 1: 9.61 |
| Moisture after preliminary drying (whole) | 30.80 % | 26.00 % | 20.80 % | | 26.40 % | 20.90 % | 18.00 % | | 20.09 % |
| Total drying time | 300 min | 195 min | 195 min | | 315 min | 180 min | 170 min | | 195min |

| | | A1 | A2 | A3 | | B1 | B2 | B3 | | C1 |
|---|---|---|---|---|---|---|---|---|---|---|
| Each concentration | Available chlorine | 881667 | 647265 | 824064 | | 632513 | 781019 | 782210 | | 616877 |
| | Free residual chlorine | 901 | 2145 | 20785 | | 2625 | 49314 | 55916 | | 58728 |
| | Cl | 122417 | 144862 | 101873 | | 132291 | 124480 | 107503 | | 136948 |
| | $Cl^-$ | 128372 | 157560 | 107320 | | 140420 | 122170 | 118690 | | 141812 |
| | $ClO_2^-$ | 400315 | 272150 | 349840 | | 280580 | 319280 | 305230 | | 235600 |
| | $ClO_3^-$ | 32855 | 29760 | 16840 | | 35670 | 9400 | 10410 | | 36800 |
| | $Ca^{2+}$ | 6.53% | 5.28% | 5.64% | | 8.94% | 9.57% | 9.62% | | 15.40 % |

| | | | | |
|---|---|---|---|---|
| Available chlorine (A + B) | | 824064 | 781019 | 616877 |
| Free residual chlorine (A) | | 20785 | 49314 | 58728 |
| Available chlorine (B) | | 803279 | 731705 | 558149 |
| Available chlorine ratio | | 1: 38.65 | 1: 14.84 | 1: 9.5 |

| | | | | |
|---|---|---|---|---|
| $ClO^-$ (A)×0.726 | | 15090 | 35802 | 42637 |
| $ClO_2^-$ (B) | | 349840 | 319280 | 235600 |
| Ion ratio $ClO^-:ClO_2^-$ | | 1: 23.18 | 1: 8.92 | 1: 5.53 |

The post-drying available chlorine ratio and ion ratio were calculated from the measurement value for a solid product in the segment of 20% moisture content after preliminary drying. Hypochlorite ions:chorite ions include 1:5.53 to 23.18.

(Composition and result of drying the combination of recovery liquids A and B (1:9.61))

This is the result of measurement when 9.61 of recovery liquid B is mixed with 1 of recovery liquid A in terms of available chlorine ratio and dried. This available chlorine ratio would be excessive in the following reaction, generating chloride and chlorate ions.

$$ClO^- + ClO_2^- \rightarrow ClO_3^- + Cl^-$$

Thus, it is understood that at this available chlorine ratio or higher, available chlorine would be below 60%, which would fail to meet the specification of high grade chlorinated lime. Therefore, this available chlorine ratio corresponds to the lower limit.

[Table 49]

(unit: ppm)

|  | Available chlorine | $Cl^-$ | $ClO_2^-$ | $ClO_3^-$ |
|---|---|---|---|---|
| Recovery liquid A | 28024 | 30171 | 0 | 0 |
| Recovery liquid B | 44531 | 4962 | 22869 | 25 |

|  | Recovery liquid A |  | Recovery liquid B |
|---|---|---|---|
| Weight mixing % | 24.94 | : | 150.75 |
| Weight ratio | 1.00 | : | 6.05 |
| Available chlorine | 3977 ppm | : | 38211 ppm |
| Available chlorine concentration ratio | 1.00 | ; | 9.61 |

(After drying)

(unit: ppm)

|  | Available chlorine | Free chlorine | $Cl^-$ | $ClO_2^-$ | $ClO_3^-$ | Ca2+ |
|---|---|---|---|---|---|---|
| High grade chlorinated lime | 616877 | 58728 | 141812 | 235600 | 36800 | 15.40 % |

(Composition and result of drying the combination of recovery liquids A and B (1:33.95))

This is the result of measurement when 33.95 of recovery liquid B is mixed with 1 of recovery liquid A in terms of available chlorine ratio and dried. It is expected that if the available chlorine ratio of recovery liquid B becomes too high, the product would not be compliant with the specification for calcium and free residual chlorine in some cases. In the result of this ratio, the calcium ion concentration after drying is 5.64%, which is at the lower limit of conditions where the amount of calcium in high grade chlorinated lime is compliant. Therefore, this available chlorine ratio substantially corresponds to the upper limit.

[Table 50]

(unit: ppm)

|  | Available chlorine | $Cl^-$ | $ClO_2^-$ | $ClO_3^-$ |
|---|---|---|---|---|
| Recovery liquid A | 28938 | 29698 | 0 | 0 |
| Recovery liquid B | 43611 | 4270 | 24033 | 15 |

|  | Recovery liquid A |  | Recovery liquid B |
|---|---|---|---|
| Weight mixing % | 6.40 | : | 144.23 |
| Weight ratio | 1.00 | : | 22.53 |
| Available chlorine | 1230 ppm | : | 41757 ppm |
| Available chlorine concentration ratio | 1.00 | ; | 33.95 |

(After drying)

(unit: ppm)

|  | Available chlorine | Free chlorine | $Cl^-$ | $ClO_2^-$ | $ClO_3^-$ | Ca2+ |
|---|---|---|---|---|---|---|
| High grade chlorinated lime | 824064 | 20785 | 107320 | 349840 | 16840 | 5.64% |

(Composition and result of drying the combination of recovery liquids A and B (List))

For segment (1), if free residual chlorine is high, hypochlorite ions and chlorite ions would react, so that available chlorine decreases, and chloride ions and chlorate ions are generated. In such a case, the dry concentration ratio decreases and the available chlorine concentration would be the lowest due to not only degradation of available chlorine, but also generation of secondary components that do not contribute to available chlorine.

Meanwhile in segments (4), (5), and (6), the ratio of generating byproducts such as chloride ions and chlorate ions is high despite reduced initial amount of addition of free residual chlorine. Although the cause thereof is unknown, it was found that when the ratio of hypochlorite ions is high in the available chlorine ratio of hypochlorite ions to chlorite ions, the specification was not met due to degradation of available chlorine, but when the ratio is low, the ratio of change into secondary components such as chloride ions and chlorate ions increases and the purity decreases to obstruct dry concentration.

[Table 51]

|  | Segment (1) | Segment (2) | Segment (3) | Segment (4) | Segment (5)* | Segment (6)* |
|---|---|---|---|---|---|---|
| A:B | 1:9.61 | 1:20.01 | 1:25.01 | 1:33.95 | 1:40.63 | 1:45.04 |

(continued)

|  | Segment (1) | Segment (2) | Segment (3) | Segment (4) | Segment (5)* | Segment (6)* |
|---|---|---|---|---|---|---|
| Available chlorine | 616,877 | 781,019 | 802,432 | 824,064 | 817,143 | 797,500 |
| Free residual chlorine | 58,728 | 49,314 | 36,272 | 20.785 | 8,599 | 3,054 |
| Cl | 136,948 | 124,480 | 113,405 | 101,873 | 99,998 | 107.605 |
| $Cl^-$ | 141,812 | 122,170 | 124,880 | 107,320 | 100.710 | 114,820 |
| $ClO_2^-$ | 235,600 | 319,280 | 339,450 | 349,840 | 353,190 | 360.520 |
| $ClO_3^-$ | 36,800 | 9,400 | 17,740 | 16,840 | 20,850 | 23,760 |
| $SO_4^{2-}$ | 2880 | 1370 | 1800 | 1140 | 660 | 320 |
| $Ca^{2+}$ | 15.40 % | 9.57 % | 7.66 % | 5.64 % | 7.48 % | 4.54% |

*Segment (5) is a reference segment with further addition of calcium hydroxide because the calcium concentration is below 5%. Segment (6) is a reference segment used because the calcium concentration does not meet the specification, but loss of color is observed in some cases even with free residual chlorine at 1000 ppm.

Since this manufacturing method obtains a product from a reaction of chlorine gas, about 1140 ppm or more sulfate ions are included. The sulfate ions are entrainment of sulfuric acid contained in a reaction tank. In addition, there is excess of calcium hydroxide in the recovery liquid, thus is primarily in a form of sodium sulfate.

[Table 52]

|  | Tosoh Corporation | Nippon Soda Co., Ltd. |
|---|---|---|
| Available chlorine concentration | 75.36 % | 69.33 % |
| $SO_4^{2-}$ | 19460 | 8110 |

(When solid product is dissolved)

Based on the following mixing proportion table, deionized water was added so that the available chlorine concentration after dilution would be 1%, 6%, and 12%, and the solution was stirred for 15 minutes with a stirrer so that there would not be any undissolved residue. The solution was then incubated for 1 hour in a 10°C incubator (obtain a clear supernatant), and the supernatant was recovered so as not to aspirate in precipitates. The free residual chlorine concentration, available chlorine concentration, and various ion concentrations (ion chromatography) of the supernatant were measured.

[Table 53]

|  | Segment A3 | | | Segment Cl | | |
|---|---|---|---|---|---|---|
| Available chlorine concentration | 1% segment | 6% segment | 12% segment | 1% segment | 6% segment | 12% segment |
| High grade chlorinated lime | 1.22 | 7.32 | 14.64 | 1.63 | 9.73 | 19.46 |
| Deionized water | 98.78 | 92.68 | 85.36 | 98.37 | 90.27 | 80.54 |

When a solid product was liquefied by dilution with ion exchanged water, free residual chlorine decreased somewhat due to promotion of degradation, but the available chlorine ratio and ion ratios were as specified in the following table.

[Table 54-1]

| | | A3 | C1 |
|---|---|---|---|
| A solution : B solution | | 1 : 33.95 | 1 : 9.61 |
| Moisture after preliminary drying (whole) | | 20.80% | 20.09% |
| Total drying time | | 195min | 195min |
| Each concentration | Available chlorine | 824064 | 616877 |
| | Free residual chlorine | 20785 | 58728 |
| | Cl | 101873 | 136948 |
| | Cl$^-$ | 107320 | 141812 |
| | ClO$_2^-$ | 349840 | 235600 |
| | ClO$_3^-$ | 16840 | 36800 |
| | Ca$^{2+}$ | 5.64% | 15.40% |

| | A3 | C1 |
|---|---|---|
| Available chlorine (A + B) | 824064 | 616877 |
| Free residual chlorine (A) | 20785 | 58728 |
| Available chlorine (B) | 803279 | 558149 |
| Available chlorine ratio | 1 : 38.65 | 1 : 9.50 |

| | | A3 | C1 |
|---|---|---|---|
| ClO$^-$ (A)×0.726 | | 15090 | 42637 |
| ClO$_2^-$ (B) IC | | 349840 | 235600 |
| Ion ratio ClO$^-$ :ClO$_2^-$ | | 1 : 23.18 | 1 : 5.53 |

<Concentration and ratio of supernatant (liquefied) after dissolution>

Diluted to available chlorine concentration of 1%, 6%, 12%

|  |  | A3-1% | A3-6% | A3-12% |  | C1-1% | C1-6% | C1-12% |
|---|---|---|---|---|---|---|---|---|
| Free residual chlorine | Actual measurement value | 187.07 | 1296.01 | 2736.70 |  | 836.70 | 5624.20 | 11378.81 |
|  | Theoretical value | 253.58 | 1521.47 | 3042.93 |  | 951.39 | 5714.24 | 11422.60 |
|  | Yield | 73.77% | 85.18% | 89.94% |  | 87.94% | 98.42% | 99.62% |
| Available chlorine | Actual measurement value | 9990.29 | 59685.03 | 120297.77 |  | 9980.20 | 59788.58 | 122251.63 |
|  | Theoretical value | 10053.59 | 60321.52 | 120643.04 |  | 10055.10 | 60022.14 | 120044.27 |
|  | Yield | 99.37% | 98.94% | 99.71% |  | 99.26% | 99.61% | 101.84% |

| Available chlorine (A + B) |  | 9990.291 | 59685.03 | 120297.77 |  | 9980.20 | 59788.58 | 122251.63 |
|---|---|---|---|---|---|---|---|---|
| Free residual chlorine (A) |  | 187.07 | 1296.01 | 2736.70 |  | 836.70 | 5624.20 | 11378.81 |
| Available chlorine (B) |  | 9803.23 | 58389.02 | 117561.07 |  | 9143.50 | 54164.39 | 110872.82 |
| Available chlorine ratio |  | 1 : 52.41 | 1 : 45.05 | 1 : 42.96 |  | 1 : 10.93 | 1 : 9.63 | 1 : 9.74 |

| ClO⁻ (A)×0.726 |  | 135.81 | 940.90 | 1986.84 |  | 607.44 | 4083.17 | 8261.01 |
|---|---|---|---|---|---|---|---|---|
| ClO₂⁻ (B)×0.476 |  | 4666.34 | 27793.17 | 55959.07 |  | 4352.30 | 25782.25 | 52775.46 |
| Ion ratio ClO⁻ : ClO₂⁻ |  | 1 : 34.36 | 1 : 29.54 | 1 : 28.16 |  | 1 : 7.16 | 1 : 6.31 | 1 : 6.39 |

(Composition when calcium was removed from solid product)

Based on the following mixing proportion table and the relational equation for removing Ca (*), deionized water was added so that the available chlorine concentration after dilution of recovery liquid in which Ca was removed would be 1%, 6%, and 12%, and the solution was stirred for 15 minutes with a stirrer so that there would not be any undissolved portion (20% sodium carbonate solution obtained from the relationship equation was added and the mixture was stirred for 1 minute) . The mixture was then incubated for 19 hours in a 10°C incubator (obtain a clear supernatant), and the supernatant was recovered so as not to aspirate in precipitates. The free residual chlorine concentration, available chlorine concentration, and various ion concentrations (ion chromatography) of the supernatant were measured.

*The relational equation (1) of calcium ion concentration (%) in high grade chlorinated lime <X1> and the amount of 20% sodium hydrogen carbonate solution added (g) <Y1> is Y1 = 14.042 X1 + 0.0185

Calcium ions were measured using an NN indicator and titrating with an EDTA solution.

1 ml of 0.05 mol/L EDTA solution = 3.705 mg $Ca(OH)_2$ × molecular weight of calcium ion (40.08)/molecular weight of calcium hydroxide (74.08)

= 1 ml of 0.05 mol/L EDTA solution = 2.005 mg $Ca^{2+}$

[Table 54-2]

| | Segment A | | | Segment C | | |
|---|---|---|---|---|---|---|
| Available chlorine concentration | 1% segment | 6% segment | 12% segment | 1% segment | 6% segment | 12% segment |
| High grade chlorinated lime | 1.26 | 7.96 | 16.97 | 1.71 | 12.37 | 32.98 |
| Deionized water | 98.74 | 92.04 | 83.03 | 98.29 | 87.63 | 67.02 |
| 20% sodium carbonate solution | 1.00 | 6.23 | 13.26 | 3.47 | 25.03 | 66.71 |
| Total | 101.00 | 106.23 | 113.26 | 103.47 | 125.03 | 166.71 |

When a solid product was diluted with deionized water and liquefied after removing calcium ion in accordance with the relational equation, suppression of degradation of free residual chlorine was observed due to increased alkali. The available chlorine ratio and ion ratios were as specified in the following table.

[Table 54-3]

| | | Segment A | Segment C |
|---|---|---|---|
| A solution:B solution | | 1 : 33.96 | 1 : 9.61 |
| Moisture after preliminary drying (whole) | | 20.00% | 20.30% |

| Total drying time | | 195 min | 195 min |
|---|---|---|---|
| Each concentration | Available chlorine | 801591 | 606811 |
| | Free residual chlorine | 19634 | 51718 |
| | Cl | 96487 | 135873 |
| | Cl− | 96700 | 136610 |
| | $ClO_2^-$ | 336190 | 240800 |
| | $ClO_3^-$ | 16600 | 24610 |
| | $Ca^{2+}$ | 5.56% | 14.40% |

| | 195 min | 195 min |
|---|---|---|
| Available chlorine (A + B) | 801591 | 606811 |
| Free residual chlorine (A) | 19634 | 51718 |
| Available chlorine (B) | 781957 | 555093 |
| Available chlorine ratio | 1:39.83 | 1:10.73 |

| | | 195 min | 195 min |
|---|---|---|---|
| $ClO^-$ (A)×0.726 | | 14254 | 37547 |
| $ClO_2^-$ (B) IC | | 336190 | 240800 |
| Ion ratio $ClO^-$:$ClO_2^-$ | | 1:23.59 | 1:6.41 |

＜Concentration and ratio after removing Ca＞

| | | A−1% | A−6% | A−12% | C−1% | C−6% | C−12% |
|---|---|---|---|---|---|---|---|
| Free residual chlorine | Actual measurement value | 236.18 | 1522.36 | 3313.01 | 967.89 | 6768.29 | 13455.28 |
| | Theoretical value | 244.94 | 1471.21 | 2941.81 | 854.72 | 5116.79 | 10231.30 |
| | Yield | 96.42% | 103.48% | 112.62% | 113.24% | 132.28% | 131.51% |
| Available chlorine | Actual measurement value | 9945.54 | 59282.104 | 118244.55 | 10364.08 | 62308.44 | 126476.25 |
| | Theoretical value | 10000.05 | 60064.62 | 120104.18 | 10028.48 | 60035.61 | 120044.55 |
| | Yield | 99.45% | 98.70% | 98.45% | 103.35% | 103.79% | 105.36% |
| $ClO_2^-$ | Actual measurement value | 4247.1 | 29212.5 | 62627 | 4372.0 | 30337.5 | 68656 |
| | Theoretical value | 4194.05 | 25191.31 | 50372.10 | 3979.59 | 23823.85 | 47637.12 |
| | Yield | 101.26% | 115.96% | 124.33% | 109.86% | 127.34% | 144.12% |
| $Cl^-$ | | 1200.9 | 8185.0 | 17524 | 2302.6 | 15526.0 | 35917 |
| $ClO_3^-$ | | 188.8 | 1230.5 | 2590 | 406.9 | 2780.5 | 7027 |
| pH | | 11.66 | 12.69 | 13.06 | 12.60 | 13.27 | 13.53 |

110

| | | 0.00 | 0.00 | 0.00 | 0.00 | 0.00 | 0.00 |
|---|---|---|---|---|---|---|---|
| $Ca_2^+$ | | 0.00 | 0.00 | 0.00 | 0.00 | 0.00 | 0.00 |
| **Available chlorine (A + B)** | | 9945.54 | 59282.10 | 118244.55 | 10364.08 | 62308.44 | 126476.25 |
| **Free residual chlorine (A)** | | 236.18 | 1522.36 | 3313.01 | 967.89 | 6768.29 | 13455.28 |
| **Available chlorine (B)** | | 9709.37 | 57759.75 | 114931.54 | 9396.19 | 55540.15 | 113020.96 |
| **Available chlorine ratio** | | 1:41.11 | 1:37.94 | 1:34.69 | 1:9.71 | 1:8.21 | 1:8.40 |
| $ClO^-$ (A)×0.726 | | 171.47 | 1105.23 | 2405.24 | 702.69 | 4913.78 | 9768.54 |
| $ClO_2^-$ (B) IC | | 4247.1 | 29212.5 | 62627 | 4372.0 | 30337.5 | 68656 |
| **Ion ratio** $ClO^-:ClO_2^-$ | | 1:24.77 | 1:26.43 | 1:26.04 | 1:6.22 | 1:6.17 | 1:7.03 |

| Total drying time | | | 195 min | 195 min |
|---|---|---|---|---|
| Each concentration | Available chlorine | | 801591 | 606811 |
| | Free residual chlorine | | 19634 | 51718 |
| | Cl | | 96487 | 135873 |
| | Cl- | | 96700 | 136610 |
| | $ClO_2^-$ | | 336190 | 240800 |
| | $ClO_3^-$ | | 16600 | 24610 |
| | $Ca^{2+}$ | | 5.56% | 14.40% |

| | | 195 min | 195 min |
|---|---|---|---|
| Available chlorine (A + B) | | 801591 | 606811 |
| Free residual chlorine (A) | | 19634 | 51718 |
| Available chlorine (B) | | 781957 | 555093 |
| Available chlorine ratio | | 1:39.83 | 1:10.73 |

| | | 195 min | 195 min |
|---|---|---|---|
| $ClO^-$ (A)×0.726 | | 14254 | 37547 |
| $ClO_2^-$ (B) IC | | 336190 | 240800 |
| Ion ratio $ClO^-$:$ClO_2^-$ | | 1:23.59 | 1:6.41 |

＜Concentration and ratio after removing Ca＞

| | | | A-1% | A-6% | A-12% | C-1% | C-6% | C-12% |
|---|---|---|---|---|---|---|---|---|
| Free residual chlorine | Actual measurement value | | 236.18 | 1522.36 | 3313.01 | 967.89 | 6768.29 | 13455.28 |
| | Theoretical value | | 244.94 | 1471.21 | 2941.81 | 854.72 | 5116.79 | 10231.30 |
| | Yield | | 96.42% | 103.48% | 112.62% | 113.24% | 132.28% | 131.51% |
| Available chlorine | Actual measurement value | | 9945.54 | 59282.104 | 118244.55 | 10364.08 | 62308.44 | 126476.25 |
| | Theoretical value | | 10000.05 | 60064.62 | 120104.18 | 10028.48 | 60035.61 | 120044.55 |
| | Yield | | 99.45% | 98.70% | 98.45% | 103.35% | 103.79% | 105.36% |
| $ClO_2^-$ | Actual measurement value | | 4247.1 | 29212.5 | 62627 | 4372.0 | 30337.5 | 68656 |
| | Theoretical value | | 4194.05 | 25191.31 | 50372.10 | 3979.59 | 23823.85 | 47637.12 |
| | Yield | | 101.268% | 115.968% | 124.33% | 109.868% | 127.34% | 144.12% |
| $Cl^-$ | | | 1200.9 | 8185.0 | 17524 | 2302.6 | 15526.0 | 35917 |
| $ClO_3^-$ | | | 188.8 | 1230.5 | 2590 | 406.9 | 2780.5 | 7027 |
| pH | | | 11.66 | 12.69 | 13.06 | 12.60 | 13.27 | 13.53 |

[Table 54-4]

| Quantification of calcium salt | | Sample weight (g) | Amount of 0.05 mol/L EDTA added (mL) | Factor of 0.05 mol/L EDTA | Dilution factor | Calcium hydroxide concentration (%) | $Ca^{2+}$ concentration (%) |
|---|---|---|---|---|---|---|---|
| A3 | 1% | 4.95 | 0.00 | 1.000 | 1.00 | 0.00 | 0.00 |
| | 6% | 4.62 | 0.00 | 1.000 | 1.00 | 0.00 | 0.00 |
| | 12% | 3.95 | 0.00 | 1.000 | 1.00 | 0.00 | 0.00 |
| C1 | 1% | 4.95 | 0.00 | 1.000 | 1.00 | 0.00 | 0.00 |
| | 6% | 5.35 | 0.00 | 1.000 | 1.00 | 0.00 | 0.00 |
| | 12% | 5.17 | 0.00 | 1.000 | 1.00 | 0.00 | 0.00 |

<Specifications and Standards for Food, Food Additives, Etc., No. 2 Additives, D Component specification/storage standard, each article, calcium hydroxide>

Quantification method

About 2 g of the product is accurately weighed. 30 ml of hydrochloric acid (1→4) is added and dissolved. Water is further added to accurately prepare a 250 mL solution as the sample solution, which is quantified by the first method among calcium salt quantification methods. 1 ml 0.05 mol/L EDTA solution = 3.705 mg $Ca(OH)_2$

<Specifications and Standards for Food, Food Additives, Etc., No. 2 Additives, B General testing method, 8. Calcium salt quantification method>

The calcium salt quantification method is a method of quantifying calcium salt content using ethylenediaminetetraacetic acid (EDTA). The method includes direct titration method using an EDTA solution (first method) and reverse titration method that adds excessive EDTA and then titrates with zinc acetate solution (second method).

Operation method

The operation method uses one of the following methods or those specified elsewhere.

First method

10 ml of sample solution specified elsewhere is accurately weighed. 50 ml of water is added and 10 ml of potassium hydroxide solution (1→10) is added. After incubating the mixture for about 1 minute, about 0.1 g of NN indicator is added, and titration is performed immediately with 0.05 mol/L EDTA solution. The end point is when the reddish purple color of the solution completely disappears and turns blue.

Second method

20 ml of sample solution specified elsewhere is accurate weighed, and 25 mL of 0.02 mol/L EDTA solution is accurately weighed and added. 50 ml of water and 5 ml of ammonium/ammonium chloride buffer (pH 10.7) are then added and the mixture is incubated for about 1 minute. 0.025 g of Eriochrome Black T/sodium chloride indicator is added, and an excessive amount of EDTA is immediately titrated with 0.02 mol/L zinc acetate solution. The end point is when the liquid turns from blue to bluish purple color. A blank test is performed separately.

1 ml of 0.05 mol/L EDTA solution = 3.705 mg Ca(OH)$_2$ × molecular weight of calcium ion (40.08)/molecular weight of calcium hydroxide (74.08)

= 1 ml of 0.05 mol/L EDTA solution = 2.005 mg Ca$^{2+}$

(Storage data)

For comparison, a storage test was conducted with commercially available high grade chlorinated lime ("Toyokuron-PTGIII") and high grade chlorinate lime produced by the present manufacturing method ("high grade chlorinated lime A") at 25°C and 40°C. "High grade chlorinated lime" official specification test was conducted as a storage test item. Bubbling due to a reaction of moisture and hydrogen peroxide water was studied (free residual chlorine) and swelling was measured.

As a result, available chlorine hardly decreased in both the 25°C and 40°C storage temperature zones. The product was compliant with the "high grade chlorinated lime" official specification test. In addition, bubbling due to hydrogen peroxide water was observed. Thus, it was confirmed that a change in composition is not observed after storage.

Furthermore, the storability was confirmed to be better compared to the commercially available "high grade chlorinated lime".

As for the sample, recovery liquid A was pre-dried to form a granulation nucleus, and pre-dried recovery liquid B was mixed therewith and dried.

[Table 55-1]

| Measurement res chlorinated lime sults as of the manufacture of "high grade A" | |
| --- | --- |
| | |
| Available chlorine concentration | 818, 640 |
| Cl$^-$ | 121,520 |
| ClO$_2^-$ | 369,460 |
| (unit: ppm) | |

(1) Toyokuron 25°C

| | "High grade chlorinated lime" official specification test | | | | | | |
| --- | --- | --- | --- | --- | --- | --- | --- |
| | Content | Attribute | Identification test (1) | Identification test (2) | Moisture | Hydrogen peroxide reaction | Swelling |
| At start | Compliant (69.51%) | Compliant | Compliant | Compliant | 18.46% | Yes | No |
| D+10 | Compliant (69.88%) | Compliant | Compliant | Compliant | 20.39% | Yes | No |
| D+20 | Compliant (69.73%) | Compliant | Compliant | Compliant | 19.40% | Yes | No |
| D+30 | Compliant (68.56%) | Compliant | Compliant | Compliant | 20.58% | Yes | No |
| D+40 | Compliant (68.51%) | Compliant | Compliant | Compliant | 20.32% | Yes | No |
| D+50 | Compliant (68.50%) | Compliant | Compliant | Compliant | 20.99% | Yes | No |
| D+60 | Compliant (67.70%) | Compliant | Compliant | Compliant | 19.94% | Yes | No |

(continued)

| | "High grade chlorinated lime" official specification test | | | | | | |
| | Content | Attribute | Identification test (1) | Identification test (2) | Moisture | Hydrogen peroxide reaction | Swelling |
|---|---|---|---|---|---|---|---|
| D+70 | Compliant (67.72%) | Compliant | Compliant | Compliant | 20.30% | Yes | No |
| D+80 | Compliant (67.31%) | Compliant | Compliant | Compliant | 20.73% | Yes | No |
| D+90 | Compliant (66.29%) | Compliant | Compliant | Compliant | 20.02% | Yes | No |
| D+100 | Compliant (66.66%) | Compliant | Compliant | Compliant | 20.37% | Yes | No |
| D+110 | Compliant (65.77%) | Compliant | Compliant | Compliant | 20.74% | Yes | No |
| D+120 | Compliant (65.95%) | Compliant | Compliant | Compliant | 22.00% | Yes | No |

[Table 55-2]

| High grade chlorinated lime A 25°C | | | | | | | |
| | "High grade chlorinated lime" official specification test | | | | | | |
| | Content | Attribute | Identification test (1) | Identification test (2) | Moisture | Hydrogen peroxide reaction | Swelling |
|---|---|---|---|---|---|---|---|
| At start | Compliant (81.51%) | Compliant | Compliant | Compliant | 7.12% | Yes | No |
| D+10 | Compliant (80.88%) | Compliant | Compliant | Compliant | 8.64% | Yes | No |
| D+20 | Compliant (80.93%) | Compliant | Compliant | Compliant | 8.27% | Yes | No |
| D+30 | Compliant (80.72%) | Compliant | Compliant | Compliant | 8.56% | Yes | No |
| D+40 | Compliant (80.74%) | Compliant | Compliant | Compliant | 8.55% | Yes | No |
| D+50 | Compliant (80.99%) | Compliant | Compliant | Compliant | 9.59% | Yes | No |
| D+60 | Compliant (80.27%) | Compliant | Compliant | Compliant | 8.45% | Yes | No |
| D+70 | Compliant (81.25%) | Compliant | Compliant | Compliant | 9.14% | Yes | No |
| D+80 | Compliant (80.61%) | Compliant | Compliant | Compliant | 9.38% | Yes | No |
| D+90 | Compliant (80.50%) | Compliant | Compliant | Compliant | 9.14% | Yes | No |
| D+100 | Compliant (80.49%) | Compliant | Compliant | Compliant | 8.38% | Yes | No |

(continued)

| High grade chlorinated lime A 25°C | | | | | | | |
|---|---|---|---|---|---|---|---|
| | "High grade chlorinated lime" official specification test | | | | | | |
| | Content | Attribut e | Identificatio n test (1) | Identificatio n test (2) | Moistur e | Hydroge n peroxid e reactio n | Swellin g |
| D+11 0 | Complian t (80.59%) | Complian t | Compliant | Compliant | 9.32% | Yes | No |
| D+12 0 | Complian t (80.81%) | Complian t | Compliant | Compliant | 9.82% | Yes | No |
| Toyokuron 40°C | | | | | | | |
| | "High grade test chlorinated lime" official specification | | | | | | |
| | Content | Attribut e | Identificatio n test (1) | Identificatio n test (2) | Moistur e | Hydroge n peroxid e reactio n | Swellin g |
| At star t | Compliant (69.51%) | Complian t | Compliant | Compliant | 18.46% | Yes | No |
| D+10 | Compliant (68.34%) | Complian t | Compliant | Compliant | 21.38% | Yes | Yes |
| D+20 | Compliant (64.85%) | Complian t | Compliant | Compliant | 20.25% | Yes | Yes |
| D+30 | Noncomplian t (59.83%) | Complian t | Compliant | Compliant | 20.89% | Yes | Yes |
| D+40 | Noncomplian t (53.58%) | Complian t | Compliant | Compliant | 21.95% | Yes | Yes |
| D+50 | Noncomplian t (46.49%) | Complian t | Compliant | Compliant | 24.12% | Yes | Yes |
| D+60 | Noncomplian t (39.58%) | Complian t | Compliant | Compliant | 24.04% | Yes | Yes |
| From D + 50, surface was moist, and the solid softened. | | | | | | | |

[Table 55-3]

| High grade chlorinated lime A 40°C | | | | | | | |
|---|---|---|---|---|---|---|---|
| | "High grade chlorinated lime" official specification test | | | | | | |
| | Content | Attribute | Identification test (1) | Identification test (2) | Moisture | Hydrogen peroxide reaction | Swellin g |
| At star t | Complian t (81.51%) | Complian t | Compliant | Compliant | 7.12% | Yes | No |
| D+10 | Complian t (81.02%) | Complian t | Compliant | Compliant | 7.98% | Yes | No |
| D+20 | Complian t (81.45%) | Complian t | Compliant | Compliant | 6.60% | Yes | No |
| D+30 | Complian t (81. 08%) | Complian t | Compliant | Compliant | 7.34% | Yes | No |

(continued)

| High grade chlorinated lime A 40°C | | | | | | | |
|---|---|---|---|---|---|---|---|
| | "High grade chlorinated lime" official specification test | | | | | | |
| | Content | Attribute | Identification test (1) | Identification test (2) | Moisture | Hydrogen peroxide reaction | Swellin |
| D+40 | Complian t (81.24%) | Complian t | Compliant | Compliant | 8.07% | Yes | No |
| D+50 | Complian t (80.71%) | Complian t | Compliant | Compliant | 8.74% | Yes | No |
| D+60 | Complian t (80.62%) | Complian t | Compliant | Compliant | 6.64% | Yes | No |
| D+70 | Complian t (81. 08%) | Complian t | Compliant | Compliant | 6.77% | Yes | No |
| D+80 | Complian t (80.68%) | Complian t | Compliant | Compliant | 8.72% | Yes | No |
| D+90 | Complian t (80.96%) | Complian t | Compliant | Compliant | 8.45% | Yes | No |
| D+10 0 | Complian t (80.95%) | Complian t | Compliant | Compliant | 7.72% | Yes | No |
| D+11 0 | Complian t (80.76%) | Complian t | Compliant | Compliant | 8.79% | Yes | No |
| D+12 0 | Complian t (80.77%) | Complian t | Compliant | Compliant | 8.63% | Yes | No |

(Oxidation-reduction potential and chlorine gas concentration)

ORP (oxidation-reduction potential) and chlorine gas concentration were measured for sample (1) and sample (2) that were the prepared mixtures (which meet the specification of sodium hypochlorite). As a control for comparison, a sodium hypochlorite solution with the same concentration was used. As a result, there was no change in ORP (oxidation-reduction potential) even when sodium hypochlorite was diluted, whereas the oxidizing power increased by dilution for samples (1) and (2). For the chlorine gas concentration, release of chlorine gas was hardly observed when contacted with an organic matter. An excellent property was observed in that only about 1/31 to 1/50 of concentration was measured.

[Table 56]

| | | | Sodium hypochlorite | Sample (1) | Sample (2) |
|---|---|---|---|---|---|
| | | | | 1:0.43 | 1:0.6 |
| Only solution | ORP (mV) | Undiluted solution (6%) | 560 | 535 | 533 |
| | | 1000 ppm | 560 | 613 | 608 |
| | | 200 ppm | 554 | 642 | 633 |
| | Chlorine gas concentration | Undiluted solution (6%) | 1.4 | 13 | 18 |
| | | 1000 ppm | 0.3 | 3 | 7 |
| | | 200 ppm | 0.1 | 2 | 4 |

(continued)

|  | | | Sodium hypochlorite | Sample (1) | Sample (2) |
|---|---|---|---|---|---|
|  | | | | 1:0.43 | 1:0.6 |
| Green onion immersion | Chlorine gas concentration | 1000 ppm | 250 | 5 | 8 |

<Instruments/equipment used>

GASTEC Corporation's gas collector GV-100S

GASTEC Corporation's detector tube No.8LL: measurement range from 0.025 to 2.0 ppm

GASTEC Corporation's detector tube No.8La: measurement range from 0.1 to 16 ppm

GASTEC Corporation's detector tube No.8H: measurement range from 25 to 1000 ppm

(Chlorine gas concentration of dissolved powder product)

By using the prepared high grade chlorinated lime liquid product, the chlorine gas concentration upon contact with an organic matter was measured in comparison with sodium hypochlorite of the same concentration. As a result, a high grade chlorinated lime liquid product did not rapidly generate chlorine gas even when contacted with an organic matter. Only about 1/1000 to 1/6000 of chlorine gas was measured. This leads to reduced adhesion amount of chlorine odor onto food products subjected to disinfect.

<Test segment>

*Sodium hypochlorite: 200 ppm, 1000 ppm
*High grade chlorinated lime liquid product: 200 ppm, 1000 ppm

50 g of each of the above solution was measured out. 5 g of cut cabbage was immersed therein (liquid ratio of 1:10) and the container was sealed. The cabbage was immersed thereafter for 1 hours at 25°C, and the chlorine gas concentration was measured.

[Table 57]

|  |  | Condition | Chlorine gas concentration |
|---|---|---|---|
| Sodium hypochlorite | 200 ppm | Bright | 50 ppm |
|  | 1000 ppm | Dark | 600 ppm |
| High grade chlorinated lime Liquid product | 200 ppm | Bright | 0.05 ppm |
|  | 1000 ppm | Dark | 0.1 ppm |

<Instruments/equipment used>

GASTEC Corporation's gas collector GV-100S
GASTEC Corporation's detector tube No.8LL: measurement range from 0.025 to 2.0 ppm
GASTEC Corporation's detector tube No.8La: measurement range from 0.1 to 16 ppm
GASTEC Corporation's detector tube No.8H: measurement range from 25 to 1000 ppm

(Examination of bactericidal effect)

The bactericidal effect on green onions, which are known to have a high number of bacteria in raw material, was examined using a liquid product (sodium hypochlorite specification) and solid product (high grade chlorinated lime specification) prepared by the present invention.

The superiority of bactericidal effect was confirmed for the liquid product in terms of both the overall viable bacteria count and E. coli group count compared to the control segment, i.e., sodium hypochlorite. In particular, the bactericidal effect on the E. coli group was confirmed to be prominent.

When the solid product was studied in two segments before and after removal of calcium in high grade chlorinated lime, a bactericidal effect on the E. coli group was similarly confirmed. High bactericidal effects on E. coli groups were confirmed as an overall feature of the bactericidal agent in the present invention.

```
[Table 58-1]

*Testing method
```

```
┌─────────────────┐
│ Raw material    │   Green
│                 │   onion`
└─────────────────┘
        ↓
┌─────────────────┐
│ Wash       with │   Wash with flowing water for 1 minute
│ water           │
└─────────────────┘
        ↓
┌─────────────────┐
│ Draining        │   Draining for 5 minutes with a strainer
└─────────────────┘
        ↓
┌─────────────────┐
│ Cut             │
└─────────────────┘
        ↓
┌─────────────────┐
│ Sterilization   │   Solid liquid ratio, raw material:liquid =
│                 │   1:10
└─────────────────┘
```

Immersion time: 30 minutes

|                      | (1)      | (2)       | (3)      | (4)       |
|----------------------|----------|-----------|----------|-----------|
| Sodium hypochlorite  | 200 ppm  | 1000 ppm  | –        | –         |
| Powerful PA          | –        | –         | 200 ppm  | 1000 ppm  |

|                             | (5)      | (6)       | (7)      | (8)       |
|-----------------------------|----------|-----------|----------|-----------|
| High grade chlorinated lime A*1 | 200 ppm  | 1000 ppm  | –        | –         |
| High grade chlorinated lime A*2 | –        | –         | 200 ppm  | 1000 ppm  |

```
        ↓
┌─────────────────┐
│ Wash       with │   Wash with flowing water for 1 minute
│ water           │
└─────────────────┘
```

```
        ↓
┌─────────────────────┐
│ Draining            │  Draining for 5 minutes with a strainer
└─────────────────────┘
        ↓
┌─────────────────────┐
│ Testing             │  Overall viable bacteria count and E. coli
└─────────────────────┘  group count after 72 hours at start and 10°C
```

*1 Before removal of Ca

*2 After removal of Ca

[Table 58-2]

| •Test result | | Overall viable bacteria count | | E. coli group count | |
|---|---|---|---|---|---|
| | | D+0 | D+3 | D+0 | D+3 |
| Raw material bacteria count | | 5.79 | - | 4.78 | - |
| Sodium hypochlorite | 200 ppm | 5.00 | 5.72 | 3.87 | 5.66 |
| | 1000 ppm | 3.57 | 5.61 | 2.64 | 4.73 |
| Powerful PA | 200 ppm | 4.20 | 5.71 | 3.85 | 4.43 |
| | 1000 ppm | 3.86 | 3.53 | 2.56 | 300 CFU or less |
| High grade chlorinated lime A*1 | 200 ppm | 5.15 | 6.63 | 3.78 | 4.64 |
| | 1000 ppm | 5.15 | 5.66 | 3.26 | 2.88 |
| High grade chlorinated lime A*2 | 200 ppm | 5.00 | 6.00 | 3.82 | 3.91 |
| | 1000 ppm | 4.79 | 5.59 | 3.36 | 300 CFU or less |

(logCFU)

(Difference 1 in reaction from the R2 method)

While the raw material is not deteriorated sodium hypochlorite, similar reaction methods include the R2 method.

The R2 method reacts sodium chlorate with sodium chloride in batches under a highly acidic condition (8 N to 11 N) using sulfuric acid to obtain chlorine dioxide gas. If the R2 method directly reacted with deteriorated sodium hypochlorite, chlorate ions are generated in recovery liquid A, which diverges from the objective of the present manufacturing method. In the present manufacturing method, both chlorate ions and chloride ions are naturally generated in the raw material deteriorated sodium hypochlorite, and chlorate ions and chloride ions each increase/decrease by performing the first reaction. There is already an excess amount of chloride ions in deteriorated sodium hypochlorite to generate hydrochloric acid. It is rather necessary to control excessive generation of hydrochloric acid due to chloride ions.

However, it is possible that the recovery rate increases by adding sodium chloride in the reaction mother liquor as in the R2 method. If so, it may be possible to reduce the cost of using sulfuric acid. Thus, the second reaction was performed using the reaction mother liquor (deteriorated sodium hypochlorite + sulfuric acid) that has completed the first reaction, 50% sulfuric acid and sodium chloride were added, and the degradation rate of chlorate ions and the recovery rate in recovery liquid B were studied.

When the second reaction was performed by adding 50% sulfuric acid and sodium chloride with the sulfuric acid concentration in the reaction mother liquor at about 17%, the degradation rate of chlorate ions in the reaction mother liquor improved as sodium chloride was added and reacted, but chloride ions increased. Furthermore, the recovery rate of chlorite ions in recovery liquid B was low, while an increase in chloride ions was observed.

It was found from the results thereof that a secondary reaction progresses in view of low acidity in the reaction mother liquor due to sulfuric acid, chlorine gas is generated by degradation of chloric acid, and chloride ions in the reaction mother liquor increases, and degradation of chlorine gas by hydrogen peroxide results in increased chloride ions and no progression in the primary reaction in recovery liquid B, so that chlorine dioxide generation and the recovery rate of chlorite ions decrease.

[Table 59-1]

(1) Reaction condition (first reaction, second reaction)

|  | (1) | (2) | (3) |
|---|---|---|---|
| Reaction mother liquor* | 200 g | 200 g | 200 g |
| Sodium chloride | 0 g | 5.0 g | 10.29 g |
| 50% sulfuric acid | 100 g | 100 g | 100 g |
| Reaction mother liquor (total) | 300 g | 305 g | 310.29 g |
| Total added sulfuric acid concentration | 17.87 % | 17.99 % | 17.37% |
| Temperature | 40 to 50°C | 40 to 50°C | 40 to 50°C |
| Reaction time | 5h | 5h | 5h |
| Air | 5h | 5h | 5h |

*Reaction mother liquor after the completion of the first reaction

(2) Recovery liquid

|  | Recovery liquid B (1) | Recovery liquid B (2) | Recovery liquid B (3) |
|---|---|---|---|
| 2N sodium hydroxide | 200 g | 200 g | 200 g |
| Hydrogen peroxide | 9 g | 9 g | 9 g |

[Table 59-2]

[Measurement results] (unit: ppm)

| After second reaction | $SO_4^{2-}$ | $Cl^-$ | $ClO_2^-$ | $ClO_3^-$ | Degradation rate* |
|---|---|---|---|---|---|
| Reaction mother liquor (1) | 316908 | 28108 | 89 | 14051 | 34.47 % |

| | | | | |
|---|---|---|---|---|
| Reaction mother liquor (2) | 299076 | 33076 | 0 | 11184 | 35.89% |
| Reaction mother liquor (3) | 263892 | 59189 | 0 | 9523 | 55.92 % |

Calculated from chlorate ions in the reaction mother liquor (after completion of first reaction)

| | Available chlorine concentration | Cl$^-$ | ClO$_2^-$ | ClO$_3^-$ |
|---|---|---|---|---|
| Recovery liquid B (1) | 12354 | 13726 | 5475 | 156 |
| Recovery liquid B (2) | 9800 | 9695 | 4475 | 159 |
| Recovery liquid B (3) | 16593 | 15259 | 8122 | 297 |

[Recovery rate of chlorite ion and available chlorine] (unit: ppm)

| | | Theoretical value | Measurement value | Recovery rate |
|---|---|---|---|---|
| Recovery liquid B (1) | Available chlorine(*) | 58970 | 12354 | 20.95 % |
| | ClO$_2^-$(*) | 28070 | 5475 | 19.51 % |
| Recovery liquid B (2) | Available chlorine(*) | 50034 | 9800 | 19.59 % |
| | ClO$_2^-$(*) | 23816 | 4475 | 18.79 % |
| Recovery liquid B (3) | Available chlorine(*) | 61128 | 16593 | 27.14 % |
| | ClO$_2^-$(*) | 29097 | 8122 | 27.91 % |

(*) Calculated from chlorate ions in the reaction mother liquor (after completion of first reaction)

(Difference 2 in reaction from the R2 method)

It was found that the degradation rate of chlorate ions reaches 100% without excessive addition of sulfuric acid by adding sodium chloride to the reaction mother liquor after the completion of the first reaction. While the degradation rate of chlorate ions increased, the yield on the other hand deteriorated.

In this regard, a reaction test was conducted using 50% w/w sulfuric acid to set the sulfuric acid concentration in the reaction mother liquor to 25.0% and 27.28%.

As a result, the primary reaction progressed due to the strongly acidic condition, and recovery of chlorite ions progressed with generation of chlorine dioxide gas. However, a large quantity of chloride ions were still recovered in recovery liquid B. This is understood as chlorine gas contamination with the progression of a secondary reaction.

It was found in view of the above that a secondary reaction tends to progress when acidity from sulfuric acid is low, but an excessive sulfuric acid concentration results in increased hydrochloric acid generation and progression of a secondary reaction. Thus, focusing too much on degradation of chloric acid results in progression of a side effect, which does not lead to an improved recovery rate. In addition, prevention of progression of a secondary reaction by controlling excessively generated hydrochloric acid is rather more important for a reaction of deteriorated sodium hypochlorite which already has sufficient chloride ions.

[Table 60-1]

(1) Reaction condition (first reaction, second reaction)

|  | (4) | (5) |
|---|---|---|
| Reaction mother liquor* | 200 g | 200 g |
| Sodium chloride | 0 g | 10.0 g |

| 50% sulfuric acid | 200 g | 250 g |
|---|---|---|
| Reaction mother liquor (total) | 400 g | 450 g |
| Total added sulfuric acid concentration | 25.00 % | 27.78 % |
| Temperature | 40 to 50°C | 40 to 50°C |
| Reaction time | 5h | 5h |
| Air | 5h | 5h |

*Reaction mother liquor after the completion of first reaction

(2) Recovery liquid

|  | Recovery liquid B (4) | Recovery liquid B (5) |
|---|---|---|
| 2N sodium hydroxide | 200 g | 200 g |
| Hydrogen peroxide | 9 g | 9 g |

[Table 60-2]

[Results] (unit: ppm)

| After second reaction | $SO_4{}^{2-}$ | $Cl^-$ | $ClO_2{}^-$ | $ClO_3{}^-$ | degradation rate |
|---|---|---|---|---|---|
| Reaction mother liquor (4) | 62398 | 45226 | 0 | 3607 | 78.68 % |
| Reaction mother liquor (5) | 61087 | 47066 | 0 | 0 | 100.00 % |

| | Available chlorine concentration | $Cl^-$ | $ClO_2{}^-$ | $ClO_3{}^-$ |
|---|---|---|---|---|
| Recovery liquid B (4) | 28729 | 24754 | 15355 | 576 |
| Recovery liquid B (5) | 33958 | 28695 | 22064 | 656 |

[Recovery rate of chlorite ion and available chlorine]

| | | Theoretical value | Measurement value | Recovery rate |
|---|---|---|---|---|
| Recovery liquid B (4) | Available chlorine(*) | 60194 | 28729 | 47.73 % |
| | $ClO_2{}^-$ (*) | 28652 | 15355 | 53.59 % |
| Recovery liquid B (5) | Available chlorine(*) | 59228 | 33958 | 57.34% |
| | $ClO_2{}^-$ (*) | 28192 | 22064 | 78.26 % |

(*) Calculated from chlorate ions in the reaction mother liquor (after completion of first reaction)

(Difference 3 in reaction from the R2 method)

It was found that addition of sodium chloride to the reaction mother liquor after the completion of the first reaction generates hydrochloric acid, which results in the degradation rate of chlorate ions reaching 100% without excessive addition of sulfuric acid.

However, it was found that the recovery rate of chlorous acid does not improve even when the degradation rate of chlorate ions reach 100% because there are two chloric acid degradation reactions in primary and secondary reactions with varying progression depending on reaction conditions, and chloride ions in recovery liquid B are brought about by generation of chlorine gas due to degradation of chlorate ions. For this reason, complete degradation of chlorate ions in view of excessive generation of hydrochloric acid results in generation of a large quantity of chloride ions and then decreased recovery rate and purity.

[Table 61-1]

(1) Reaction condition (first reaction, second reaction)

| | (6) | (7) | (8) |
|---|---|---|---|
| Reaction mother liquor* | 400 g | 400 g | 400 g |
| Sodium chloride | 20g | 20 g | 20 g |
| 50% sulfuric acid | 500 g | 500 g | 500 g |
| Reaction mother liquor (total) | 920 g | 920 g | 920 g |
| Total added sulfuric acid concentration | 27.2 % | 27.2 % | 27.2 % |
| Temperature | 40 to 50°C | 40 to 50°C | 40 to 50°C |
| Reaction time | 5h | 5h | 5h |
| Air | 5h | 5h | 5h |

*Reaction mother liquor after completion of the first reaction

(2) Recovery liquid

| | Recovery liquid B (6) | Recovery liquid B (7) | Recovery liquid B (8) |
|---|---|---|---|
| 2N sodium hydroxide | 200 g | – | – |
| 1.8N hydrogen peroxide | – | 200 g | – |
| 1.4N sodium hydroxide | – | – | 214.15 g |
| 35% hydrogen peroxide | 16 g | 16 g | 16 g |

[Table 61-2]

[Measurement results] (unit: ppm)

| After second reaction | $SO_4^{2-}$ | $Cl^-$ | $ClO_2^-$ | $ClO_3^-$ | Degradation rate |
|---|---|---|---|---|---|
| Reaction mother liquor | 500198 | 23404 | 30 | 0 | 100.0 % |

| (6) | | | | | |
|---|---|---|---|---|---|
| Reaction mother liquor (7) | 505048 | 20816 | 0 | 0 | 100.0 % |
| Reaction mother liquor (8) | 488163 | 22880 | 0 | 0 | 100.0 % |

| | Available chlorine concentration | Cl⁻ | ClO₂⁻ | ClO₃⁻ |
|---|---|---|---|---|
| Recovery liquid B (6) | 63112 | 52593 | 33604 | 1068 |
| Recovery liquid B (7) | 67458 | 40641 | 36015 | 263 |
| Recovery liquid B (8) | 67569 | 46060 | 35904 | 69 |

[Recovery rate of chlorite ion and available chlorine]

| | | Theoretical value | Measurement value | Recovery rate |
|---|---|---|---|---|
| Recovery liquid B (6) | Available chlorine(*) | 106340 | 63112 | 59.35 % |
| | ClO₂⁻(*) | 50618 | 33604 | 66.39 % |
| Recovery liquid B (7) | Available chlorine(*) | 117817 | 67458 | 57.26 % |
| | ClO₂⁻(*) | 56081 | 36015 | 64.22 % |
| Recovery liquid B (8) | Available chlorine(*) | 116985 | 67569 | 57.76 % |
| | ClO₂⁻(*) | 55685 | 35904 | 64.48 % |

(*4) Calculated from chlorate ions in the reaction mother liquor (after completion of first reaction)

(Fundamental test 1 for studying the conditions for the first reaction and increase in chlorate ions)

The degree of increase/decrease in chloride ions and chlorate ions after the first reaction using deteriorated sodium hypochlorite was studied. The first reaction is generally performed at ordinary temperature at 20°C to 30°C, and at most 40°C due to generation of heat in a reaction.

When only the primary reaction was experimentally performed, a decrease in chloride ions and an increase in chlorate ions were confirmed. It was found that chlorate ions continue to increase up to about 40°C.

However, if a primary reaction is performed at 40°C, chlorite ions would be detected in a recovery liquid. Thus, it was found that a reaction is preferably performed at ordinary temperature around 30°C as the temperature condition of the primary reaction. It is also understood that too much increase in the temperature is not preferred because steam is generated and entrainment of sulfuric acid, hydrochloric acid, or the like to the recovery liquid increases.

[Table 62-1]

(1) Raw material

| Raw material | Available chlorine concentration | $ClO_3^-$ | $Cl^-$ |
|---|---|---|---|
| Deteriorated sodium hypochlorite (*) | 67570 | 20577 | 44855 |

*12% low salt sodium hypochlorite

(2) Reaction condition (first reaction, second reaction)

| | Reaction mother liquor |
|---|---|
| | First reaction |
| Deteriorated hypo | 200 g |
| 50% sulfuric acid | 25 g |
| Total added | 5.56 % |

| sulfuric acid concentration | |
|---|---|
| Reaction time | 2h 30m |
| (Air time) | 2h |

(3) Reaction temperature

| | (1) | (2) | (3) | (4) |
|---|---|---|---|---|
| Reaction temperature | 20°C | 30°C | 40°C | 50°C |

(4) Recovery liquid

| | Recovery liquid A |
|---|---|
| 2N sodium hydroxide | 200 g |

[Table 62-2]

| [Measurement results] (unit: ppm) Reaction mother liquor (after completion of the first reaction) |
|---|

(continued)

|  | (1) 20°C | (2) 30°C | (3) 40°C | (4) 50°C |
|---|---|---|---|---|
| Final liquid amount | 219 | 219 | 218 | 218 |
| Available chlorine | 11000 | 7870 | 8300 | 5570 |
| Cl- | 30258.8 | 25921.4 | 31942.4 | 30570.0 |
| $ClO_2^-$ | - | - | - | - |
| $ClO_3^-$ | 36452.4 | 32967.4 | 40662.6 | 40603.6 |
| Cl- reduction rate | 32.54 % | 42.21 % | 28.79 % | 31.85 % |
| $ClO_3^-$ increase rate | 177.15 % | 160.21 % | 197.61 % | 197.33 % |
| Recovery liquid A | | | | |
|  | (1) 20°C | (2) 30°C | (3) 40°C | (4) 50°C |
| Final liquid amount | 207 | 207 | 208 | 208 |
| Available chlorine | 32020 | 34150 | 33680 | 35520 |
| $Cl^-$ | 23945.9 | 26969.5 | 27120.8 | 28865.6 |
| $ClO_2^-$ | 22.1 | 24.7 | 103.3 | 71.5 |
| $ClO_3^-$ | 145.4 | 194.8 | 99.6 | 437.5 |
| $SO_4^{2-}$ | 32.6 | 29.9 | 24.8 | 26.6 |

(Fundamental test 2 for studying the conditions for the first reaction and increase in chlorate ions)

The effect of acidity in the first reaction was studied by further adding 65 w/w% sulfuric acid for re-verification of the previous test.

As result, chlorate ions increased more when the acidity was increased as compared to the effect of temperature. On the other hand, it is thought that hydrochloric acid generation also progresses. As a result, chlorite ions and chlorate ions would be detected in recovery liquid A, and a deterioration in the recovery rate is also possible. Therefore, it is not preferable to excessively increase the sulfuric acid concentration in the reaction mother liquor in the first reaction.

[Table 63-1]

(1) Raw material

| Raw material | Available chlorine concentration | ClO$_3$$^-$ | Cl$^-$ |
|---|---|---|---|
| Deteriorated sodium hypochlorite (*) | 67570 | 20577 | 44855 |

*12% low salt sodium hypochlorite

(2) Reaction condition (first reaction, second reaction)

| | Reaction mother liquor |
|---|---|
| | First reaction |
| Deteriorated hypo | 200 g |
| 50% sulfuric acid | 25 g |

| 65% sulfuric acid | 25 g added in the middle |
|---|---|
| Total added sulfuric acid concentration | 11.5 % |
| Reaction time | 2h 30m |
| (Air time) | 2h |

(3) Reaction temperature

| | (1) | (2) | (3) | (4) |
|---|---|---|---|---|
| Reaction temperature | 20°C | 30°C | 40°C | 50°C |

(4) Recovery liquid

| | Recovery liquid A |
|---|---|
| 2N sodium hydroxide | 200 g |

[Table 63-2]

| [Measurement results] (unit: ppm) | | | | |
|---|---|---|---|---|
| Receiving solution | (1) 20°C | (2) 30°C | (3) 40°C | (4) 50°C |
| Final liquid amount | 240 | 240 | 239 | 239 |
| Available chlorine | < 25 | < 25 | < 25 | < 25 |
| $Cl^-$ | 17681.8 | 20023.0 | 19742.2 | 24436.2 |
| $ClO_2^-$ | - | - | - | - |
| $ClO_3^-$ | 34958.2 | 35515.2 | 32078.6 | 37960.4 |
| $Cl^-$ decrease rate | 60.58 % | 55.36 % | 55.99 % | 45.52 % |
| $ClO_3^-$ increase rate | 169.89 % | 172.6 % | 155.9 % | 184.48 % |
| | | | | |
| Recovery liquid A | (1) 20°C | (2) 30°C | (3) 40°C | (4) 50°C |
| Final liquid amount | 209 | 209 | 210 | 210 |
| Available chlorine | 49340 | 47760 | 45440 | 44170 |
| $Cl^-$ | 42367.4 | 40490.1 | 38570.5 | 37112.9 |
| $ClO_2^-$ | 117.7 | 91.0 | 124.1 | 188.9 |
| $ClO_3^-$ | 231.8 | 190.9 | 287.3 | 266.4 |
| $SO_4^{2-}$ | 110.5 | 58.4 | 45.3 | 42.3 |

(Explanation of the tests)

While re-reacting deteriorated sodium hypochlorite, which is sodium hypochlorite with deteriorated quality, to re-manufacture sodium hypochlorite is not in itself a novel technology, the inability to add the cost of re-manufacture to the unit price of a product is a problem.

In this regard, the focus was placed on chloride ions and chlorate ions generated in deteriorated sodium hypochlorite to examine whether a disinfectant with ions of various chlorine oxides in a state of a complex can be manufactured by reactions of such ions.

It was confirmed as a result thereof that an attempt to recover both chlorine gas and chlorine dioxide gas with a single solution after reacting deteriorated sodium hypochlorite would lead to problems such as degradation into chloride ions and generation of chlorate ions. It was found that a reaction condition for recovering chlorine gas and chlorine dioxide gas in a stepwise manner from a reaction mother liquor consisting of deteriorated sodium hypochlorite and sulfuric acid is required for individual recovery.

It was found that the reaction condition also varies depending on the grade (low salt or general grade) of the raw material sodium hypochlorite.

The recovery liquid is recovered only with sodium hydroxide or calcium hydroxide in the first reaction, and recovered by adding hydrogen peroxide to sodium hydroxide in the second reaction.

For recovery in the second reaction, an intermediate vessel for preventing reflux is provided between a reaction tank and a recovery vessel. It is preferable to add hydrogen peroxide water to prevent chlorine gas contamination, especially for general grade sodium hypochlorite.

These lead to increasing the purity by removing residual alkaline components and chloride ions when drying into a granulated form.

It is also necessary for the mixing ratio of recovery liquid A and recovery liquid B to comply with the specification and standard for a food additive, sodium hypochlorite or high grade chlorinated lime. For sodium hypochlorite, the available chlorine of recovery liquid B cannot exceed 0.6 given that the available chlorine of recovery liquid A is 1. In view of the feature of the present invention, it is desirable to adjust the available chlorine within the range of 0.43 to 0.6. Likewise for high grade chlorinated lime, the available chlorine of recovery liquid B needs to be 33.95 or less given that the available chlorine of recovery liquid A is 1. In view of the feature of the present product, 1:9.6 is desirable.

When drying and concentrating, the time can be shortened, and loss of available chlorine or change in the composition can be prevented by slurrying each solution to a certain degree and then mixing and drying, rather than combining the two solutions at the solution stage. A dry solid manufactured by these methods would be a chlorine oxide solid having very low degradation of free residual chlorine and composition of the solution maintained for a long period of time.

As is clear from the tests above, the invention can not only use sodium hypochlorite with deteriorated quality as a raw material to restore this to waste or low cost sodium hypochlorite, but also regenerate the material into a disinfectant with added value to end consumers.

As disclosed above, the present invention is exemplified by the use of its preferred embodiments. However, it is understood that the scope of the present invention should be interpreted based solely on the Claims. The present application claims priority to Japanese Patent Application No. 2018-71515 (filed on April 3, 2018). The entire content thereof is incorporated herein by reference. It is also understood that any patent, any patent application, and any references cited herein should be incorporated herein by reference in the same manner as the contents are specifically described herein.

[Industrial Applicability]

The composition of the invention is useful as a disinfectant. The method of the invention regenerates sodium hypochlorite with deteriorated quality to newly provide a useful disinfectant.

## Claims

1. A dry solid comprising a hypochlorite and a chlorite.

2. The dry solid of claim 1, wherein the solid is in a dry granulated form.

3. The dry solid of claim 1 or 2, wherein the solid comprises calcium hypochlorite.

4. The dry solid of any one of claims 1 to 3, wherein the solid has the following properties:

   (1) comprises available chlorine at 60.0% or more;
   (2) has a chlorine odor;
   (3) when 5 ml of water is added to 0.5 g of the solid and shaken and red litmus paper is immersed therein, the litmus paper changes its color to blue and then loses its color; and
   (4) when 2 ml of acetic acid (1 → 4) is added to 0.1 g of the solid, the solid dissolves while generating gas, and a solution prepared by adding 5 ml of water thereto and filtrate exhibits a reaction of calcium salt.

5. The dry solid of any one of claims 1 to 4, wherein the solid comprises an $SO_4$ based component in the range from the detection limit or higher and 8100 ppm or lower.

6. The dry solid of any one of claims 1 to 5, wherein a ratio of the hypochlorite to the chlorite in the solid is 1:5 to 25.

7. The dry solid of any one of claims 1 to 6, wherein an available chlorine concentration in the solid is within a range of 600,000 ppm to 900,000 ppm, and a free residual chlorine concentration is within a range of 900 ppm to 60,000 ppm.

8. A liquid obtained by dissolving the dry solid of any one of claims 1 to 7.

9. The liquid of claim 8, wherein a ratio of hypochlorite ions to chlorite ions is 1:7 to 35, when diluted with water so that an available chlorine concentration would be 1%.

10. The liquid of claim 8 or 9, wherein a free residual chlorine concentration is within a range of 150 ppm to 900 ppm, when diluted with water so that an available chlorine concentration would be 1%.

11. The liquid of claim 8, wherein a ratio of hypochlorite ions to chlorite ions is 1:6 to 30, when diluted with water so that an available chlorine concentration would be 6%.

12. The liquid of claim 8 or 11, wherein a free residual chlorine concentration is within a range of 1,000 ppm to 6,000 ppm, when diluted with water so that an available chlorine concentration would be 6%.

13. The liquid of claim 8, wherein a ratio of hypochlorite ions to chlorite ions is 1:6 to 30, when diluted with water so that an available chlorine concentration would be 12%.

14. The liquid of claim 8 or 13, wherein a free residual chlorine concentration is within a range of 2,500 ppm to 12,000 ppm, when diluted with water so that an available chlorine concentration would be 12%.

15. A method of manufacturing a dry solid comprising a hypochlorite and a chlorite, comprising:

a step of preparing a solution comprising a hypochlorite ion, a chlorate ion, and a chloride ion;
a first reaction step for adding sulfuric acid to the solution to generate chlorine gas;
a step of reacting the generated chlorine gas with sodium hydroxide or calcium hydroxide and recovering a reaction product as a hypochlorite ion in recovery liquid A;
a second reaction step for adding sulfuric acid to a reaction mother liquor after the first reaction step at a concentration that is higher than that in the first reaction step to generate chlorine dioxide gas;
a step of reacting the generated chlorine dioxide gas with sodium hydroxide and hydrogen peroxide and recovering a reaction product as a chlorite ion in recovery liquid B;
a step of mixing recovery liquid A with recovery liquid B; and
a step of drying and solidifying the resulting mixture.

16. The method of claim 15, wherein the recovery liquid A comprises calcium hydroxide.

17. The method of claim 15 or 16, further comprising a step of adding hydrogen peroxide to the reaction mother liquor after a first reaction.

18. The method of any one of claims 15 to 17, wherein an available chlorine concentration of the recovery liquid B is within a range of 9.6 to 33.95, given that an available chlorine concentration of the recovery liquid A is 1, in the step of mixing the recovery liquid A with the recovery liquid B.

19. The method of any one of claims 15 to 18, wherein the recovery liquid A and the recovery liquid B are each slurried and mixed in the step of mixing the recovery liquid A with the recovery liquid B.

20. The method of any one of claims 15 to 19, wherein the step of mixing the recovery liquid A with the recovery liquid B comprises a step of preliminary drying the recovery liquid A to form a granulation nucleus, slurrying the recovery liquid B, and adding dried recovery liquid A to the slurried recovery liquid B.

21. The method of any one of claims 15 to 20, wherein the step of drying and solidifying comprises a step of drying with warm air for 20 to 30 minutes.

22. The method of any one of claims 15 to 21, wherein the step of drying and solidifying comprises reducing moisture content of each of the recovery liquid A and the recovery liquid B to 20% or less.

23. A method of manufacturing a new disinfectant from a solution comprising a hypochlorite ion, a chlorate ion, and a chloride ion; comprising:

a step of quantifying a hypochlorite ion concentration, a chlorate ion concentration, and a chloride ion concentration in the solution;
a first reaction step for adding sulfuric acid to the solution to generate chlorine gas;
a step of reacting the generated chlorine gas with sodium hydroxide or calcium hydroxide and recovering a reaction product as a hypochlorite ion in recovery liquid A;
a second reaction step for adding sulfuric acid to a reaction mother liquor after the first reaction step at a concentration that is higher than that in the first reaction step to generate chlorine dioxide gas;
a step of reacting the generated chlorine dioxide gas with sodium hydroxide and hydrogen peroxide and recovering a reaction product as a chlorite ion in recovery liquid B; and
a step of mixing the recovery liquid A with the recovery liquid B to obtain a new disinfectant.

24. The method of claim 23, wherein the solution comprising a hypochlorite ion, a chlorate ion, and a chloride ion is a solution comprising a hypochlorite with deteriorated quality.

25. The method of claim 24, wherein the solution comprising a hypochlorite with deteriorated quality is derived from a low salt grade sodium hypochlorite solution.

26. The method of claim 24, wherein the solution comprising a hypochlorite with deteriorated quality is derived from a general grade sodium hypochlorite solution.

27. The method of claim 25, wherein the disinfectant is a solid product, and a sulfuric acid concentration in a reaction mother liquor in the first reaction step is 4.00 to 6.37%, a sulfuric acid concentration in a reaction mother liquor in the second reaction step is 30.00 to 40.00%, and a sulfuric acid concentration used in the second reaction step is 50.0 w/w% to 70.0 w/w%.

28. The method of claim 25, wherein the disinfectant is a liquid product, and a sulfuric acid concentration in a reaction mother liquor in the first reaction step is 4.00 to 6.37%, a sulfuric acid concentration in a reaction mother liquor in the second reaction step is 30.00 to 59.04%, and a sulfuric acid concentration used in the second reaction step is 50.0 w/w% to 70.0 w/w%.

29. The method of claim 26, wherein a sulfuric acid concentration in a reaction mother liquor in the first reaction step is 4.00 to 4.50%, a sulfuric acid concentration in a reaction mother liquor in the second reaction step is 25.00 to 30.00%, and a sulfuric acid concentration used in the second reaction step is 65 w/w%.

30. The method of any one of claims 23 to 29, satisfying:

$$(1) \ Y = -1.2676X + 9.84393;$$

and

$$(2) \ X \leq 4;$$

wherein a chloride concentration in a raw material is X%, and a sulfuric acid concentration in a reaction mother liquor is Y% in a first reaction.

31. The method of any one of claims 23 to 30, wherein the recovery liquid A comprises sodium hydroxide or calcium hydroxide.

32. The method of any one of claims 23 to 31, wherein the recovery liquid B comprises sodium hydroxide and hydrogen peroxide.

33. The method of any one of claims 23 to 32, wherein the first reaction step is performed while blowing air.

34. The method of any one of claims 23 to 33, wherein the second reaction step is performed while blowing air.

35. The method of any one of claims 23 to 34, wherein an intermediate trapping vessel comprising hydrogen peroxide is provided between a reaction tank and a recovery vessel comprising recovery liquid B.

36. The method of any one of claims 23 to 35, further comprising a step of adding hydrogen peroxide to a reaction mother liquor after a first reaction.

37. The method of any one of claims 23 to 36, wherein an available chlorine concentration of the recovery liquid B is 0.43 to 0.6, given that an available chlorine concentration of the recovery liquid A is 1, in the step of mixing the recovery liquid A with the recovery liquid B.

38. The method of any one of claims 23 to 37, wherein the disinfectant comprises sodium hypochlorite.

39. The method of claim 38, wherein the disinfectant has the following properties:

(1) comprises available chlorine at 4.0% or more;

(2) has a chlorine odor;

(3) exhibits a reaction of a sodium salt and a reaction of a hypochlorite;

(4) a solution preparing by adding 100 ml of phosphate buffer (pH 8) to 4 ml of an aqueous solution (1 → 25) of this product has a maximum absorbance section at a wavelength of 291 to 294 nm; and

(5) red litmus paper, when immersed in this product, changes its color to blue and then loses its color.

40. The method of any one of claims 23 to 39, wherein the disinfectant comprises an $SO_4$ based component in the range from the detection limit or higher and 8100 ppm or lower.

41. The method of any one of claims 23 to 40, wherein a ratio of hypochlorite ions to chlorite ions in the disinfectant is 1:0.24 to 0.3.

42. The method of any one of claims 23 to 41, wherein an available chlorine concentration in the disinfectant is about 60,000 ppm, and a free residual chlorine concentration is about 60,000 ppm.

43. A disinfectant manufactured by the method of any one of claims 23 to 42.

44. The disinfectant of claim 43, wherein a ratio of hypochlorite ions to chlorite ions is 1:0.24 to 0.3.

45. The disinfectant of claim 43 or 44, wherein an available chlorine concentration in the disinfectant is about 60,000 ppm, and a free residual chlorine concentration is about 60,000 ppm.

46. A liquid chlorine oxide manufactured by using the dry solid of any one of claims 1 to 7, prepared by a method comprising:

(a) a step of dissolving the dry solid into water to prepare a solution with an elevated pH;

(b) a step of adding a non-calcium inorganic alkaline agent to the solution while maintaining a pH of the solution prepared in step (a) to allow a calcium salt to precipitate and form a solid/liquid mixed phase with a reduced calcium ion concentration in a liquid phase, comprising the liquid phase and a solid phase comprising a calcium salt; and

(c) retrieving only the liquid phase from the solid/liquid mixed phase formed in step (b) to obtain a liquid chlorine oxide.

47. A liquid chlorine oxide manufactured by using the dry solid of any one of claims 1 to 7, prepared by a method comprising:

(a) a step of dissolving the dry solid into water to prepare a solution with a pH of 10.0 or greater;

(b) a step of adding a non-calcium inorganic alkaline agent to the solution while maintaining a pH of the solution prepared in step (a) at 10.0 or greater to allow a calcium salt to precipitate and form a solid/liquid mixed phase with a calcium ion concentration of 24 ppm or less in a liquid phase, comprising the liquid phase and a solid phase comprising a calcium salt; and

(c) retrieving only the liquid phase from the solid/liquid mixed phase formed in step (b) to obtain a liquid chlorine oxide.

48. The liquid or liquid chlorine oxide of any one of claims 8 to 14, 46, and 47, wherein a calcium concentration is substantially at or below a detection limit.

49. The liquid or liquid chlorine oxide of any one of claims 8 to 14, 46, and 47, wherein a calcium concentration is 24 ppm or less.

50. Use of the dry solid of any one of claims 1 to 7, the liquid of any one of claims 8 to 14, or the liquid or liquid chlorine oxide of any one of claims 46 to 49 as a disinfectant.

51. Use of the dry solid of any one of claims 1 to 7, the liquid of any one of claims 8 to 14, the disinfectant of any one of claims 43 to 45, or the liquid or liquid chlorine oxide of any one of claims 46 to 49 as a food additive.

52. Use of the dry solid of any one of claims 1 to 7, the liquid of any one of claims 8 to 14, the disinfectant of any one of claims 43 to 45, or the liquid or liquid chlorine oxide of any one of claims 46 to 49, for disinfecting a food product.

FIG.1

<Identification test(1) Sodium hypochlorite(3)Loss of color of potassium permanganate>

From the left, sodium hypochlorite, available chlorine ratio of 1:0.6, available chlorine ratio of 1:0.7, sodium chlorite

# FIG.2

<Sample 1 (available chlorine ratio 1: 0.429) absorbance>
Sodium hypochlorite

<Sample 2 (available chlorine ratio 1: 0.6) absorbance>
Sodium hypochlorite

<table>
<tr><td colspan="2"><b>INTERNATIONAL SEARCH REPORT</b></td><td>International application No.<br>PCT/JP2019/014649</td></tr>
</table>

**A. CLASSIFICATION OF SUBJECT MATTER**

Int.Cl. A01N59/08(2006.01)i, A01N25/08(2006.01)i, A01P1/00(2006.01)i, A23L3/3454(2006.01)i, A61L2/18(2006.01)i, C01B11/06(2006.01)i, C01B11/10(2006.01)i, C02F1/50(2006.01)i

According to International Patent Classification (IPC) or to both national classification and IPC

**B. FIELDS SEARCHED**

Minimum documentation searched (classification system followed by classification symbols)
Int.Cl. A01N, A23L, A61L, C01B, C02F, C11D,

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y<br>A | JP 58-215499 A (UEHARA, Motoyasu) 14 December 1983, page 2, lower left column to page 3, upper left column (Family: none) | 1, 2, 5, 8, 43, 45-50<br>51, 52<br>15-42 |
| Y | JP 2009-72064 A (KEWPIE CORPORATION) 09 April 2009, paragraphs [0006]-[0018] (Family: none) | 51, 52 |

☒ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 10 June 2019 (10.06.2019) | 25 June 2019 (25.06.2019) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2019/014649

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP 2005-523867 A (SK AQUATECH CO., LTD.) 11 August 2005, paragraphs [0036]-[0037], [0040] & US 2005/0224750 A1 (paragraphs [0039], [0040], [0043]) & WO 2003/093170 A1 & KR 10-2002-0050194 A & KR 10-2003-0085627 A & AU 2003235188 A | 1-6<br>15-42 |
| X<br>A | JP 2013-204169 A (KURITA WATER INDUSTRIES LTD.) 07 October 2013, paragraph [0032] & WO 2013/145440 A1 & KR 10-2014-0112575 A & CN 104204352 A | 8, 9, 11, 13, 43-50<br>15-42 |

Form PCT/ISA/210 (continuation of second sheet) (January 2015)

## INTERNATIONAL SEARCH REPORT

| International application No. |
| --- |
| PCT/JP2019/014649 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.: 7, 10, 12, 14
because they relate to parts of the international application that do not comply with the prescribed requirements to such an extent that no meaningful international search can be carried out, specifically:
See extra sheet

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
| --- |
| PCT/JP2019/014649 |

\<Continuation of Box No. II\>

Claims 7, 10, 12, and 14 have the statements that the respective ranges of "effective chlorine concentration" and "free residual chlorine concentration" are specified, but the ranges of the specified "effective chlorine concentration" and "free residual chlorine concentration" do not almost overlap. However, since "effective chlorine concentration" and "free residual chlorine concentration" have the same meaning, said statements are contrary to common technical knowledge and have unclear meaning, and thus the technical matters specified by said claims cannot be comprehended.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5931253 B

- JP 2018071515 A